# EUROPEAN PATENT APPLICATION

(11) **EP 4 653 469 A1**
(43) Date of publication of application: **26.11.2025**
(21) Application number: 24744404.5
(22) Date of filing: 19.01.2024
(51) Int. Cl.: C07K 19/00, C07K 14/04, C12N 15/38, C12N 15/62, C12N 15/63, C12N 5/10, A61K 39/25, A61K 48/00, A61P 31/22, G01N 33/53

(54) **FUSION PROTEIN OF GE AND GI OF VARICELLA-ZOSTER VIRUS AND USE**

(30) Priority: 19.01.2023 CN 202310079253
(71) Applicant: Xiamen University, Xiamen, Fujian 361005 (CN); Xiamen Innovax Biotech Co., Ltd., Xiamen, Fujian 361022 (CN); Beijing Wantai Biological Pharmacy Enterprise Co., Ltd., Beijing 102206 (CN)
(72) Inventor: LI, Tingting, Xiamen, Fujian 361005 (CN); LI, Shaowei, Xiamen, Fujian 361005 (CN); ZHANG, Sibo, Xiamen, Fujian 361005 (CN); ZENG, Yarong, Xiamen, Fujian 361005 (CN); CUI, Lingyan, Xiamen, Fujian 361005 (CN); YU, Jinfeng, Xiamen, Fujian 361005 (CN); CHEN, Tingting, Xiamen, Fujian 361005 (CN); GU, Ying, Xiamen, Fujian 361005 (CN); CHENG, Tong, Xiamen, Fujian 361005 (CN); XIA, Ningshao, Xiamen, Fujian 361005 (CN)
(74) Representative: Lavoix
(86) International application number: PCT/CN2024/073289
(87) International publication number: WO 2024/153235

(57) **Abstract**

The present invention provides fusion proteins of gE and gl (or variants thereof) that can be used for preventing or treating varicella-zoster virus infection, and use of such fusion proteins (or variants thereof).

## Description

The present application claims the benefit of Chinese Application No. 2023100792531, filed January 19, 2023, the disclosure of the Chinese application is incorporated herein by reference in its entirety.

### Technical Field

The present disclosure relates to the fields of immunology and molecular virology, in particular to the field of prevention and treatment of varicella-zoster virus. Specifically, the present disclosure relates to a fusion protein of gE and gI, or variant thereof, that can be used to prevent or treat a varicella-zoster virus infection, and the use of such fusion protein or variant thereof.

### Background Art

Varicella-zoster virus (VZV) is a DNA virus, and comprises an envelope. VZV particle is spherical in shape, which is composed of 150 hexamers and 12 pentamers assembled into a regular icosahedral structure with T=16. VZV particle can be roughly divided radially, from the spherical center to the outside, into four parts, including virus nucleic acid (virus DNA), capsid, tegument protein and envelope protein in sequence from the inside to the outside. The core of the virus is DNA, which controls the viral life cycle. The icosahedral nucleocapsid of VZV is very stable and plays an important role in protecting the internal genetic materials. The outer part of the virus capsid is tegument protein, which is composed of a variety of proteins encoded by the virus DNA, and plays a role in the process of virus infection, expression in host cells, immune response, assembly and exocytosis. The outer surface of the VZV particle is an envelope formed by a variety of glycoproteins and lipids, in which the main glycoproteins are gB, gC, gE, gH, gL, gI, etc., and they play an important role in identifying host cells, immune response, and enhancing the stability of virus particles.

After being infected with VZV, people are prone to papules and chickenpox, often complicated by pneumonia, and have a high mortality rate (mostly in children). After the host recovers, a small amount of VZV will still remain in its neuronal cells, and when VZV is activated again, it will induce symptoms such as herpes zoster and postherpetic neuralgia, and in severe cases, it will cause malignant complications such as encephalomyelitis and conjunctivitis (mostly in immunodeficient or immunocompromised people aged ≥ 50 years). Vaccination is the best solution to prevent diseases such as chickenpox and herpes zoster, but there are currently few vaccines available. The main protein component of Shingrix^{™}, the world's first recombinant herpes zoster vaccine, is the envelope glycoprotein E (gE) of VZV, indicating that gE is an important target for studying herpes zoster vaccines. As the glycoprotein with the highest content on the VZV envelope, gE contains a total of 623 amino acid residues, and some of its amino acid residues play an important role in the passage of VZV. Among them, the amino acids 24-71 and 163-208 of gE are the key regions for it to recognize IDE (Insulin-degrading enzyme) receptor and form heterodimers with gI, respectively, so that whether gE can form a correct secondary structure is also the key to its interaction with IDE. At present, some subunit vaccines or preclinical vaccines used to prevent VZV infection are designed around gE protein. The content of envelope glycoprotein I (gI) on VZV particles is second only to gE. gI is composed of 354 amino acid residues in total, which can be roughly divided into three parts: the extracellular segment (the segment of amino acids 1-271, in which the segment of amino acids 1-20 is a signal peptide), the hydrophobic transmembrane region, and the intracellular segment comprising the C-terminal. When gI is missing, the replication rate of VZV will be greatly slowed down, and the synthesis process of its viral syncytium will also be seriously affected, and thus gI plays an important role in the replication and amplification of VZV in culture models such as Vero cells, T cells, and human skin tissue. In addition, gI, as a molecular chaperone protein, can promote the maturation of gE protein. When the gI gene is knocked out, the gE protein will co-localize with the Golgi marker molecule in the perinuclear region and cannot be normally transported to the cytoplasmic membrane to participate in the assembly of VZV particles.

Current studies on VZV infection and immune mechanisms have shown that potent VZV-specific CD4⁺ T cell immune responses are closely related to protective immunity against herpes zoster. Current clinical studies of Shingrix^{™} vaccine using gE as immunogen have shown that the vaccine can induce gE-specific CD4⁺ T cell responses, which are 10 times or more higher than that of ZOSTAVAX vaccine, and the protection rate in people aged 60 to 60 years is 97.4%, which is higher than the 64% of the ZOSTAVAX vaccine.

However, there is still a need for a VZV vaccine with higher protectiveness in the art.

### Contents of the present disclosure

The present disclosure provides a gE-gI fusion protein, and demonstrates through its preparation and characterization that the gE-gI fusion protein can induce an organism to produce high levels of neutralizing antibodies and cytokines, which is superior to the single gE protein, the blend of gE protein and gI protein, the gE-gI heterodimer and the marketed Shingrix^{™} vaccine, and has a potent immunostimulatory effect. The fusion protein of the present disclosure has the advantage of being a diagnostic antigen or a vaccine candidate in preventing and/or treating a varicella-zoster virus infection.

### Fusion protein

Therefore, in one aspect, the present disclosure provides a fusion protein, comprising a first peptide segment and a second peptide segment, wherein the first peptide segment comprises or is composed of an extracellular region of varicella-zoster virus (VZV) gE protein or a fragment thereof; and the second peptide segment comprises or is composed of an extracellular domain of VZV gI protein or a fragment thereof.

In certain embodiments, the first peptide segment comprises one or more of amino acids at positions 24-71, 163-208 of VZV gE protein; and/or the second peptide segment comprises one or more of amino acids at positions 95, 105-125 of VZV gI protein.

In some embodiments, the first peptide segment comprises at least 100, at least 150, at least 200 or at least 250 consecutive amino acid residues within the amino acid residues of the gE protein at positions corresponding to the positions 150-410 of SEQ ID NO: 11; and/or, the second peptide segment comprises at least 50, at least 100, at least 150 or at least 180 consecutive amino acid residues within the amino acid residues of the gI protein at positions corresponding to the positions 45-240 of SEQ ID NO: 12.

In some embodiments, the first peptide segment comprises the amino acid residues of the gE protein at positions corresponding to the positions 150-410 (or positions 150-417, or positions 150-425, or positions 150-437, or positions 150-445, or positions 150-457, or positions 150-465, or positions 150-477, or positions 150-485, or positions 150-497, or positions 150-507, or positions 150-517, or positions 150-525, or positions 150-537, or positions 143-410, or positions 143-417, or positions 143-425, or positions 143-437, or positions 143-445, or positions 143-457, or positions 143-465, or positions 143-477, or positions 143-485, or positions 143-497, or positions 143-507, or positions 143-517, or positions 143-525, or positions 143-537, or positions 120-410, or positions 120-417, or positions 120-425, or positions 120-437, or positions 120-445, or positions 120-457, or positions 120-465, or positions 120-477, or positions 120-485, or positions 120-497, or positions 120-507, or positions 120-517, or positions 120-525, or positions 120-537, or positions 103-410, or positions 103-417, or positions 103-425, or positions 103-437, or positions 103-445, or positions 103-457, or positions 103-465, or positions 103-477, or positions 103-485, or positions 103-497, or positions 103-507, or positions 103-517, or positions 103-525, or positions 103-537, or positions 90-410, or positions 90-417, or positions 90-425, or positions 90-437, or positions 90-445, or positions 90-457, or positions 90-465, or positions 90-477, or positions 90-485, or positions 90-497, or positions 90-507, or positions 90-517, or positions 90-525, or positions 90-537, or positions 83-410, or positions 83-417, or positions 83-425, or positions 83-437, or positions 83-445, or positions 83-457, or positions 83-465, or positions 83-477, or positions 83-485, or positions 83-497, or positions 83-507, or positions 83-517, or positions 83-525, or positions 83-537, or positions 70-410, or positions 70-417, or positions 70-425, or positions 70-437, or positions 70-445, or positions 70-457, or positions 70-465, or positions 70-477, or positions 70-485, or positions 70-497, or positions 70-507, or positions 70-517, or positions 70-525, or positions 70-537, or positions 63-410, or positions 63-417, or positions 63-425, or positions 63-437, or positions 63-445, or positions 63-457, or positions 63-465, or positions 63-477, or positions 63-485, or positions 63-497, or positions 63-507, or positions 63-517, or positions 63-525, or positions 63-537, or positions 50-410, or positions 50-417, or positions 50-425, or positions 50-437, or positions 50-445, or positions 50-457, or positions 50-465, or positions 50-477, or positions 50-485, or positions 50-497, or positions 50-507, or positions 50-517, or positions 50-525, or positions 50-537, or positions 43-410, or positions 43-417, or positions 43-425, or positions 43-437, or positions 43-445, or positions 43-457, or positions 43-465, or positions 43-477, or positions 43-485, or positions 43-497, or positions 43-507, or positions 43-517, or positions 43-525, or positions 43-537, or positions 31-410, or positions 31-417, or positions 31-425, or positions 31-437, or positions 31-445, or positions 31-457, or positions 31-465, or positions 31-477, or positions 31-485, or positions 31-497, or positions 31-507, or positions 31-517, or positions 31-525, or positions 31-537, or positions 23-410, or positions 23-417, or positions 23-425, or positions 23-437, or positions 23-445, or positions 23-457, or positions 23-465, or positions 23-477, or positions 23-485, or positions 23-497, or positions 23-507, or positions 23-517, or positions 23-525, or positions 23-537) of SEQ ID NO: 11.

In certain embodiments, the first peptide segment comprises or is composed of the amino acid residues of the gE protein at positions corresponding to the positions 150-410 (or positions 150-417, or positions 150-425, or positions 150-437, or positions 150-445, or positions 150-457, or positions 150-465, or positions 150-477, or positions 150-485, or positions 150-497, or positions 150-507, or positions 150-517, or positions 150-525, or positions 150-537, or positions 143-410, or positions 143-417, or positions 143-425, or positions 143-437, or positions 143-445, or positions 143-457, or positions 143-465, or positions 143-477, or positions 143-485, or positions 143-497, or positions 143-507, or positions 143-517, or positions 143-525, or positions 143-537, or positions 120-410, or positions 120-417, or positions 120-425, or positions 120-437, or positions 120-445, or positions 120-457, or positions 120-465, or positions 120-477, or positions 120-485, or positions 120-497, or positions 120-507, or positions 120-517, or positions 120-525, or positions 120-537, or positions 103-410, or positions 103-417, or positions 103-425, or positions 103-437, or positions 103-445, or positions 103-457, or positions 103-465, or positions 103-477, or positions 103-485, or positions 103-497, or positions 103-507, or positions 103-517, or positions 103-525, or positions 103-537, or positions 90-410, or positions 90-417, or positions 90-425, or positions 90-437, or positions 90-445, or positions 90-457, or positions 90-465, or positions 90-477, or positions 90-485, or positions 90-497, or positions 90-507, or positions 90-517, or positions 90-525, or positions 90-537, or positions 83-410, or positions 83-417, or positions 83-425, or positions 83-437, or positions 83-445, or positions 83-457, or positions 83-465, or positions 83-477, or positions 83-485, or positions 83-497, or positions 83-507, or positions 83-517, or positions 83-525, or positions 83-537, or positions 70-410, or positions 70-417, or positions 70-425, or positions 70-437, or positions 70-445, or positions 70-457, or positions 70-465, or positions 70-477, or positions 70-485, or positions 70-497, or positions 70-507, or positions 70-517, or positions 70-525, or positions 70-537, or positions 63-410, or positions 63-417, or positions 63-425, or positions 63-437, or positions 63-445, or positions 63-457, or positions 63-465, or positions 63-477, or positions 63-485, or positions 63-497, or positions 63-507, or positions 63-517, or positions 63-525, or positions 63-537, or positions 50-410, or positions 50-417, or positions 50-425, or positions 50-437, or positions 50-445, or positions 50-457, or positions 50-465, or positions 50-477, or positions 50-485, or positions 50-497, or positions 50-507, or positions 50-517, or positions 50-525, or positions 50-537, or positions 43-410, or positions 43-417, or positions 43-425, or positions 43-437, or positions 43-445, or positions 43-457, or positions 43-465, or positions 43-477, or positions 43-485, or positions 43-497, or positions 43-507, or positions 43-517, or positions 43-525, or positions 43-537, or positions 31-410, or positions 31-417, or positions 31-425, or positions 31-437, or positions 31-445, or positions 31-457, or positions 31-465, or positions 31-477, or positions 31-485, or positions 31-497, or positions 31-507, or positions 31-517, or positions 31-525, or positions 31-537, or positions 23-410, or positions 23-417, or positions 23-425, or positions 23-437, or positions 23-445, or positions 23-457, or positions 23-465, or positions 23-477, or positions 23-485, or positions 23-497, or positions 23-507, or positions 23-517, or positions 23-525, or positions 23-537) of SEQ ID NO: 11.

In certain embodiments, the first peptide segment comprises the amino acid residues of the gE protein at positions corresponding to the positions 70-485 (or positions 70-497, or positions 70-507, or positions 70-517, or positions 70-525, or positions 70-537, or positions 63-485, or positions 63-497, or positions 63-507, or positions 63-517, or positions 63-525, or positions 63-537, or positions 50-485, or positions 50-497, or positions 50-507, or positions 50-517, or positions 50-525, or positions 50-537, or positions 43-485, or positions 43-497, or positions 43-507, or positions 43-517, or positions 43-525, or positions 43-537, or positions 31-485, or positions 31-497, or positions 31-507, or positions 31-517, or positions 31-525, or positions 31-537, or positions 23-485, or positions 23-497, or positions 23-507, or positions 23-517, or positions 23-525, or positions 23-537) of SEQ ID NO: 11.

In certain embodiments, the first peptide segment comprises or is composed of the amino acid residues of the gE protein at positions corresponding to the positions 70-485 (or positions 70-497, or positions 70-507, or positions 70-517, or positions 70-525, or positions 70-537, or positions 63-485, or positions 63-497, or positions 63-507, or positions 63-517, or positions 63-525, or positions 63-537, or positions 50-485, or positions 50-497, or positions 50-507, or positions 50-517, or positions 50-525, or positions 50-537, or positions 43-485, or positions 43-497, or positions 43-507, or positions 43-517, or positions 43-525, or positions 43-537, or positions 31-485, or positions 31-497, or positions 31-507, or positions 31-517, or positions 31-525, or positions 31-537, or positions 23-485, or positions 23-497, or positions 23-507, or positions 23-517, or positions 23-525, or positions 23-537) of SEQ ID NO: 11.

In certain embodiments, the first peptide segment comprises the amino acid residues of the gE protein at positions corresponding to the positions 63-497 (or positions 63-507, or positions 63-517, or positions 63-525, or positions 63-537, or positions 50-497, or positions 50-507, or positions 50-517, or positions 50-525, or positions 50-537, or positions 43-497, or positions 43-507, or positions 43-517, or positions 43-525, or positions 43-537, or positions 31-497, or positions 31-507, or positions 31-517, or positions 31-525, or positions 31-537, or positions 23-497, or positions 23-507, or positions 23-517, or positions 23-525, or positions 23-537) of SEQ ID NO: 11.

In certain embodiments, the first peptide segment comprises or is composed of the amino acid residues of the gE protein at positions corresponding to the positions 63-497 (or positions 63-507, or positions 63-517, or positions 63-525, or positions 63-537, or positions 50-497, or positions 50-507, or positions 50-517, or positions 50-525, or positions 50-537, or positions 43-497, or positions 43-507, or positions 43-517, or positions 43-525, or positions 43-537, or positions 31-497, or positions 31-507, or positions 31-517, or positions 31-525, or positions 31-537, or positions 23-497, or positions 23-507, or positions 23-517, or positions 23-525, or positions 23-537) of SEQ ID NO: 11.

In certain embodiments, the first peptide segment comprises the amino acid residues of the gE protein at positions that correspond the positions 31-537 of SEQ ID NO: 11.

In certain embodiments, the first peptide segment comprises or is composed of the amino acid residues of the gE protein at positions corresponding to the positions 31-537 or positions 23-537 of SEQ ID NO: 11.

In certain embodiments, the first peptide segment is composed of the extracellular region of the gE protein. In some embodiments, the second peptide segment is connected to the C-terminal of the first peptide segment optionally via a linker (e.g., a peptide linker, for example, a peptide linker as set forth in SEQ ID NO: 15), and the first peptide segment is composed of the extracellular region of the gE protein.

In some embodiments, the first peptide segment is composed of the extracellular region of the gE protein that does not contain a signal peptide or contains a partial signal peptide sequence.

In some embodiments, the first peptide segment comprises or is composed of the amino acid residues at positions 23-350 (or positions 23-358, or positions 23-365, or positions 23-375, or positions 23-385, or positions 23-401, or positions 23-410, or positions 23-420, or positions 23-430, or positions 23-440, or positions 23-500, or positions 23-510, or positions 23-520, or positions 23-537, or positions 23-538, or positions 23-539, or positions 23-540, or positions 23-541, or positions 23-542, or positions 23-543, or positions 23-544, or positions 23-545, or positions 23-546) of the VZV gE protein.

In certain embodiments, the first peptide segment comprises or is composed of the amino acid residues at positions 27-350 (or positions 27-358, or positions 27-365, or positions 27-375, or positions 27-385, or positions 27-401, or positions 27-410, or positions 27-420, or positions 27-430, or positions 27-440, or positions 27-500, or positions 27-510, or positions 27-520, or positions 27-537, or positions 27-538, or positions 27-539, or positions 27-540, or positions 27-541, or positions 27-542, or positions 27-543, or positions 27-544, or positions 27-545, or positions 27-546) of the VZV gE protein.

In certain embodiments, the first peptide segment comprises or is composed of the amino acid residues at positions 31-350 (or positions 31-358, or positions 31-365, or positions 31-375, or positions 31-385, or positions 31-401, or positions 31-410, or positions 31-420, or positions 31-430, or positions 31-440, or positions 31-500, or positions 31-510, or positions 31-520, or positions 31-537, or positions 31-538, or positions 31-539, or positions 31-540, or positions 31-541, or positions 31-542, or positions 31-543, or positions 31-544, or positions 31-545, or positions 31-546) of the VZV gE protein.

In some embodiments, the first peptide segment comprises or is composed of the amino acid residues at positions 31-510 of the VZV gE protein.

In some embodiments, the first peptide segment comprises or is composed of amino acid residues at positions 23-537 (or positions 23-538, or positions 23-539, or positions 23-540, or positions 23-541, or positions 23-542, or positions 23-543, or positions 23-544, or positions 23-545, or positions 23-546) of the VZV gE protein.

In some embodiments, the first peptide segment comprises or is composed of the amino acid residues at positions 23-537 of the VZV gE protein.

In certain embodiments, the gE protein has: (a) an amino acid sequence as set forth in any one of SEQ ID NOs: 11, 46-55; (b) an amino acid sequence having an identity of at least 90% (e.g., at least 95%, at least 96%, at least 97%, at least 98%, at least 99%) as compared to the amino acid sequence as set forth in any one of SEQ ID NOs: 11, 46-55; or, (c) a sequence having a substitution (preferably conservative substitution), addition or deletion of one or several (e.g., 1, 2, 3, 4, 5, 6, 7, 8 or 9) amino acids as compared to the amino acid sequence as set forth in any one of SEQ ID NOs: 11, 46-55.

In certain embodiments, the amino acid sequence of the gE protein is set forth in SEQ ID NO: 11.

In certain embodiments, the first peptide segment comprises or is composed of an amino acid sequence as set forth in any one of SEQ ID NOs: 13, 33-42, 64-69, or, a sequence having an identity of at least 90%, such as at least 95%, at least 96%, at least 97%, at least 98%, at least 99%, or, having a substitution (preferably conservative substitution), addition or deletion of one or several (e.g., 1, 2, 3, 4, 5, 6, 7, 8 or 9) amino acids, as compared to the amino acid sequence as set forth in any one of SEQ ID NOs: 13, 33-42, 64-69.

In certain embodiments, the second peptide segment comprises the amino acid residues of the gI protein at positions corresponding to the positions 45-240 (or positions 45-251, or positions 45-260, or positions 45-271, or positions 41-240, or positions 41-251, or positions 41-260, or positions 41-271, or positions 30-240, or positions 30-251, or positions 30-260, or positions 30-271, or positions 21-240, or positions 21-251, or positions 21-260, or positions 21-271) of SEQ ID NO: 12.

In certain embodiments, the second peptide segment comprises or is composed of the amino acid residues of the gI protein at positions corresponding to the positions 45-240 (or positions 45-251, or positions 45-260, or positions 45-271, or positions 41-240, or positions 41-251, or positions 41-260, or positions 41-271, or positions 30-240, or positions 30-251, or positions 30-260, or positions 30-271, or positions 21-240, or positions 21-251, or positions 21-260, or positions 21-271) of SEQ ID NO: 12.

In certain embodiments, the second peptide segment comprises the amino acid residues of the gI protein at positions that correspond to the positions 30-260 (or positions 30-271, or positions 21-260, or positions 21-271) of SEQ ID NO: 12.

In certain embodiments, the second peptide segment comprises or is composed of the amino acid residues of the gI protein at positions corresponding to the positions 30-260 (or positions 30-271, or positions 21-260, or positions 21-271) of SEQ ID NO: 12.

In certain embodiments, the second peptide segment comprises the amino acid residues of the gI protein at positions corresponding to the positions 21-271 of SEQ ID NO: 12.

In certain embodiments, the second peptide segment is composed of the extracellular region of the gI protein. In some embodiments, the second peptide segment is connected to the N-terminal of the first peptide segment optionally through a linker (e.g., a peptide linker, for example, a peptide linker as set forth in SEQ ID NO: 15), and the second peptide segment is composed of the extracellular region of the gI protein.

In some embodiments, the second peptide segment is composed of the extracellular region of the gI protein that does not contain a signal peptide or contains a partial signal peptide sequence.

In some embodiments, the second peptide segment comprises or is composed of the amino acid residues of the gI protein at positions corresponding to the positions 21-271 of SEQ ID NO: 12.

In some embodiments, the second peptide segment comprises or is composed of the amino acid residues at positions 21-220 (or positions 21-230, or positions 21-235, or positions 21-250, or positions 21-260, or positions 21-271) of the VZV gI protein.

In some embodiments, the second peptide segment comprises or is composed of the amino acid residues at positions 30-220 (or positions 30-230, or positions 30-235, or positions 30-250, or positions 30-260, or positions 30-271) of the VZV gI protein.

In some embodiments, the second peptide segment comprises or is composed of the amino acids at positions 36-220 (or positions 36-230, or positions 36-235, or positions 36-250, or positions 36-260, or positions 36-271) of the VZV gI protein.

In certain embodiments, the first peptide segment comprises the amino acids of the gE protein at positions corresponding to the positions 63-497 (or positions 63-507, or positions 63-517, or positions 63-525, or positions 63-537, or positions 50-497, or positions 50-507, or positions 50-517, or positions 50-525, or positions 50-537, or positions 43-497, or positions 43-507, or positions 43-517, or positions 43-525, or positions 43-537, or positions 31-497, or positions 31-507, or positions 31-517, or positions 31-525, or positions 31-537, or positions 23-497, or positions 23-507, or positions 23-517, or positions 23-525, or positions 23-537) of SEQ ID NO: 11; and the second peptide segment comprises the amino acid residues of the gI protein at positions corresponding to the positions 30-260 (or positions 30-271, or positions 21-260, or positions 21-271) of SEQ ID NO: 12.

In certain embodiments, the first peptide segment comprises or is composed of the amino acids of the gE protein at positions corresponding to the positions 63-497 (or positions 63-507, or positions 63-517, or positions 63-525, or positions 63-537, or positions 50-497, or positions 50-507, or positions 50-517, or positions 50-525, or positions 50-537, or positions 43-497, or positions 43-507, or positions 43-517, or positions 43-525, or positions 43-537, or positions 31-497, or positions 31-507, or positions 31-517, or positions 31-525, or positions 31-537, or positions 23-497, or positions 23-507, or positions 23-517, or positions 23-525, or positions 23-537) of SEQ ID NO: 11; and the second peptide segment comprises or is composed of the amino acid residues of the gI protein at positions corresponding to the positions 30-260 (or positions 30-271, or positions 21-260, or positions 21-271) of SEQ ID NO: 12.

In certain embodiments, the gI protein has: (a) an amino acid sequence as set forth in any one of SEQ ID NOs: 12, 56-58; (b) an amino acid sequence having an identity of at least 90% (e.g., at least 95%, at least 96%, at least 97%, at least 98%, at least 99%) as compared to the amino acid sequence as set forth in any one of SEQ ID NOs: 12, 56-58; or, (c) a sequence having a substitution (preferably conservative substitution), addition or deletion of one or several (e.g., 1, 2, 3, 4, 5, 6, 7, 8 or 9) amino acids as compared to the amino acid sequence as set forth in any one of SEQ ID NOs: 12, 56-58.

In certain embodiments, the amino acid sequence of the gI protein is set forth in SEQ ID NO: 12.

In certain embodiments, the second peptide segment comprises or is composed of the amino acid residues at positions 36-235 of the VZV gI protein.

In certain embodiments, the second peptide segment comprises or is composed of the amino acid residues at positions 21-271 of the VZV gI protein.

In certain embodiments, the second peptide segment comprises or is composed of an amino acid sequence as set forth in any one of SEQ ID NOs: 14, 43-45, or, a sequence having an identity of at least 90%, such as at least 95%, at least 96%, at least 97%, at least 98%, at least 99%, or, having a substitution (preferably conservative substitution), addition or deletion of one or several (e.g., 1, 2, 3, 4, 5, 6, 7, 8 or 9) amino acids, as compared to the amino acid sequence as set forth in any one of SEQ ID NOs: 14, 43-45.

In certain embodiments, the second peptide segment is connected to the N-terminal or C-terminal of the first peptide segment optionally via a linker (e.g., a peptide linker, such as a peptide linker as set forth in SEQ ID NO: 15).

In certain embodiments, the linker is a peptide linker comprising one or more (e.g., 5 to 20) flexible amino acids.

In certain embodiments, the linker is a peptide linker comprising one or more glycines and/or one or more serines.

In certain embodiments, the fusion protein comprises or is composed of an amino acid sequence as set forth in any one of SEQ ID NOs: 6-7, 20-32, 59-63, 70.

It is easy for a person skilled in the art to understand that, in order to facilitate protein expression, the fusion protein optionally comprises a signal peptide sequence. The signal peptide sequence may be present in the first peptide segment or the second peptide segment, or the signal peptide sequence may be present outside the first peptide segment and the second peptide segment (e.g., both the first peptide segment and the second peptide segment do not contain a signal peptide sequence, and the fusion protein additionally comprises a signal peptide sequence outside the first peptide segment and the second peptide segment).

In certain embodiments, the second peptide segment is connected to the N-terminal of the first peptide segment optionally via a linker (e.g., a peptide linker, for example, a peptide linker as set forth in SEQ ID NO: 15), and the second peptide segment comprises at its N-terminal a signal peptide sequence (e.g., a signal peptide sequence derived from VZV gI protein). In certain embodiments, the second peptide segment is connected to the C-terminal of the first peptide segment optionally via a linker (e.g., a peptide linker, for example, a peptide linker as set forth in SEQ ID NO: 15), and the first peptide segment comprises at its N-terminal a signal peptide sequence (e.g., a signal peptide sequence derived from a VZV gE protein). In certain embodiments, neither the first peptide segment nor the second peptide segment comprises a signal peptide sequence, and the fusion protein further comprises a signal peptide sequence at its N-terminal.

In certain embodiments, the fusion protein comprises a signal peptide and/or a detectable label (e.g., a tag protein).

In certain embodiments, the fusion protein comprises at its N-terminal a signal peptide (e.g., a natural signal peptide of VZV gE protein, a natural signal peptide of VZV gI protein, a gp67 signal peptide, a melittin signal peptide (MSP), or a tissue-type plasminogen activator signal peptide (tPA signal peptide)).

In certain embodiments, the fusion protein comprises at its C-terminal a detectable label.

In another aspect, the present disclosure also provides an RNA molecule, comprising a nucleotide sequence encoding the fusion protein as described above.

In some embodiments, the nucleotide sequence encoding the fusion protein is codon-optimized or not optimized according to the codon preference of a host cell (e.g., an insect cell or a mammalian cell).

In some embodiments, the RNA molecule further comprises one or more selected from the group consisting of 5'UTR, Kozak sequence, start codon, stop codon, 3'UTR, poly-A tail.

In some embodiments, the RNA molecule comprises, from 5' to 3': 5'UTR, Kozak sequence, start codon, nucleotide sequence encoding the fusion protein, stop codon, 3'UTR, poly-A tail.

In certain embodiments, the RNA molecule has one or more of the following features:
(1) the nucleotide sequence encoding the fusion protein has a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the nucleotide sequence as set forth in SEQ ID NO: 71; in certain embodiments, the nucleotide sequence encoding the fusion protein is set forth in SEQ ID NO: 71;
(2) there are one or more stop codons at the 3' end of the nucleotide sequence encoding the fusion protein;
(3) the 5'UTR has a nucleotide sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the nucleotide sequence as set forth in SEQ ID NO: 17; in certain embodiments, the 5'UTR has a nucleotide sequence as set forth in SEQ ID NO: 17;
(4) the 3'UTR has a nucleotide sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the nucleotide sequence as set forth in SEQ ID NO: 18; in certain embodiments, the 3'UTR has a nucleotide sequence as set forth in SEQ ID NO: 18;
(5) the Kozak sequence is set forth in SEQ ID NO: 72;
(6) the poly-A tail comprises one or more polyadenylic acid sequences, each of the one or more polyadenylic acid sequences is independently composed of 20 to 120 consecutive adenylic acids; preferably, the poly-A tail comprises a plurality of polyadenylic acid sequences, and the adjacent polyadenylic acid sequences are connected by a non-A spacer sequence; preferably, the poly-A tail has a nucleotide sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 19; in some embodiments, the poly-A tail has a nucleotide sequence as set forth in SEQ ID NO: 19;
(7) the 5' end of the RNA molecule is modified with a 5' cap (e.g., CAP-0, CAP-1, CAP-2).

In another aspect, the present disclosure also provides an isolated nucleic acid molecule, which comprises a nucleotide sequence encoding the fusion protein as described above or the RNA molecule as described above.

In some embodiments, the nucleotide sequence encoding the fusion protein is codon-optimized or not optimized according to the codon preference of a host cell (e.g., insect cell or mammalian cell).

In another aspect, the present disclosure also provides a vector, which comprises the isolated nucleic acid molecule as described above.

In another aspect, the present disclosure provides a delivery composition, which comprises a delivery carrier, and the RNA molecule, isolated nucleic acid molecule or vector as described above.

In certain embodiments, the delivery carrier is selected from the group consisting of lipid particle, sugar particle, metal particle, protein particle, liposome, exosome, microvesicle and viral vector (e.g., replication-defective retrovirus, lentivirus, adenovirus or adeno-associated virus).

In certain embodiments, the delivery composition comprises the RNA molecule as described above.

In certain embodiments, the delivery carrier is a lipid nanoparticle (LNP).

In certain embodiments, the lipid nanoparticle comprises lipids ALC-0315, ALC-0519, DSPC (1,2-distearoyl-sn-glycero-3-phosphocholine) and cholesterol.

In certain embodiments, the molar percentage of ALC-0315 in the lipid nanoparticle is in the range of 40% to 55% (e.g., 40% to 46.3%, 40% to 48%, 40% to 50%, 40% to 52%, 43% to 46.3%, 43% to 58%, 43% to 50%, 43% to 52%, 43% to 55%). In certain embodiments, the molar percentage of ALC-0315 in the lipid nanoparticle is 46.3%.

In certain embodiments, the molar percentage of cholesterol in the lipid nanoparticle is in the range of 35% to 50% (e.g., 35% to 42.7%, 35% to 45%, 40% to 42.7%, 40% to 45%, 40% to 50%). In certain embodiments, the molar percentage of cholesterol in the lipid nanoparticle is 42.7%.

In some embodiments, the molar percentage of DSPC in the lipid nanoparticle is in the range of 5% to 12% (e.g., 5% to 9.4%, 5% to 10%, 5% to 11%, 8% to 9.4%, 8% to 10%, 8% to 11%, 8% to 12%, 9% to 9.4%, 9% to 10%, 9% to 11%, 9% to 12%). In some embodiments, the molar percentage of DSPC in the lipid nanoparticle is 46.3%.

In some embodiments, the molar percentage of ALC-0519 in the lipid nanoparticle is in the range of 1.0% to 2.0% (e.g., 1.0% to 1.6%, 1.0% to 1.8%, 1.3% to 1.6%, 1.3% to 1.8%, 1.3% to 2.0%). In some embodiments, the molar percentage of ALC-0519 in the lipid nanoparticle is 1.6%.

In some embodiments, the delivery composition is a vaccine (e.g., a nucleic acid vaccine).

In another aspect, the present disclosure also provides a host cell, which comprises the RNA molecule, isolated nucleic acid molecule or vector as described above.

In certain embodiments, the host cell is selected from the group consisting of prokaryotic cell (e.g., *E. coli* cell) and eukaryotic cell (e.g., yeast cell, insect cell, plant cell, mammalian cell).

In certain embodiments, the host cell is an insect cell.

In certain embodiments, the host cell is a mammalian cell.

In another aspect, the present disclosure also provides a method for preparing the fusion protein as described above, comprising culturing the host cell as described above under a condition that allows protein expression, and recovering the fusion protein from a cell culture.

### Therapeutic Use

In another aspect, the present disclosure also provides an immunogenic composition, which comprises the fusion protein, RNA molecule, isolated nucleic acid molecule, vector or delivery composition as described above, and optionally a pharmaceutically acceptable carrier and/or excipient (e.g., adjuvant).

In certain embodiments, the immunogenic composition comprises the fusion protein, RNA molecule, isolated nucleic acid molecule, vector or delivery composition as described above and an adjuvant, wherein the adjuvant is selected from the group consisting of: aluminum salt adjuvant, zinc-aluminum hybrid adjuvant (e.g., FH002C), Freund's adjuvant, oil emulsion adjuvant (e.g., MF59 adjuvant), cytokine, TLR agonist, CpG adjuvant, nucleic acid adjuvant, liposome, saponin adjuvant, AS01B adjuvant and any combination thereof.

In certain embodiments, the immunogenic composition comprises the fusion protein or delivery composition as described above, and the adjuvant.

In certain embodiments, the immunogenic composition comprises the fusion protein as described above, and the AS01B adjuvant.

In certain embodiments, the immunogenic composition comprises the delivery composition as described above, and the CpG adjuvant.

In certain embodiments, the immunogenic composition is a vaccine.

The immunogenic composition as described above can be formulated into any dosage form known in the medical field, for example, tablet, pill, suspension, emulsion, solution, gel, capsule, powder, granule, elixir, lozenge, suppository, injection (including solution for injection, sterile powder for injection, and concentrated solution for injection), inhalant, spray, etc. The preferred dosage form depends on the intended mode of administration and therapeutic use. The immunogenic composition of the present disclosure should be sterile and stable under production and storage conditions. A preferred dosage form is an injection. Such an injection can be a sterile solution for injection. In addition, the sterile solution for injection can be prepared as a sterile lyophilized powder (e.g., by vacuum drying or freeze drying) for storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., a solution containing 0.01% polysorbate 20), pH buffer solution (e.g., phosphate buffer solution), Ringer's solution, and any combination thereof.

The immunogenic compositions described above can be administered by any suitable method known in the art, including, but not limited to, oral, buccal, sublingual, ocular, local, parenteral, rectal, intrathecal, intracytoplasmic reticulum, inguinal, intravesical, topical (e.g., powder, ointment or drop), or nasal route. However, for many therapeutic uses, the preferred route/mode of administration is parenteral administration (e.g., intravenous or bolus, subcutaneous, intraperitoneal, intramuscular). The skilled artisan will appreciate that the route and/or mode of administration will vary depending on the intended purpose.

The immunogenic composition as described above should be administered in an amount sufficient to induce an immune response against VZV. The appropriate amount of the immunogen can be determined based on the specific disease or condition to be treated or prevented, the severity, the age of the subject, and other personal attributes of the specific subject (e.g., the general state of health of the subject and the robustness of the subject's immune system). The determination of the effective dose is also guided by animal model studies, followed by human clinical trials, and by an administration regimen that significantly reduces the occurrence or severity of the target disease symptoms or conditions in the subject.

In another aspect, the present disclosure also provides the use of the fusion protein, or RNA molecule, or isolated nucleic acid molecule, or vector, or delivery composition, or host cell, or immunogenic composition as described above in the manufacture of an immunogenic composition for inducing an immune response against VZV in a subject and/or for preventing and/or treating a VZV infection or a disease associated with VZV infection in a subject.

In certain embodiments, the immunogenic composition is a vaccine.

In certain embodiments, the VZV infection is a primary infection or recurrent infection of VZV.

In certain embodiments, the disease associated with VZV infection is selected from the group consisting of: herpes zoster, varicella, and complication thereof (e.g., postherpetic neuralgia, pneumonia, encephalomyelitis, conjunctivitis).

In certain embodiments, the subject is a mammal, such as a human.

In another aspect, the present disclosure also provides a method for inducing an immune response against VZV in a subject and/or for preventing and/or treating a VZV infection or a disease associated with VZV infection in a subject, comprising: administering an effective amount of the fusion protein, RNA molecule, isolated nucleic acid molecule, vector, delivery composition, host cell or immunogenic composition as described above to the subject in need thereof.

In certain embodiments, the VZV infection is a primary infection or recurrent infection of VZV.

In certain embodiments, the disease associated with VZV infection is selected from the group consisting of: herpes zoster, varicella, and complication thereof (e.g., postherpetic neuralgia, pneumonia, encephalomyelitis, conjunctivitis).

In certain embodiments, the subject is a mammal, such as a human.

### Detection Use

In another aspect, the present disclosure also provides a method for detecting the presence of VZV gI protein-specific antibody and/or VZV gE protein-specific antibody in a sample, which comprises using the fusion protein as described above.

In certain embodiments, the method is an immunological detection, such as immunoblotting, enzyme immunoassay (e.g., ELISA), chemiluminescent immunoassay, fluorescent immunoassay or radioimmunoassay.

In certain embodiments, the method comprises: (1) contacting the sample with the fusion protein as described above; (2) detecting the formation of a fusion protein-antibody immune complex or detecting the amount of the immune complex; in which the formation of the immune complex indicates the presence of VZV gI protein-specific antibody and/or VZV gE protein-specific antibody in the sample.

In certain embodiments, the method further comprises using a second antibody with a detectable label (e.g., an enzyme (e.g., horseradish peroxidase or alkaline phosphatase), a chemiluminescent agent (e.g., acridinium ester compound, luminol and derivative thereof, or ruthenium derivative), a fluorescent dye (e.g., fluorescein or fluorescent protein), a radionuclide, or biotin) to detect the presence of VZV gI protein-specific antibody and/or VZV gE protein-specific antibody in the sample.

In certain embodiments, the second antibody is specific for a constant region contained in an antibody of a species (e.g., human) from which the sample to be tested is derived.

In certain embodiments, the second antibody is an anti-immunoglobulin (e.g., human immunoglobulin) antibody, such as an anti-IgG antibody.

In certain embodiments, the sample is a body fluid sample (e.g., whole blood, plasma, serum, salivary excreta, or urine) from a subject (e.g., a mammal, preferably a human).

In certain embodiments, the method is used for diagnostic purpose or non-diagnostic purpose. In certain embodiments, the method is used for non-diagnostic purpose.

In another aspect, the present disclosure also provides the use of the fusion protein, or RNA molecule, or isolated nucleic acid molecule, or vector, or delivery composition, or host cell as described above in the manufacture of a detection reagent, the detection reagent is used to detect the presence of VZV gI protein-specific antibody and/or VZV gE protein-specific antibody in a sample.

In certain embodiments, the detection reagent detects the presence of VZV gI protein-specific antibody and/or VZV gE protein-specific antibody in a sample by the method as described above.

In certain embodiments, the sample is a body fluid sample (e.g., whole blood, plasma, serum, saliva or urine) from a subject (e.g., a mammal, preferably a human).

### Kit

In another aspect, the present disclosure also provides a kit, which comprises the fusion protein, or RNA molecule, or isolated nucleic acid molecule, or vector, or delivery composition, or host cell as described above.

In certain embodiments, the kit comprises the fusion protein as described above, and a second antibody, wherein the second antibody is as defined above.

### Definition of Terms

In the present disclosure, unless otherwise specified, the scientific and technical terms used herein have the meanings commonly understood by those skilled in the art. In addition, the virology, biochemistry, and immunology laboratory operation steps used herein are all routine steps widely used in the corresponding fields. At the same time, in order to better understand the present disclosure, the definitions and explanations of relevant terms are provided below.

When the terms "for example", "e.g.", "such as", "including", "comprising" or their variations are used herein, these terms will not be considered as restrictive terms, but will be interpreted as meaning "but not limited to" or "not limited to".

Unless otherwise specified herein or clearly contradicted by the context, the terms "a", "an" and "the" and similar referents should be interpreted as covering the singular and plural in the context of describing the present disclosure (especially in the context of the following claims).

As used herein, the term "VZV" is an abbreviation for "varicella-zoster virus", which is a DNA virus.

As used herein, the terms "gE protein", "gE", and "glycoprotein E" refer to a type of envelope glycoprotein of VZV, which have the same meaning and can be used interchangeably. The specific amino acid sequence of the wild-type gE protein can be obtained from a public database (e.g., GenBank database), for example, the amino acid sequence shown in GenBank accession number AB097933 (pOka strain), AB097932 (vOka strain), NC001348 (Dumas strain), AY548170 (MSP strain), QCA45176.1, AEW89412.1, ANS12941.1, QCA43570.1, QWE79571.1, AEW89124.1, AQT34120.1, or AEW88980.1.

In the present disclosure, when referring to the amino acid sequence of gE protein, it is described with reference to the sequence as set forth in SEQ ID NO: 11. For example, the expression "amino acid residues in the gE protein at positions corresponding to the positions 150-410 of SEQ ID NO: 11" refers to the amino acid sites/residues in a sequence that are at positions equivalent to the positions of the amino acid residues 150-410 of SEQ ID NO: 11 when the sequence is optimally aligned with SEQ ID NO: 11, i.e., when the sequence is aligned with SEQ ID NO: 11 to obtain the highest percentage identity.

Those skilled in the art understand that VZV may include a plurality of isolates, and there may be differences between the amino acid sequences of gE proteins of various isolates. Further, those skilled in the art understand that, despite possible sequence differences, the gE proteins of different isolates of VZV have extremely high identity (usually greater than 95%, such as greater than 96%, greater than 97%, greater than 98%, or greater than 99%) in amino acid sequence and have substantially the same biological function. Therefore, in the present disclosure, the term "gE protein" includes not only the protein as set forth in any one of SEQ ID NOs: 11, 46-55, but also the gE proteins of various VZV isolates. And, when describing the fragment of the gE protein, it includes not only the fragment of any one of SEQ ID NOs: 11, 46-55, but also the corresponding fragments in the gE proteins of various VZV isolates. For example, the expression "amino acid residues at positions 23-537 of gE protein" includes amino acid residues at positions 23-537 of any one of SEQ ID NOs: 11, 46-55, and the corresponding fragments in the gE proteins of various VZV isolates.

As used herein, "gE protein extracellular region" refers to the part of the gE protein that does not include the intracellular region and the transmembrane region. For example, from the analysis of specific sequence, in some embodiments, the gE protein extracellular region refers to a region in the gE protein that contains at least the amino acid residues at positions corresponding to the amino acid positions 1-537 of SEQ ID NO: 11. At present, studies have also shown that the amino acid residues in the gE protein at positions corresponding to the amino acid positions 1-546 of SEQ ID NO: 11 can also be used as the extracellular region to perform the corresponding functions of the extracellular region of gE protein. Therefore, in certain embodiments, the extracellular region of gE protein refers to the amino acid residues in the gE protein at positions corresponding to the amino acid positions 1-546 (or positions 1-538, or positions 1-539, or positions 1-540, or positions 1-541, or positions 1-542, or positions 1-543, or positions 1-544, or positions 1-545) of SEQ ID NO: 11. It is understood by those skilled in the art that a mutation or variation (including but not limited to, substitution, deletion and/or addition) can be naturally generated or artificially introduced into SEQ ID NO: 11 without affecting the biological properties of the gE protein. For example, the gE proteins of different strains of VZV may naturally differ in amino acid sequence, but have substantially the same biological properties. Therefore, when describing the extracellular region of the gE protein, it includes not only the amino acid residues at positions 1-537 (or positions 1-538, or positions 1-539, or positions 1-540, or positions 1-541, or positions 1-542, or positions 1-543, or positions 1-544, or positions 1-545, or positions 1-546) of SEQ ID NO: 11, but also the amino acid residues in a natural or artificial variant of SEQ ID NO: 11 (e.g., the gE protein as set forth in any one of SEQ ID NOs: 46-55) that are at positions corresponding to the amino acid positions 1-537 (or positions 1-538, or positions 1-539, or positions 1-540, or positions 1-541, or positions 1-542, or positions 1-543, or positions 1-544, or positions 1-545, or positions 1-546) of SEQ ID NO: 11.

As used herein, the terms "gI protein", "gI", and "glycoprotein I" refer to an envelope glycoprotein of VZV, and they have the same meaning and can be used interchangeably. Wherein, the specific amino acid sequence of wild-type gI protein can be obtained from a public database (e.g., GenBank database), for example, can be the amino acid sequence as shown in GenBank accession number AB097933 (pOka strain), AB097932 (vOka strain), NC001348 (Dumas strain), AY548170 (MSP strain), AEW89483.1, AGY34059.1 or AEW89051.1.

In the present disclosure, when referring to the amino acid sequence of gI protein, it is described with reference to the sequence as set forth in SEQ ID NO: 12. For example, the expression "amino acid residues in gI protein at positions corresponding to positions 45-240 of SEQ ID NO: 12" refers to the amino acid sites/residues in a sequence that are at positions equivalent to the positions of amino acid residues 45-240 of SEQ ID NO: 12 when the sequence is optimally aligned with SEQ ID NO: 12, i.e., when the sequence is aligned with SEQ ID NO: 12 to obtain the highest percentage identity.

Those skilled in the art understand that VZV may include a plurality of isolates, and there may be differences between the amino acid sequences of gI proteins of various isolates. Further, those skilled in the art understand that, despite possible sequence differences, gI proteins of different isolates of VZV have extremely high identity (usually greater than 95%, such as greater than 96%, greater than 97%, greater than 98%, greater than 99%, greater than 99.5%, or greater than 99.8%) in amino acid sequence, and have substantially the same biological function. Therefore, in the present disclosure, the term "gI protein" includes not only the protein as set forth in any one of SEQ ID NOs: 12 and 56-58, but also the gI proteins of various VZV isolates. Moreover, when describing the fragment of the gI protein, it includes not only the fragment of any one of SEQ ID NOs: 12 and 56-58, but also the corresponding fragments in the gI proteins of various VZV isolates. For example, the expression "amino acid residues at positions 21-271 of gI protein" includes amino acid residues at positions 21-271 of any one of SEQ ID NOs: 12 and 56-58, and the corresponding fragments in the gI proteins of various VZV isolates.

As used herein, the "extracellular region of gI protein" refers to the part of gI protein that does not include the intracellular region and the transmembrane region. For example, from the analysis of the specific sequence, in some embodiments, the extracellular region of gI protein refers to the amino acid residues in the gI protein that are at positions corresponding the positions 1-271 of SEQ ID NO: 12. Those skilled in the art understand that a mutation or variation (including but not limited to substitution, deletion and/or addition) may be naturally or artificially introduced into SEQ ID NO: 12 without affecting the biological properties of the gI protein. For example, the gI proteins of different strains of VZV may differ naturally in amino acid sequence, but have substantially the same biological properties. Therefore, when describing the extracellular region of the gI protein, it includes not only the amino acid residues at positions 1-271 of SEQ ID NO: 12, but also the amino acid residues in the natural or artificial variants of SEQ ID NO: 12 (e.g., the gI protein as set forth in any one of SEQ ID NOs: 56-58) that are at positions corresponding to the positions 1-271 of SEQ ID NO: 12.

According to the present disclosure, the expression "corresponding fragment" or "corresponding amino acid position" refers to the fragments or amino acid sites/residues at equivalent positions in sequences being compared when the sequences are optimally aligned, i.e., when the sequences are aligned to obtain the highest percentage identity.

As used herein, the term "identity" is used to refer to the matching of sequences between two polypeptides or between two nucleic acids. When a position in both sequences being compared is occupied by the same nucleotide or amino acid residue (e.g., a position in each of the two DNA molecules is occupied by adenine nucleotide, or a position in each of the two polypeptides is occupied by lysine), then the molecules are identical at that position. The "percent identity" between two sequences is a function of the number of matching positions shared by the two sequences divided by the number of positions compared × 100. For example, if 6 out of 10 positions of the two sequences match, then the two sequences have an identity of 60%. For example, the DNA sequences CTGACT and CAGGTT have an identity of 50% (3 out of a total of 6 positions match). Typically, the comparison is made when the two sequences are aligned to produce maximum identity. Such an alignment can be achieved by using, for example, the method of Needleman et al. (1970) J. Mol. Biol. 48: 443-453, which can be conveniently performed by a computer program such as the Align program (DNAstar, Inc.). The percent identity between two amino acid sequences can also be determined using the algorithm of E. Meyers and W. Miller (Comput. Appl Biosci., 4: 11-17 (1988)) which has been incorporated into the ALIGN program (version 2.0) using a PAM120 weight residue table, a gap length penalty of 12, and a gap penalty of 4. In addition, the percent identity between two amino acid sequences can be determined using the algorithm of Needleman and Wunsch (J MoI Biol. 48: 444-453 (1970)) which has been incorporated into the GAP program of the GCG software package (available at www.gcg.com) using a Blossum 62 matrix or a PAM250 matrix and a gap weight of 16, 14, 12, 10, 8, 6, or 4 and a length weight of 1, 2, 3, 4, 5, or 6.

As used herein, the term "conservative substitution" refers to an amino acid substitution that does not adversely affect or change the expected properties of the protein/polypeptide comprising the amino acid sequence. For example, a conservative substitution can be introduced by standard techniques known in the art such as site-directed mutagenesis and PCR-mediated mutagenesis. Conservative amino acid substitutions include substitutions of amino acid residues with amino acid residues having similar side chains, such as substitutions with residues physically or functionally similar to the corresponding amino acid residues (e.g., having similar size, shape, charge, chemical properties, including the ability to form covalent bond or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative amino acid substitutions are well known in the art (see, for example, Brummell et al., Biochem. 32: 1180-1187 (1993); Kobayashi et al. Protein Eng. 12(10): 879-884 (1999); and Burks et al. Proc. Natl Acad. Set USA 94: 412-417 (1997), which are incorporated herein by reference).

As used herein, the term "vector" refers to a nucleic acid delivery vehicle into which a polynucleotide can be inserted. When a vector is capable of expressing a protein encoded by the inserted polynucleotide, the vector is called an expression vector. The vector can be introduced into a host cell by transformation, transduction or transfection so that the genetic material elements it carries are expressed in the host cell. Vectors are well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); bacteriophage such as λ phage or M13 phage and animal virus, etc. Animal viruses that can be used as vectors include but are not limited to retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including but not limited to promoter sequence, transcription start sequence, enhancer sequence, selection element and reporter gene. In addition, the vector may also contain an origin of replication.

As used herein, the term "host cell" refers to a cell that can be used to introduce a vector, including but not limited to prokaryotic cell such as *Escherichia coli* or *Bacillus subtilis,* fungal cell such as yeast cell or *Aspergillus,* insect cell such as *S2 Drosophila* cell or *Sf9,* or animal cell such as fibroblast, CHO cell, COS cell, NSO cell, HeLa cell, BHK cell, HEK 293 cell or human cell.

Those skilled in the art will understand that the design of the expression vector may depend on factors such as the choice of the host cell to be transformed, the desired expression level, etc. A vector can be introduced into a host cell to produce a transcript, protein, or peptide, including the protein, isolated nucleic acid molecule and so on as described herein.

According to the present disclosure, the term "pharmaceutically acceptable carrier and/or excipient" refers to a carrier and/or excipient that is pharmacologically and/or physiologically compatible with a subject and an active ingredient, which is well known in the art (see, for example, Remington's Pharmaceutical Sciences. Edited by Gennaro AR, 19th ed. Pennsylvania: Mack Publishing Company, 1995), and includes but is not limited to: pH regulator, surfactant, adjuvant, ionic strength enhancer. For example, the pH regulator includes but is not limited to phosphate buffer; the surfactant includes but is not limited to cationic, anionic or non-ionic surfactant, such as Tween-80; the adjuvant includes but is not limited to aluminum salt adjuvant, zinc-aluminum hybrid adjuvant (e.g., FH002C), Freund's adjuvant, oil emulsion adjuvant, cytokine, TLR agonist, CpG adjuvant, liposome, AS01B adjuvant or combination thereof; the ionic strength enhancer includes but is not limited to sodium chloride.

According to the present disclosure, the term "adjuvant" refers to a non-specific immunopotentiator, which can enhance the body's immune response to an antigen or change the type of immune response when it is delivered to the body together with the antigen or in advance. There are many kinds of adjuvants, including but not limited to aluminum salt adjuvant, zinc aluminum hybrid adjuvant (e.g., FH002C), Freund's adjuvant, oil emulsion adjuvant (e.g., MF59 adjuvant), cytokine, TLR agonist, CpG adjuvant, liposome, AS01B adjuvant or combination thereof. In the present disclosure, it is particularly preferred that the adjuvant is AS01B adjuvant, CpG adjuvant or any combination thereof.

According to the present disclosure, the term "effective amount" refers to an amount that can effectively achieve the intended purpose. For example, an effective amount for preventing or treating a disease (e.g., VZV infection) refers to an amount that can effectively prevent, arrest or delay the occurrence of a disease (e.g., VZV infection), or alleviate, reduce or treat the severity of an existing disease (e.g., a disease caused by VZV infection). Determining such an effective amount is within the capabilities of those skilled in the art. For example, the amount effective for therapeutic use will depend on the severity of the disease to be treated, the overall state of the patient's own immune system, the patient's general condition such as age, weight and gender, the mode of administering drug, and other treatment administered at the same time, etc.

In the present disclosure, the terms "polypeptide" and "protein" have the same meaning and can be used interchangeably. And in the present disclosure, amino acids are generally represented by single-letter and three-letter abbreviations known in the art. For example, alanine can be represented by A or Ala.

As used herein, the term "subject" refers to an animal, such as a vertebrate. Preferably, the subject is a mammal, such as a human, bovine, equine, feline, canine, rodent or primate. Particularly preferably, the subject is a human. As used herein, the term can be used interchangeably with "patient".

### Beneficial effects of the disclosure

The VZV gE-gI fusion protein, as disclosed herein, exhibits good reactivity with both gE-specific mouse antibodies and gI-specific mouse monoclonal antibodies. Different from the reported gE-gI dimer, the gE-gI fusion protein is capable of being expressed through a single plasmid and a single clone. It concurrently retains the properties of both gE and gI proteins, and can be used as a detection antigen for diagnostic reagents and an immunogen for vaccines, showing its promising application prospects and values in the diagnosis of varicella-zoster virus.

In particular, compared with the single gE protein, the blend of gE protein and gI protein, the gE-gI heterodimer, and the marketed Shingrix^{™} vaccine, the gE-gI fusion protein of the present disclosure has significantly excellent activity in induction of neutralizing antibodies and T cell response, showing its encouraging application prospects and values in the prevention and/or treatment of herpes zoster.

The embodiments of the present disclosure will be described in detail below in conjunction with the drawings and examples, but those skilled in the art will understand that the following drawings and examples are only used to illustrate the present disclosure, rather than to limit the scope of the present disclosure. According to the following detailed description of the drawings and preferred embodiments, the various objects and advantages of the present disclosure will become apparent to those skilled in the art.

### Brief Description of the Drawings

Fig. 1 shows the results of SDS polyacrylamide gel electrophoresis (SDS-PAGE) (left panel) and Western Blot (right panel) of the gE-gI fusion protein samples derived from insect cells at various purification stages (after purification by sequential chromatography on Q-FF column (Panel A), on DEAE-FF column (Panel B) and on Butyl column (Panel C)) in Example 1, therein lane M: molecular weight marker; lane 1: gE-gI fusion protein.
Fig. 2 shows the results of SDS polyacrylamide gel electrophoresis (left panel) and Western Blot (right panel) of the gE-gI fusion protein samples derived from CHO cells at various purification stages (after sequential purification by chromatography on Ni-6FF column (Panel A), and size exclusion chromatography on Superdex200 Increase (Panel B)) in Example 1, therein lane M: molecular weight marker; lane 1: gE-gI fusion protein.
Fig. 3 shows the results of high-performance size-exclusion chromatography (HPSEC) of the gE-gI fusion protein derived from insect cells in Example 2.
Fig. 4 shows the results of high-performance size-exclusion chromatography (HPSEC) of the gE-gI fusion protein derived from CHO cells in Example 2.
Fig. 5 shows the results of analytical ultracentrifugation of the gE-gI fusion protein derived from insect cells in Example 3.
Fig. 6 shows the results of analytical ultracentrifugation of the gE-gI fusion protein derived from CHO cells in Example 3.
Fig. 7 shows the results of enzyme-linked immunosorbent assay of the gE-gI fusion protein derived from insect cells with gE protein-specific antibodies (6G7, 6H6, 9H9, 10H6, 11B11, 11B12, 11E3, 12E12, 13B6, 14G1, 17B7) and gI protein-specific antibodies (12D2, 13B2, 14B11, 18B2, 19F2) in Example 4.
Fig. 8 shows the results of enzyme-linked immunosorbent assay of the gE-gI fusion protein derived from CHO cells with gE protein-specific antibodies (1B11, 4G4, 6G7, 6H6, 11B11, 11B12, 11E3, 14G1) and gI protein-specific antibodies (7E7, 18B2, 9D11, 16F1, 13B2) in Example 4.
Fig. 9 shows the antigen-specific IgG titers in mouse serum induced by the gE-gI fusion protein with FH002C adjuvant, Freund's complete adjuvant (Freund's) and aluminum adjuvant (Al) in Example 5, respectively.
Fig. 10 shows the neutralizing antibody titers in mouse serum induced by the gE-gI fusion protein with FH002C adjuvant, Freund's complete adjuvant (Freund's) and aluminum adjuvant (Al), as detected by Elispot assay and plaque assay in Example 5, respectively.
Fig. 11 shows the T cell immune response induced by the saline blank without gE-gI fusion protein (Saline), and by the gE-gI fusion protein combined with FH002C adjuvant, Freund's complete adjuvant (Freund's) and aluminum adjuvant (Al) respectively, in Example 6, therein the left panel shows the proportion of induced IL2⁺ CD4⁺ T cells, and the right panel shows the proportion of induced IFNγ⁺ CD4⁺ T cells.
Fig. 12 shows the binding antibody titers and neutralizing antibody titers induced by two different vaccines (the vaccine with insect cell-derived gE-gI fusion protein and AS01B adjuvant, and the Shingrix vaccine) after two injections in Example 7.
Fig. 13 shows the CD4⁺ T cell responses stimulated by the saline blank without gE-gI fusion protein (Saline), and by two different vaccines (the vaccine with insect cell-derived gE-gI fusion protein and AS01B adjuvant, and the Shingrix vaccine) in Example 7, therein the left panel shows the proportion of induced IFNγ⁺ CD4⁺ T cells, and the right panel shows the proportion of induced IL2⁺ CD4⁺ T cells.
Fig. 14 shows the CD8⁺ T cell responses stimulated by the saline blank without gE-gI fusion protein (Saline), and by two different vaccines (the vaccine with insect cell-derived gE-gI fusion protein and AS01B adjuvant, and the Shingrix vaccine) in Example 7, therein the left panel shows the proportion of induced IFNγ⁺ CD8⁺ T cells, and the right panel shows the proportion of induced IL2⁺ CD8⁺ T cells.
Fig. 15 shows the levels of IFN-γ (left panel) and IL-2 (right panel) stimulated by the saline blank without gE-gI fusion protein (Saline), and by two different vaccines (the vaccine with insect cell-derived gE-gI fusion protein and AS01B adjuvant, and the Shingrix vaccine), as detected by Elispot assay in Example 7.
Fig. 16 shows the binding antibody titers (left panel), curves of binding antibody titers (middle panel) and neutralizing antibody titers (right panel) induced by two different vaccines (the vaccine with CHO-derived gE-gI fusion protein and AS01B adjuvant, and the Shingrix vaccine) after two injections in Example 8.
Fig. 17 shows the CD4⁺ T cell responses stimulated by the saline blank without gE-gI fusion protein (Saline), and by two different vaccines (the vaccine with CHO-derived gE-gI fusion protein and AS01B adjuvant, and the Shingrix vaccine) in Example 8, therein the left panel shows the proportion of induced IFNγ⁺ CD4⁺ T cells, and the right panel shows the proportion of induced IL2⁺ CD4⁺ T cells.
Fig. 18 shows the CD8⁺ T cell responses stimulated by the saline blank without gE-gI fusion protein (Saline), and by two different vaccines (the vaccine with CHO-derived gE-gI fusion protein and AS01B adjuvant, and the Shingrix vaccine) in Example 8, therein the left panel shows the proportion of induced IFNγ⁺ CD8⁺ T cells, and the right panel shows the proportion of induced IL2⁺ CD8⁺ T cells.
Fig. 19 shows the levels of IFN-γ (left panel) and IL-2 (right panel) induced by the saline blank without gE-gI fusion protein (Saline), and by two different vaccines (the vaccine with CHO-derived gE-gI fusion protein and AS01B adjuvant, and the Shingrix vaccine), as detected by Elispot assay in Example 8.
Fig. 20 shows the binding antibody IgG titers induced by different forms of gE-gI protein (gE protein alone, gE protein + gI protein, gE-gI fusion protein, and co-expressed gE-gI heterodimer) combined with AS01B-like adjuvant in Example 9, therein the left panel shows the antibody titers on the third week, and the right panel shows the antibody titers on the fifth week.
Fig. 21 shows the T cell responses induced by different forms of gE-gI protein (gE protein alone, gE protein + gI protein, gE-gI fusion protein, and co-expressed gE-gI heterodimer) combined with AS01B-like adjuvant, as detected by flow cytometry in Example 9, therein, the upper left panel shows the proportion of induced IFNγ⁺ CD4⁺ T cells, the upper right panel shows the proportion of induced IL2⁺ CD4⁺ T cells, the lower left panel shows the proportion of induced IFNγ⁺ CD8⁺ T cells, and the lower right panel shows the proportion of induced IL2⁺ CD8⁺ T cells.
Fig. 22 shows the levels of IFN-γ (left panel) and IL-2 (right panel) induced by different forms of gE-gI protein (gE protein alone, gE protein + gI protein, gE-gI fusion protein, and co-expressed gE-gI heterodimer) combined with AS01B-like adjuvant, as detected by Elispot in Example 9.
Fig. 23 shows the induced antigen-specific IgG titers after 2 injections of the fusion proteins constructed based on different mutants in Example 10.
Fig. 24 shows the induced antigen-specific CD4⁺ and CD8⁺ cell responses after 2 injections of the fusion proteins constructed based on different mutants in Example 10.
Fig. 25 shows the results of Western Blot of the fusion proteins constructed based on different gE truncates in Example 11.
Fig. 26 shows the results of capillary electrophoresis of the mRNA encoding gE-gI fusion protein in Example 12.
Fig. 27 shows the particle size of the LNP-encapsulated mRNA encoding gE-gI fusion protein in Example 12.
Fig. 28 shows the titers of the binding antibody IgG induced by immunization with the gE-gI mRNA/LNP and by the gE-gI mRNA/LNP combined with CpG adjuvant in Example 12.
Fig. 29 shows the CD4⁺ T and CD8⁺ T cell responses induced by immunization with the gE-gI mRNA/LNP and by the gE-gI mRNA/LNP combined with CpG adjuvant in Example 12.
Fig. 30 shows the monoclonality of the Top1 clone (CB4019-clone-16) in Example 13.
Fig. 31 shows the SDS-PAGE diagram of the gE-gI fusion protein obtained in Example 13.
Fig. 32 shows the HPLC diagram of the gE-gI fusion protein derived from the CHO stable cell line in Example 13.
Fig. 33 shows the titers of binding antibody IgG induced by the gE-gI fusion protein derived from the CHO stable cell line combined with AS01B adjuvant, as well as by Shingrix vaccine, in Example 13.
Fig. 34 shows the titers of neutralizing antibodies induced by the gE-gI fusion protein derived from CHO stable cell line combined with AS01B adjuvant, as well as by the Shingrix vaccine, in Example 13.
Fig. 35 shows the CD4⁺ T and CD8⁺ T cell responses induced by the gE-gI fusion protein derived from CHO stable cell line combined with AS01B adjuvant, as well as by the Shingrix vaccine, in Example 13.
Fig. 36 shows the levels of IFN-γ and IL-2 induced by the gE-gI fusion protein derived from CHO stable cell line combined with AS01B adjuvant, as well as by the Shingrix vaccine, as detected by Elispot assay in Example 13.

### Sequence information

The description of the sequences involved in the present disclosure is provided in the following table.

**Table 1: Sequence information**

| SEQ ID NO | Sequence information |
|---|---|
| 1 | gE (aa. 23-537) encoding nucleotide sequence |
| | CGTATAACGAATCCGGTCAGAGCATCCGTCTTGCGATACGATGATTTTCACATCGATG AAGACAAACTGGATACAAACTCCGTATATGAGCCTTACTACCATTCAGATCATGCGG AGTCTTCATGGGTAAATCGGGGAGAGTCTTCGCGAAAAGCGTACGATCATAACTCAC CTTATATATGGCCACGTAATGATTATGATGGATTTTTAGAGAACGCACACGAACACC ATGGGGTGTATAATCAGGGCCGTGGTATCGATAGCGGGGAACGGTTAATGCAACCCA CACAAATGTCTGCACAGGAGGATCTTGGGGACGATACGGGCATCCACGTTATCCCTA CGTTAAACGGCGATGACAGACATAAAATTGTAAATGTGGACCAACGTCAATACGGTG ACGTGTTTAAAGGAGATCTTAATCCAAAACCCCAAGGCCAAAGACTCATTGAGGTGT CAGTGGAAGAAAATCACCCGTTTACTTTACGCGCACCGATTCAGCGGATTTATGGAG TCCGGTACACCGAGACTTGGAGCTTTTTGCCGTCATTAACCTGTACGGGAGACGCAG CGCCCGCCATCCAGCATATATGTTTAAAACATACAACATGCTTTCAAGACGTGGTGG TGGATGTGGATTGCGCGGAAAATACTAAAGAGGATCAGTTGGCCGAAATCAGTTACC GTTTTCAAGGTAAGAAGGAAGCGGACCAACCGTGGATTGTTGTAAACACGAGCACAC TGTTTGATGAACTCGAATTAGACCCCCCCGAGATTGAACCGGGTGTCTTGAAAGTAC TTCGGACAGAAAAACAATACTTGGGTGTGTACATTTGGAACATGCGCGGCTCCGATG GTACGTCTACCTACGCCACGTTTTTGGTCACCTGGAAAGGGGATGAAAAAACAAGAA ACCCTACGCCCGCAGTAACTCCTCAACCAAGAGGGGCTGAGTTTCATATGTGGAATT ACCACTCGCATGTATTTTCAGTTGGTGATACGTTTAGCTTGGCAATGCATCTTCAGTA TAAGATACATGAAGCGCCATTTGATTTGCTGTTAGAGTGGTTGTATGTCCCCATCGAT CCTACATGTCAACCAATGCGGTTATATTCTACGTGTTTGTATCATCCCAACGCACCCC AATGCCTCTCTCATATGAATTCCGGTTGTACATTTACCTCGCCACATTTAGCCCAGCG |
| | TGTTGCAAGCACAGTGTATCAAAATTGTGAACATGCAGATAACTACACCGCATATTG TCTGGGAATATCTCATATGGAGCCTAGCTTTGGTCTAATCTTACACGACGGGGGCACC ACGTTAAAGTTTGTAGATACACCCGAGAGTTTGTCGGGATTATACGTTTTTGTGGTGT ATTTTAACGGGCATGTTGAAGCCGTAGCATACACTGTTGTATCCACAGTAGATCATTT TGTAAACGCAATTGAAGAGCGTGGATTTCCGCCAACGGCCGGTCAGCCACCGGCGAC TACTAAACCCAAGGAAATTACCCCCGTAAACCCCGGAACGTCACCACTTCTACGA |
| 2 | gI (aa. 21-271) encoding nucleotide sequence |
| | TTGATCTTCAAGGGCGACCACGTGAGCTTGCAAGTTAACAGCAGTCTCACGTCTATCC TTATTCCCATGCAAAATGATAATTATACAGAGATAAAAGGACAGCTTGTCTTTATTGG AGAGCAACTACCTACCGGGACAAACTATAGCGGAACACTGGAACTGTTATACGCGG ATACGGTGGCGTTTTGTTTCCGGTCAGTACAAGTAATAAGATACGACGGATGTCCCC GGATTAGAACGAGCGCTTTTATTTCGTGTAGGTACAAACATTCGTGGCATTATGGTAA CTCAACGGATCGGATATCAACAGAGCCGGATGCTGGTGTAATGTTGAAAATTACCAA ACCGGGAATAAATGATGCTGGTGTGTATGTACTTCTTGTTCGGTTAGACCATAGCAG ATCCACCGATGGTTTCATTCTTGGTGTAAATGTATATACAGCGGGCTCGCATCACAAC ATTCACGGGGTTATCTACACTTCTCCATCTCTACAGAATGGATATTCTACAAGAGCCC TTTTTCAACAAGCTCGTTTGTGTGATTTACCCGCGACACCCAAAGGGTCCGGTACCTC CCTGTTTCAACATATGCTTGATCTTCGTGCCGGTAAATCGTTAGAGGATAACCCTTGG TTACATGAGGACGTTGTTACGACAGAAACTAAGTCCGTTGTTAAGGAGGGGATAGAA AATCACGTATATCCAACGGATATGTCCACGTTACCCGAAAAGTCCCTTAATGATCCTC CAGAATAA |
| 3 | Linker encoding nucleotide sequence |
| | GGAGGGGGAGGGTCAGGAGGGGGAGGGTCAGGAGGGGGAGGGTCAGGAGGGGGAG GGTCA |
| 4 | gE-gI (HIS tag) nucleotide sequence |
| | CGTATAACGAATCCGGTCAGAGCATCCGTCTTGCGATACGATGATTTTCACATCGATG AAGACAAACTGGATACAAACTCCGTATATGAGCCTTACTACCATTCAGATCATGCGG AGTCTTCATGGGTAAATCGGGGAGAGTCTTCGCGAAAAGCGTACGATCATAACTCAC CTTATATATGGCCACGTAATGATTATGATGGATTTTTAGAGAACGCACACGAACACC ATGGGGTGTATAATCAGGGCCGTGGTATCGATAGCGGGGAACGGTTAATGCAACCCA CACAAATGTCTGCACAGGAGGATCTTGGGGACGATACGGGCATCCACGTTATCCCTA CGTTAAACGGCGATGACAGACATAAAATTGTAAATGTGGACCAACGTCAATACGGTG ACGTGTTTAAAGGAGATCTTAATCCAAAACCCCAAGGCCAAAGACTCATTGAGGTGT CAGTGGAAGAAAATCACCCGTTTACTTTACGCGCACCGATTCAGCGGATTTATGGAG TCCGGTACACCGAGACTTGGAGCTTTTTGCCGTCATTAACCTGTACGGGAGACGCAG CGCCCGCCATCCAGCATATATGTTTAAAACATACAACATGCTTTCAAGACGTGGTGG TGGATGTGGATTGCGCGGAAAATACTAAAGAGGATCAGTTGGCCGAAATCAGTTACC GTTTTCAAGGTAAGAAGGAAGCGGACCAACCGTGGATTGTTGTAAACACGAGCACAC TGTTTGATGAACTCGAATTAGACCCCCCCGAGATTGAACCGGGTGTCTTGAAAGTAC TTCGGACAGAAAAACAATACTTGGGTGTGTACATTTGGAACATGCGCGGCTCCGATG GTACGTCTACCTACGCCACGTTTTTGGTCACCTGGAAAGGGGATGAAAAAACAAGAA ACCCTACGCCCGCAGTAACTCCTCAACCAAGAGGGGCTGAGTTTCATATGTGGAATT ACCACTCGCATGTATTTTCAGTTGGTGATACGTTTAGCTTGGCAATGCATCTTCAGTA TAAGATACATGAAGCGCCATTTGATTTGCTGTTAGAGTGGTTGTATGTCCCCATCGAT CCTACATGTCAACCAATGCGGTTATATTCTACGTGTTTGTATCATCCCAACGCACCCC AATGCCTCTCTCATATGAATTCCGGTTGTACATTTACCTCGCCACATTTAGCCCAGCG TGTTGCAAGCACAGTGTATCAAAATTGTGAACATGCAGATAACTACACCGCATATTG TCTGGGAATATCTCATATGGAGCCTAGCTTTGGTCTAATCTTACACGACGGGGGCACC ACGTTAAAGTTTGTAGATACACCCGAGAGTTTGTCGGGATTATACGTTTTTGTGGTGT ATTTTAACGGGCATGTTGAAGCCGTAGCATACACTGTTGTATCCACAGTAGATCATTT TGTAAACGCAATTGAAGAGCGTGGATTTCCGCCAACGGCCGGTCAGCCACCGGCGAC TACTAAACCCAAGGAAATTACCCCCGTAAACCCCGGAACGTCACCACTTCTACGAGG AGGGGGAGGGTCAGGAGGGGGAGGGTCAGGAGGGGGAGGGTCAGGAGGGGGAGGG TCATTGATCTTCAAGGGCGACCACGTGAGCTTGCAAGTTAACAGCAGTCTCACGTCT |
| | ATCCTTATTCCCATGCAAAATGATAATTATACAGAGATAAAAGGACAGCTTGTCTTTA TTGGAGAGCAACTACCTACCGGGACAAACTATAGCGGAACACTGGAACTGTTATACG CGGATACGGTGGCGTTTTGTTTCCGGTCAGTACAAGTAATAAGATACGACGGATGTC CCCGGATTAGAACGAGCGCTTTTATTTCGTGTAGGTACAAACATTCGTGGCATTATGG TAACTCAACGGATCGGATATCAACAGAGCCGGATGCTGGTGTAATGTTGAAAATTAC CAAACCGGGAATAAATGATGCTGGTGTGTATGTACTTCTTGTTCGGTTAGACCATAGC AGATCCACCGATGGTTTCATTCTTGGTGTAAATGTATATACAGCGGGCTCGCATCACA ACATTCACGGGGTTATCTACACTTCTCCATCTCTACAGAATGGATATTCTACAAGAGC CCTTTTTCAACAAGCTCGTTTGTGTGATTTACCCGCGACACCCAAAGGGTCCGGTACC TCCCTGTTTCAACATATGCTTGATCTTCGTGCCGGTAAATCGTTAGAGGATAACCCTT GGTTACATGAGGACGTTGTTACGACAGAAACTAAGTCCGTTGTTAAGGAGGGGATAG AAAATCACGTATATCCAACGGATATGTCCACGTTACCCGAAAAGTCCCTTAATGATC CTCCAGAACATCACCACCATCACCACCACCACCATCACTAA |
| 5 | gI-gE encoding nucleotide sequence |
| | TTGATCTTCAAGGGCGACCACGTGAGCTTGCAAGTTAACAGCAGTCTCACGTCTATCC TTATTCCCATGCAAAATGATAATTATACAGAGATAAAAGGACAGCTTGTCTTTATTGG AGAGCAACTACCTACCGGGACAAACTATAGCGGAACACTGGAACTGTTATACGCGG ATACGGTGGCGTTTTGTTTCCGGTCAGTACAAGTAATAAGATACGACGGATGTCCCC GGATTAGAACGAGCGCTTTTATTTCGTGTAGGTACAAACATTCGTGGCATTATGGTAA CTCAACGGATCGGATATCAACAGAGCCGGATGCTGGTGTAATGTTGAAAATTACCAA ACCGGGAATAAATGATGCTGGTGTGTATGTACTTCTTGTTCGGTTAGACCATAGCAG ATCCACCGATGGTTTCATTCTTGGTGTAAATGTATATACAGCGGGCTCGCATCACAAC ATTCACGGGGTTATCTACACTTCTCCATCTCTACAGAATGGATATTCTACAAGAGCCC TTTTTCAACAAGCTCGTTTGTGTGATTTACCCGCGACACCCAAAGGGTCCGGTACCTC CCTGTTTCAACATATGCTTGATCTTCGTGCCGGTAAATCGTTAGAGGATAACCCTTGG TTACATGAGGACGTTGTTACGACAGAAACTAAGTCCGTTGTTAAGGAGGGGATAGAA AATCACGTATATCCAACGGATATGTCCACGTTACCCGAAAAGTCCCTTAATGATCCTC CAGAAGGAGGGGGAGGGTCAGGAGGGGGAGGGTCAGGAGGGGGAGGGTCAGGAGG GGGAGGGTCACGTATAACGAATCCGGTCAGAGCATCCGTCTTGCGATACGATGATTT TCACATCGATGAAGACAAACTGGATACAAACTCCGTATATGAGCCTTACTACCATTC AGATCATGCGGAGTCTTCATGGGTAAATCGGGGAGAGTCTTCGCGAAAAGCGTACGA TCATAACTCACCTTATATATGGCCACGTAATGATTATGATGGATTTTTAGAGAACGCA CACGAACACCATGGGGTGTATAATCAGGGCCGTGGTATCGATAGCGGGGAACGGTTA ATGCAACCCACACAAATGTCTGCACAGGAGGATCTTGGGGACGATACGGGCATCCAC GTTATCCCTACGTTAAACGGCGATGACAGACATAAAATTGTAAATGTGGACCAACGT CAATACGGTGACGTGTTTAAAGGAGATCTTAATCCAAAACCCCAAGGCCAAAGACTC ATTGAGGTGTCAGTGGAAGAAAATCACCCGTTTACTTTACGCGCACCGATTCAGCGG ATTTATGGAGTCCGGTACACCGAGACTTGGAGCTTTTTGCCGTCATTAACCTGTACGG GAGACGCAGCGCCCGCCATCCAGCATATATGTTTAAAACATACAACATGCTTTCAAG ACGTGGTGGTGGATGTGGATTGCGCGGAAAATACTAAAGAGGATCAGTTGGCCGAA ATCAGTTACCGTTTTCAAGGTAAGAAGGAAGCGGACCAACCGTGGATTGTTGTAAAC ACGAGCACACTGTTTGATGAACTCGAATTAGACCCCCCCGAGATTGAACCGGGTGTC TTGAAAGTACTTCGGACAGAAAAACAATACTTGGGTGTGTACATTTGGAACATGCGC GGCTCCGATGGTACGTCTACCTACGCCACGTTTTTGGTCACCTGGAAAGGGGATGAA AAAACAAGAAACCCTACGCCCGCAGTAACTCCTCAACCAAGAGGGGCTGAGTTTCAT ATGTGGAATTACCACTCGCATGTATTTTCAGTTGGTGATACGTTTAGCTTGGCAATGC ATCTTCAGTATAAGATACATGAAGCGCCATTTGATTTGCTGTTAGAGTGGTTGTATGT CCCCATCGATCCTACATGTCAACCAATGCGGTTATATTCTACGTGTTTGTATCATCCC AACGCACCCCAATGCCTCTCTCATATGAATTCCGGTTGTACATTTACCTCGCCACATT TAGCCCAGCGTGTTGCAAGCACAGTGTATCAAAATTGTGAACATGCAGATAACTACA CCGCATATTGTCTGGGAATATCTCATATGGAGCCTAGCTTTGGTCTAATCTTACACGA CGGGGGCACCACGTTAAAGTTTGTAGATACACCCGAGAGTTTGTCGGGATTATACGT TTTTGTGGTGTATTTTAACGGGCATGTTGAAGCCGTAGCATACACTGTTGTATCCACA GTAGATCATTTTGTAAACGCAATTGAAGAGCGTGGATTTCCGCCAACGGCCGGTCAG CCACCGGCGACTACTAAACCCAAGGAAATTACCCCCGTAAACCCCGGAACGTCACCA CTTCTACGA |
| 6 | gE-gI amino acid sequence |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 7 | gI-gE amino acid sequence |
| | LIFKGDHVSLQVNSSLTSILIPMQNDNYTEIKGQLVFIGEQLPTGTNYSGTLELLYADTVAF CFRSVQVIRYDGCPRIRTSAFISCRYKHSWHYGNSTDRISTEPDAGVMLKITKPGINDAGV YVLLVRLDHSRSTDGFILGVNVYTAGSHHNIHGVIYTSPSLQNGYSTRALFQQARLCDLP ATPKGSGTSLFQHMLDLRAGKSLEDNPWLHEDVVTTETKSVVKEGIENHVYPTDMSTLP EKSLNDPPEGGGGSGGGGSGGGGSGGGGSRITNPVRASVLRYDDFHIDEDKLDTNSVYE PYYHSDHAESSWVNRGESSRKAYDHNSPYIWPRNDYDGFLENAHEHHGVYNQGRGIDS GERLMQPTQMSAQEDLGDDTGIHVIPTLNGDDRHKIVNVDQRQYGDVFKGDLNPKPQG QRLIEVSVEENHPFTLRAPIQRIYGVRYTETWSFLPSLTCTGDAAPAIQHICLKHTTCFQDV VVDVDCAENTKEDQLAEISYRFQGKKEADQPWIVVNTSTLFDELELDPPEIEPGVLKVLR TEKQYLGVYIWNMRGSDGTSTYATFLVTWKGDEKTRNPTPAVTPQPRGAEFHMWNYH SHVFSVGDTFSLAMHLQYKIHEAPFDLLLEWLYVPIDPTCQPMRLYSTCLYHPNAPQCLS HMNSGCTFTSPHLAQRVASTVYQNCEHADNYTAYCLGISHMEPSFGLILHDGGTTLKFV DTPESLSGLYVFVVYFNGHVEAVAYTVVSTVDHFVNAIEERGFPPTAGQPPATTKPKEITP VNPGTSPLLR |
| 8 | 10×His encoding nucleotide sequence |
| | CATCACCACCATCACCACCACCACCATCAC |
| 9 | 10×His amino acid sequence |
| | HHHHHHHHHH |
| 10 | gE-gI (HIS tag) amino acid sequence |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPEHHHHHHHHHH |
| 11 | gE full-length amino acid sequence |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSLAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LLRYAAWTGGLAAVVLLCLVIFLICTAKRMRVKAYRVDKSPYNQSMYYAGLPVDDFED SESTDTEEEFGNAIGGSHGGSSYTVYIDKTR |
| 12 | gI full-length amino acid sequence |
| | MFLIQCLISAVIFYIQVTNALIFKGDHVSLQVNSSLTSILIPMQNDNYTEIKGQLVFIGEQLP TGTNYSGTLELLYADTVAFCFRSVQVIRYDGCPRIRTSAFISCRYKHSWHYGNSTDRISTE PDAGVMLKITKPGINDAGVYVLLVRLDHSRSTDGFILGVNVYTAGSHHNIHGVIYTSPSL QNGYSTRALFQQARLCDLPATPKGSGTSLFQHMLDLRAGKSLEDNPWLHEDVVTTETKS VVKEGIENHVYPTDMSTLPEKSLNDPPENLLIIIPIVASVMILTAMVIVIVISVKRRRIKKHPI YRPNTKTRRGIQNATPESDVMLEAAIAQLATIREESPPHSVVNPFVK |
| 13 | gE (aa. 23-537) amino acid sequence |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLR |
| 14 | gI (aa. 21-271) amino acid sequence |
| | LIFKGDHVSLQVNSSLTSILIPMQNDNYTEIKGQLVFIGEQLPTGTNYSGTLELLYADTVAF CFRSVQVIRYDGCPRIRTSAFISCRYKHSWHYGNSTDRISTEPDAGVMLKITKPGINDAGV YVLLVRLDHSRSTDGFILGVNVYTAGSHHNIHGVIYTSPSLQNGYSTRALFQQARLCDLP ATPKGSGTSLFQHMLDLRAGKSLEDNPWLHEDVVTTETKSVVKEGIENHVYPTDMSTLP EKSLNDPPE |
| 15 | Linker amino acid sequence |
| | GGGGSGGGGSGGGGSGGGGS |
| 16 | T7 promotor sequence |
| | TAATACGACTCACTATA |
| 17 | 5'UTR sequence |
| | AGAAUAAACUAGUAUUCUUCUGGUCCCCACAGACUCAGAGAGAACCC |
| 18 | 3'UTR sequence |
| | CUCGAGCUGGUACUGCAUGCACGCAAUGCUAGCUGCCCCUUUCCCGUCCUGGGUAC CCCGAGUCUCCCCCGACCUCGGGUCCCAGGUAUGCUCCCACCUCCACCUGCCCCAC UCACCACCUCUGCUAGUUCCAGACACCUCCCAAGCACGCAGCAAUGCAGCUCAAAA CGCUUAGCCUAGCCACACCCCCACGGGAAACAGCAGUGAUUAACCUUUAGCAAUA AACGAAAGUUUAACUAAGCUAUACUAACCCCAGGGUUGGUCAAUUUCGUGCCAGC CACACCCUGGAGCUAGC |
| 19 | PolyA nucleotide sequence |
| | AAAAAAAAAAAAAAAAAAAAAAAAAAAGCAUAUGACUAAAAAAAAAAA AAAAAAAAAAAAAAAAAAAAAAAAAAAAAAAAAAAAAAAAAAAAAAAA AA |
| 20 | Fusion protein gE-gI (gE-I40T) |
| | RITNPVRASVLRYDDFHTDEDKLDTNSVYEPYYHSDHAESSWVNRGESSRKAYDHNSPY IWPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 21 | Fusion protein gE-gI (gE-E124D) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQDDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 22 | Fusion protein gE-gI (gE-L162R) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDRNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 23 | Fusion protein gE-gI (gE-W319R) |
| | RITNPVRASVLRYDDFHIDEDKLDTNSVYEPYYHSDHAESSWVNRGESSRKAYDHNSPYI |
| | WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTR KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 24 | Fusion protein gE-gI (gE-L359S) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSSAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 25 | Fusion protein gE-gI (gE-Q404K) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPKCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 26 | Fusion protein gE-gI (gE-L536I) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLIRGGGGSGGGGSGGGGSGGGGS LIFKGDHVSLQVNSSLTSILIPMQNDNYTEIKGQLVFIGEQLPTGTNYSGTLELLYADTVAF |
| | CFRSVQVIRYDGCPRIRTSAFISCRYKHSWHYGNSTDRISTEPDAGVMLKITKPGINDAGV YVLLVRLDHSRSTDGFILGVNVYTAGSHHNIHGVIYTSPSLQNGYSTRALFQQARLCDLP ATPKGSGTSLFQHMLDLRAGKSLEDNPWLHEDVVTTETKSVVKEGIENHVYPTDMSTLP EKSLNDPPE |
| 27 | Fusion protein gE-gI (gE-I40T, D150N) |
| | RITNPVRASVLRYDDFHTDEDKLDTNSVYEPYYHSDHAESSWVNRGESSRKAYDHNSPY IWPRNDYDGFLENAHEHHGVYNQGRGIDSGERLMQPTQMSAQEDLGDDTGIHVIPTLNG DDRHKIVNVNQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 28 | Fusion protein gE-gI (gE-A186V, L536I) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRVPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLIRGGGGSGGGGSGGGGSGGGGS LIFKGDHVSLQVNSSLTSILIPMQNDNYTEIKGQLVFIGEQLPTGTNYSGTLELLYADTVAF CFRSVQVIRYDGCPRIRTSAFISCRYKHSWHYGNSTDRISTEPDAGVMLKITKPGINDAGV YVLLVRLDHSRSTDGFILGVNVYTAGSHHNIHGVIYTSPSLQNGYSTRALFQQARLCDLP ATPKGSGTSLFQHMLDLRAGKSLEDNPWLHEDVVTTETKSVVKEGIENHVYPTDMSTLP EKSLNDPPE |
| 29 | Fusion protein gE-gI (gE-T512M, L536I) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPMAGQPPATTKPKEITPVNPGTSPLIRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLNDPPE |
| 30 | Fusion protein gE-gI (gI-T211P) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG |
| | DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGPSLFQHMLDLRAGKSLEDNPWLHEDVVTTETKSVVKEGIENHVYPTDMSTL PEKSLNDPPE |
| 31 | Fusion protein gE-gI (gI-K246Q) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVQEGIENHVYPTDMSTL PEKSLNDPPE |
| 32 | Fusion protein gE-gI (gI-N267S) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLRGGGGSGGGGSGGGGSGGGG SLIFKGDHVSLQVNSSLTSILIPMQNDNYTEIKGQLVFIGEQLPTGTNYSGTLELLYADTVA FCFRSVQVIRYDGCPRIRTSAFISCRYKHSWHYGNSTDRISTEPDAGVMLKITKPGINDAG VYVLLVRLDHSRSTDGFILGVNVYTAGSHHNIHGVIYTSPSLQNGYSTRALFQQARLCDL PATPKGSGTSLFQHMLDLRAGKSLEDNPWLHEDVVTTETKSVVKEGIENHVYPTDMSTL PEKSLSDPPE |
| 33 | gE (aa. 23-537, I40T) |
| | RITNPVRASVLRYDDFHTDEDKLDTNSVYEPYYHSDHAESSWVNRGESSRKAYDHNSPY IWPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLR |
| 34 | gE (aa. 23-537, E124D) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQDDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLR |
| 35 | gE (aa. 23-537, L162R) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDRNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLR |
| 36 | gE (aa. 23-537, W319R) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTR KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLR |
| 37 | gE (aa. 23-537, L359S) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSSAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLR |
| 38 | gE (aa. 23-537, Q404K) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPKCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLR |
| 39 | gE (aa. 23-537, L536I) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLIR |
| 40 | gE (aa. 23-537, I40T&D150N) |
| | RITNPVRASVLRYDDFHTDEDKLDTNSVYEPYYHSDHAESSWVNRGESSRKAYDHNSPY IWPRNDYDGFLENAHEHHGVYNQGRGIDSGERLMQPTQMSAQEDLGDDTGIHVIPTLNG DDRHKIVNVNQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLLR |
| 41 | gE (aa. 23-537, A186V&L536I) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRVPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPTAGQPPATTKPKEITPVNPGTSPLIR |
| 42 | gE (aa. 23-537, T512M&L536I) |
| | RITNPVRASVLRYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVFVVYFNGHVEAVAYTVVST VDHFVNAIEERGFPPMAGQPPATTKPKEITPVNPGTSPLIR |
| 43 | gI (aa. 21-271, T211P) |
| | LIFKGDHVSLQVNSSLTSILIPMQNDNYTEIKGQLVFIGEQLPTGTNYSGTLELLYADTVAF CFRSVQVIRYDGCPRIRTSAFISCRYKHSWHYGNSTDRISTEPDAGVMLKITKPGINDAGV YVLLVRLDHSRSTDGFILGVNVYTAGSHHNIHGVIYTSPSLQNGYSTRALFQQARLCDLP ATPKGSGPSLFQHMLDLRAGKSLEDNPWLHEDVVTTETKSVVKEGIENHVYPTDMSTLP EKSLNDPPE |
| 44 | gI (aa. 21-271, K246Q) |
| | LIFKGDHVSLQVNSSLTSILIPMQNDNYTEIKGQLVFIGEQLPTGTNYSGTLELLYADTVAF CFRSVQVIRYDGCPRIRTSAFISCRYKHSWHYGNSTDRISTEPDAGVMLKITKPGINDAGV YVLLVRLDHSRSTDGFILGVNVYTAGSHHNIHGVIYTSPSLONGYSTRALFOOARLCDLP |
| | ATPKGSGTSLFQHMLDLRAGKSLEDNPWLHEDVVTTETKSVVQEGIENHVYPTDMSTLP EKSLNDPPE |
| 45 | gI (aa. 21-271, N267S) |
| | LIFKGDHVSLQVNSSLTSILIPMQNDNYTEIKGQLVFIGEQLPTGTNYSGTLELLYADTVAF CFRSVQVIRYDGCPRIRTSAFISCRYKHSWHYGNSTDRISTEPDAGVMLKITKPGINDAGV YVLLVRLDHSRSTDGFILGVNVYTAGSHHNIHGVIYTSPSLQNGYSTRALFQQARLCDLP ATPKGSGTSLFQHMLDLRAGKSLEDNPWLHEDVVTTETKSVVKEGIENHVYPTDMSTLP EKSLSDPPE |
| 46 | Full-length gE (Dumas strain) |
| | MGTVNKPVVGVLMGFGIITGTLRITNPVRASVLRYDDFHTDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSLAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LLRYAAWTGGLAAVVLLCLVIFLICTAKRMRVKAYRVDKSPYNQSMYYAGLPVDDFED SESTDTEEEFGNAIGGSHGGSSYTVYIDKTR |
| 47 | Full-length gE (MSP strain) |
| | MGTVNKPVVGVLMGFGIITGTLRITNPVRASVLRYDDFHTDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVNQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSLAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LLRYAAWTGGLAAVVLLCLVIFLICTAKRMRVKAYRVDKSPYNQSMYYAGLPVDDFED SESTDTEEEFGNAIGGSHGGSSYTVYIDKTR |
| 48 | Full-length gE (GenBank: AEW88980) |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSLAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPMAGQPPATTKPKEITPVNPGTSP LIRYAAWTGGLAAVVLLCLVIFLICTAKRMRVKAYRVDKSPYNQSMYYAGLPVDDFED SESTDTEEEFGNAIGGSHRGSSYTVYIDKSP |
| 49 | Full-length gE (GenBank: AEW89124) |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG |
| | DTFSLAMHLQYKIHEAPFDLLLEWLYVPIDPTCQPMRLYSTCLYHPNAPKCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LLRYAAWTGGLAAVVLLCLVIFLICTAKRMRVKAYRVDKSPYNQSMYYAGLPVDDFED SESTDTEEEFGNAIGGSHGGSSYTVYIDKTR |
| 50 | Full-length gE (GenBank: QCA43570) |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTRKGDEKTRNPTPAVTPQPRGAEFHMWNYHSHVFSVGD TFSLAMHLQYKIHEAPFDLLLEWLYVPIDPTCQPMRLYSTCLYHPNAPQCLSHMNSGCTF TSPHLAQRVASTVYQNCEHADNYTAYCLGISHMEPSFGLILHDGGTTLKFVDTPESLSGL YVFVVYFNGHVEAVAYTVVSTVDHFVNAIEERGFPPTAGQPPATTKPKEITPVNPGTSPL LRYAAWTGGLAAVVLLCLVIFLICTAKRMRVKAYRVDKSPYNQSMYYAGLPVDDFEDS ESTDTEEEFGNAIGGSHGGSSYTVYIDKTR |
| 51 | Full-length gE (GenBank: QCA45176) |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQDDLGDDTGIHVIPTLNGDDRHKIVNVDQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSLAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LLRYAAWTGGLAAVVLLCLVIFLICTAKRMRVKAYRVDKSPYNQSMYYAGLPVDDFED SESTDTEEEFGNAIGGSHGGSSYTVYIDKTR |
| 52 | Full-length gE (GenBank: QWE79571) |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSSAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LLRYAAWTGGLAAVVLLCLVIFLICTAKRMRVKAYRVDKSPYNQSMYYAGLPVDDFED SESTDTEEEFGNAIGGSHGGSSYTVYIDKTR |
| 53 | Full-length gE (GenBank: AEW89412) |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDRNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSLAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LLRYAAWTGGLAAVVLLCLVIFLICTAKRMRVKAYRVDKSPYNQSMYYAGLPVDDFED |
| | SESTDTEEEFGNAIGGSHGGSSYTVYIDKTR |
| 54 | Full-length gE (GenBank: ANS12941) |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKPQGQRLIEVSV EENHPFTLRVPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSLAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LIRYAAWTGGLAAVVLLCLVIFLICTAKRMRVKAYRVDKSPYNQSMYYAGLPVDDFED SESTDTEEEFGNAIGGSHGGSSYTVYIDKTR |
| 55 | Full-length gE (GenBank: AQT34120) |
| | MGTVNKPVVGVLMGFGIITGTLRITNPVRASVLRYDDFHIDEDKLDTNSVYEPYYHSDH AESSWVNRGESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKPQGQRLIEVSV EENHPFTLRAPIQRIYGVRYTETWSFLPSLTCTGDAAPAIQHICLKHTTCFQDVVVDVDCA ENTKEDQLAEISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWNYHSHVFSVG DTFSLAMHLQYKIHEAPFDLLLEWLYVPIDPTCQPMRLYSTCLYHPNAPQCLSHMNSGCT FTSPHLAQRVASTVYQNCEHADNYTAYCLGISHMEPSFGLILHDGGTTLKFVDTPESLSG LYVFVVYFNGHVEAVAYTVVSTVDHFVNAIEERGFPPTAGQPPATTKPKEITPVNPGTSP LIRYAAWTGGLAAVVLLCLVIFLICTAKRMRVKAYRVDKSPYNQSMYYAGLPVDDFED SESTDTEEEFGNAIGGSHGGSSYTVYIDKTR |
| 56 | Full-length gI (GenBank: AEW89051) |
| | MFLIQCLISAVIFYIQVTNALIFKGDHVSLQVNSSLTSILIPMQNDNYTEIKGQLVFIGEQLP TGTNYSGTLELLYADTVAFCFRSVQVIRYDGCPRIRTSAFISCRYKHSWHYGNSTDRISTE PDAGVMLKITKPGINDAGVYVLLVRLDHSRSTDGFILGVNVYTAGSHHNIHGVIYTSPSL QNGYSTRALFQQARLCDLPATPKGSGTSLFQHMLDLRAGKSLEDNPWLHEDVVTTETKS VVQEGIENHVYPTDMSTLPEKSLNDPPENLLIIIPIVASVMILTAMVIVIVISVKRRRIKKHPI YRPNTKTRRGIQNATPESDVMLEAAIAQLATIREESPPHSVVNPFVK |
| 57 | Full-length gI (GenBank: AEW89483) |
| | MFLIQCLISAVIFYIQVTNALIFKGDHVSLQVNSSLTSILIPMQNDNYTEIKGQLVFIGEQLP TGTNYSGTLELLYADTVAFCFRSVQVIRYDGCPRIRTSAFISCRYKHSWHYGNSTDRISTE PDAGVMLKITKPGINDAGVYVLLVRLDHSRSTDGFILGVNVYTAGSHHNIHGVIYTSPSL QNGYSTRALFQQARLCDLPATPKGSGPSLFQHMLDLRAGKSLEDNPWLHEDVVTTETKS VVKEGIENHVYPTDMSTLPEKSLNDPPENLLIIIPIVASVMILTAMVIVIVISVKRRRIKKHPI YRPNTKTRRGIQNATPESDVMLEAAIAQLATIREESPPHSVVNPFVK |
| 58 | Full-length gI (GenBank: AGY34059) |
| | MFLIQCLISAVIFYIQVTNALIFKGDHVSLQVNSSLTSILIPMQNDNYTEIKGQLVFIGEQLP TGTNYSGTLELLYADTVAFCFRSVQVIRYDGCPRIRTSAFISCRYKHSWHYGNSTDRISTE PDAGVMLKITKPGINDAGVYVLLVRLDHSRSTDGFILGVNVYTAGSHHNIHGVIYTSPSL QNGYSTRALFQQARLCDLPATPKGSGTSLFQHMLDLRAGKSLEDNPWLHEDVVTTETKS VVKEGIENHVYPTDMSTLPEKSLSDPPENLLIIIPIVASVMILTAMVIVIVISVKRRRIKKHPI YRPNTKTRRGIQNATPESDVMLEAAIAQLATIREESPPHSVVNPFVK |
| 59 | Fusion protein gE(43-517)-gI |
| | DKLDTNSVYEPYYHSDHAESSWVNRGESSRKAYDHNSPYIWPRNDYDGFLENAHEHHG VYNQGRGIDSGERLMQPTQMSAQEDLGDDTGIHVIPTLNGDDRHKIVNVDQRQYGDVF |
| | KGDLNPKPQGQRLIEVSVEENHPFTLRAPIQRIYGVRYTETWSFLPSLTCTGDAAPAIQHIC LKHTTCFQDVVVDVDCAENTKEDQLAEISYRFQGKKEADQPWIVVNTSTLFDELELDPP EIEPGVLKVLRTEKQYLGVYIWNMRGSDGTSTYATFLVTWKGDEKTRNPTPAVTPQPRG AEFHMWNYHSHVFSVGDTFSLAMHLQYKIHEAPFDLLLEWLYVPIDPTCQPMRLYSTCL YHPNAPQCLSHMNSGCTFTSPHLAQRVASTVYQNCEHADNYTAYCLGISHMEPSFGLIL HDGGTTLKFVDTPESLSGLYVFVVYFNGHVEAVAYTVVSTVDHFVNAIEERGFPPTAGQ PPGGGGSGGGGSGGGGSGGGGSLIFKGDHVSLQVNSSLTSILIPMQNDNYTEIKGQLVFIG EQLPTGTNYSGTLELLYADTVAFCFRSVQVIRYDGCPRIRTSAFISCRYKHSWHYGNSTD RISTEPDAGVMLKITKPGINDAGVYVLLVRLDHSRSTDGFILGVNVYTAGSHHNIHGVIYT SPSLQNGYSTRALFQQARLCDLPATPKGSGTSLFQHMLDLRAGKSLEDNPWLHEDVVTT ETKSVVKEGIENHVYPTDMSTLPEKSLNDPPE |
| 60 | Fusion protein gE(63-497)-gI |
| | SWVNRGESSRKAYDHNSPYIWPRNDYDGFLENAHEHHGVYNQGRGIDSGERLMQPTQM SAQEDLGDDTGIHVIPTLNGDDRHKIVNVDQRQYGDVFKGDLNPKPQGQRLIEVSVEEN HPFTLRAPIQRIYGVRYTETWSFLPSLTCTGDAAPAIQHICLKHTTCFQDVVVDVDCAENT KEDQLAEISYRFQGKKEADQPWIVVNTSTLFDELELDPPEIEPGVLKVLRTEKQYLGVYI WNMRGSDGTSTYATFLVTWKGDEKTRNPTPAVTPQPRGAEFHMWNYHSHVFSVGDTFS LAMHLQYKIHEAPFDLLLEWLYVPIDPTCQPMRLYSTCLYHPNAPQCLSHMNSGCTFTSP HLAQRVASTVYQNCEHADNYTAYCLGISHMEPSFGLILHDGGTTLKFVDTPESLSGLYVF VVYFNGHVEAVAYTVVSTVGGGGSGGGGSGGGGSGGGGSLIFKGDHVSLQVNSSLTSIL IPMQNDNYTEIKGQLVFIGEQLPTGTNYSGTLELLYADTVAFCFRSVQVIRYDGCPRIRTS AFISCRYKHSWHYGNSTDRISTEPDAGVMLKITKPGINDAGVYVLLVRLDHSRSTDGFIL GVNVYTAGSHHNIHGVIYTSPSLQNGYSTRALFQQARLCDLPATPKGSGTSLFQHMLDLR AGKSLEDNPWLHEDVVTTETKSVVKEGIENHVYPTDMSTLPEKSLNDPPE |
| 61 | Fusion protein gE(83-477)-gI |
| | WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYVGGGGSGGGGSGGGGSGGG GSLIFKGDHVSLQVNSSLTSILIPMQNDNYTEIKGQLVFIGEQLPTGTNYSGTLELLYADTV AFCFRSVQVIRYDGCPRIRTSAFISCRYKHSWHYGNSTDRISTEPDAGVMLKITKPGINDA GVYVLLVRLDHSRSTDGFILGVNVYTAGSHHNIHGVIYTSPSLQNGYSTRALFQQARLCD LPATPKGSGTSLFQHMLDLRAGKSLEDNPWLHEDVVTTETKSVVKEGIENHVYPTDMST LPEKSLNDPPE |
| 62 | Fusion protein gE(103-457)-gI |
| | NQGRGIDSGERLMQPTQMSAQEDLGDDTGIHVIPTLNGDDRHKIVNVDQRQYGDVFKG DLNPKPQGQRLIEVSVEENHPFTLRAPIQRIYGVRYTETWSFLPSLTCTGDAAPAIQHICLK HTTCFQDVVVDVDCAENTKEDQLAEISYRFQGKKEADQPWIVVNTSTLFDELELDPPEIE PGVLKVLRTEKQYLGVYIWNMRGSDGTSTYATFLVTWKGDEKTRNPTPAVTPQPRGAE FHMWNYHSHVFSVGDTFSLAMHLQYKIHEAPFDLLLEWLYVPIDPTCQPMRLYSTCLYH PNAPQCLSHMNSGCTFTSPHLAQRVASTVYQNCEHADNYTAYCLGISHMEPSFGLILHG GGGSGGGGSGGGGSGGGGSLIFKGDHVSLQVNSSLTSILIPMQNDNYTEIKGQLVFIGEQL PTGTNYSGTLELLYADTVAFCFRSVQVIRYDGCPRIRTSAFISCRYKHSWHYGNSTDRIST EPDAGVMLKITKPGINDAGVYVLLVRLDHSRSTDGFILGVNVYTAGSHHNIHGVIYTSPS LQNGYSTRALFQQARLCDLPATPKGSGTSLFQHMLDLRAGKSLEDNPWLHEDVVTTETK SVVKEGIENHVYPTDMSTLPEKSLNDPPE |
| 63 | Fusion protein gE(143-417)-gI |
| | RHKIVNVDQRQYGDVFKGDLNPKPQGQRLIEVSVEENHPFTLRAPIQRIYGVRYTETWSF LPSLTCTGDAAPAIQHICLKHTTCFQDVVVDVDCAENTKEDQLAEISYRFQGKKEADQP |
| | WIVVNTSTLFDELELDPPEIEPGVLKVLRTEKQYLGVYIWNMRGSDGTSTYATFLVTWK GDEKTRNPTPAVTPQPRGAEFHMWNYHSHVFSVGDTFSLAMHLQYKIHEAPFDLLLEWL YVPIDPTCQPMRLYSTCLYHPNAPQCLSHMNSGCTFTSGGGGSGGGGSGGGGSGGGGSL IFKGDHVSLQVNSSLTSILIPMQNDNYTEIKGQLVFIGEQLPTGTNYSGTLELLYADTVAF CFRSVQVIRYDGCPRIRTSAFISCRYKHSWHYGNSTDRISTEPDAGVMLKITKPGINDAGV YVLLVRLDHSRSTDGFILGVNVYTAGSHHNIHGVIYTSPSLQNGYSTRALFQQARLCDLP ATPKGSGTSLFQHMLDLRAGKSLEDNPWLHEDVVTTETKSVVKEGIENHVYPTDMSTLP EKSLNDPPE |
| 64 | gE (aa. 43-517) |
| | DKLDTNSVYEPYYHSDHAESSWVNRGESSRKAYDHNSPYIWPRNDYDGFLENAHEHHG VYNQGRGIDSGERLMQPTQMSAQEDLGDDTGIHVIPTLNGDDRHKIVNVDQRQYGDVF KGDLNPKPQGQRLIEVSVEENHPFTLRAPIQRIYGVRYTETWSFLPSLTCTGDAAPAIQHIC LKHTTCFQDVVVDVDCAENTKEDQLAEISYRFQGKKEADQPWIVVNTSTLFDELELDPP EIEPGVLKVLRTEKQYLGVYIWNMRGSDGTSTYATFLVTWKGDEKTRNPTPAVTPQPRG AEFHMWNYHSHVFSVGDTFSLAMHLQYKIHEAPFDLLLEWLYVPIDPTCQPMRLYSTCL YHPNAPQCLSHMNSGCTFTSPHLAQRVASTVYQNCEHADNYTAYCLGISHMEPSFGLIL HDGGTTLKFVDTPESLSGLYVFVVYFNGHVEAVAYTVVSTVDHFVNAIEERGFPPTAGQ PP |
| 65 | gE (aa. 63-497) |
| | SWVNRGESSRKAYDHNSPYIWPRNDYDGFLENAHEHHGVYNQGRGIDSGERLMQPTQM SAQEDLGDDTGIHVIPTLNGDDRHKIVNVDQRQYGDVFKGDLNPKPQGQRLIEVSVEEN HPFTLRAPIQRIYGVRYTETWSFLPSLTCTGDAAPAIQHICLKHTTCFQDVVVDVDCAENT KEDQLAEISYRFQGKKEADQPWIVVNTSTLFDELELDPPEIEPGVLKVLRTEKQYLGVYI WNMRGSDGTSTYATFLVTWKGDEKTRNPTPAVTPQPRGAEFHMWNYHSHVFSVGDTFS LAMHLQYKIHEAPFDLLLEWLYVPIDPTCQPMRLYSTCLYHPNAPQCLSHMNSGCTFTSP HLAQRVASTVYQNCEHADNYTAYCLGISHMEPSFGLILHDGGTTLKFVDTPESLSGLYVF VVYFNGHVEAVAYTVVSTV |
| 66 | gE (aa. 83-477) |
| | WPRNDYDGFLENAHEHHGVYNQGRGIDSGERLMQPTQMSAQEDLGDDTGIHVIPTLNG DDRHKIVNVDQRQYGDVFKGDLNPKPQGQRLIEVSVEENHPFTLRAPIQRIYGVRYTET WSFLPSLTCTGDAAPAIQHICLKHTTCFQDVVVDVDCAENTKEDQLAEISYRFQGKKEAD QPWIVVNTSTLFDELELDPPEIEPGVLKVLRTEKQYLGVYIWNMRGSDGTSTYATFLVTW KGDEKTRNPTPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVASTVYQNCEHAD NYTAYCLGISHMEPSFGLILHDGGTTLKFVDTPESLSGLYV |
| 67 | gE (aa. 103-457) |
| | NQGRGIDSGERLMQPTQMSAQEDLGDDTGIHVIPTLNGDDRHKIVNVDQRQYGDVFKG DLNPKPQGQRLIEVSVEENHPFTLRAPIQRIYGVRYTETWSFLPSLTCTGDAAPAIQHICLK HTTCFQDVVVDVDCAENTKEDQLAEISYRFQGKKEADQPWIVVNTSTLFDELELDPPEIE PGVLKVLRTEKQYLGVYIWNMRGSDGTSTYATFLVTWKGDEKTRNPTPAVTPQPRGAE FHMWNYHSHVFSVGDTFSLAMHLQYKIHEAPFDLLLEWLYVPIDPTCQPMRLYSTCLYH PNAPQCLSHMNSGCTFTSPHLAQRVASTVYQNCEHADNYTAYCLGISHMEPSFGLILH |
| 68 | gE (aa. 143-417) |
| | RHKIVNVDQRQYGDVFKGDLNPKPQGQRLIEVSVEENHPFTLRAPIQRIYGVRYTETWSF LPSLTCTGDAAPAIQHICLKHTTCFQDVVVDVDCAENTKEDQLAEISYRFQGKKEADQP WIVVNTSTLFDELELDPPEIEPGVLKVLRTEKQYLGVYIWNMRGSDGTSTYATFLVTWK GDEKTRNPTPAVTPQPRGAEFHMWNYHSHVFSVGDTFSLAMHLQYKIHEAPFDLLLEWL YVPIDPTCQPMRLYSTCLYHPNAPQCLSHMNSGCTFTS |
| 69 | gE (aa. 31-537) |
| | SVLRYDDFHIDEDKLDTNSVYEPYYHSDHAESSWVNRGESSRKAYDHNSPYIWPRNDYD GFLENAHEHHGVYNQGRGIDSGERLMQPTQMSAQEDLGDDTGIHVIPTLNGDDRHKIVN VDQRQYGDVFKGDLNPKPQGQRLIEVSVEENHPFTLRAPIQRIYGVRYTETWSFLPSLTC TGDAAPAIQHICLKHTTCFQDVVVDVDCAENTKEDQLAEISYRFQGKKEADQPWIVVNT STLFDELELDPPEIEPGVLKVLRTEKQYLGVYIWNMRGSDGTSTYATFLVTWKGDEKTR NPTPAVTPQPRGAEFHMWNYHSHVFSVGDTFSLAMHLQYKIHEAPFDLLLEWLYVPIDP TCQPMRLYSTCLYHPNAPQCLSHMNSGCTFTSPHLAQRVASTVYQNCEHADNYTAYCL GISHMEPSFGLILHDGGTTLKFVDTPESLSGLYVFVVYFNGHVEAVAYTVVSTVDHFVNA IEERGFPPTAGQPPATTKPKEITPVNPGTSPLLR |
| 70 | Fusion protein gE(31-537)-gI |
| | SVLRYDDFHIDEDKLDTNSVYEPYYHSDHAESSWVNRGESSRKAYDHNSPYIWPRNDYD GFLENAHEHHGVYNQGRGIDSGERLMQPTQMSAQEDLGDDTGIHVIPTLNGDDRHKIVN VDQRQYGDVFKGDLNPKPQGQRLIEVSVEENHPFTLRAPIQRIYGVRYTETWSFLPSLTC TGDAAPAIQHICLKHTTCFQDVVVDVDCAENTKEDQLAEISYRFQGKKEADQPWIVVNT STLFDELELDPPEIEPGVLKVLRTEKQYLGVYIWNMRGSDGTSTYATFLVTWKGDEKTR NPTPAVTPQPRGAEFHMWNYHSHVFSVGDTFSLAMHLQYKIHEAPFDLLLEWLYVPIDP TCQPMRLYSTCLYHPNAPQCLSHMNSGCTFTSPHLAQRVASTVYQNCEHADNYTAYCL GISHMEPSFGLILHDGGTTLKFVDTPESLSGLYVFVVYFNGHVEAVAYTVVSTVDHFVNA IEERGFPPTAGQPPATTKPKEITPVNPGTSPLLRGGGGSGGGGSGGGGSGGGGSLIFKGDH VSLQVNSSLTSILIPMQNDNYTEIKGQLVFIGEQLPTGTNYSGTLELLYADTVAFCFRSVQ VIRYDGCPRIRTSAFISCRYKHSWHYGNSTDRISTEPDAGVMLKITKPGINDAGVYVLLVR LDHSRSTDGFILGVNVYTAGSHHNIHGVIYTSPSLQNGYSTRALFQQARLCDLPATPKGS GTSLFQHMLDLRAGKSLEDNPWLHEDVVTTETKSVVKEGIENHVYPTDMSTLPEKSLND PPE |
| 71 | RNA sequence encoding fusion protein |
| | CGUAUAACGAAUCCGGUCAGAGCAUCCGUCUUGCGAUACGAUGAUUUUCACAUCG AUGAAGACAAACUGGAUACAAACUCCGUAUAUGAGCCUUACUACCAUUCAGAUCA UGCGGAGUCUUCAUGGGUAAAUCGGGGAGAGUCUUCGCGAAAAGCGUACGAUCAU AACUCACCUUAUAUAUGGCCACGUAAUGAUUAUGAUGGAUUUUUAGAGAACGCAC ACGAACACCAUGGGGUGUAUAAUCAGGGCCGUGGUAUCGAUAGCGGGGAACGGUU AAUGCAACCCACACAAAUGUCUGCACAGGAGGAUCUUGGGGACGAUACGGGCAUC CACGUUAUCCCUACGUUAAACGGCGAUGACAGACAUAAAAUUGUAAAUGUGGACC AACGUCAAUACGGUGACGUGUUUAAAGGAGAUCUUAAUCCAAAACCCCAAGGCCA AAGACUCAUUGAGGUGUCAGUGGAAGAAAAUCACCCGUUUACUUUACGCGCACCG AUUCAGCGGAUUUAUGGAGUCCGGUACACCGAGACUUGGAGCUUUUUGCCGUCAU UAACCUGUACGGGAGACGCAGCGCCCGCCAUCCAGCAUAUAUGUUUAAAACAUAC AACAUGCUUUCAAGACGUGGUGGUGGAUGUGGAUUGCGCGGAAAAUACUAAAGAG GAUCAGUUGGCCGAAAUCAGUUACCGUUUUCAAGGUAAGAAGGAAGCGGACCAAC CGUGGAUUGUUGUAAACACGAGCACACUGUUUGAUGAACUCGAAUUAGACCCCCC CGAGAUUGAACCGGGUGUCUUGAAAGUACUUCGGACAGAAAAACAAUACUUGGGU GUGUACAUUUGGAACAUGCGCGGCUCCGAUGGUACGUCUACCUACGCCACGUUUU UGGUCACCUGGAAAGGGGAUGAAAAAACAAGAAACCCUACGCCCGCAGUAACUCC UCAACCAAGAGGGGCUGAGUUUCAUAUGUGGAAUUACCACUCGCAUGUAUUUUCA GUUGGUGAUACGUUUAGCUUGGCAAUGCAUCUUCAGUAUAAGAUACAUGAAGCGC CAUUUGAUUUGCUGUUAGAGUGGUUGUAUGUCCCCAUCGAUCCUACAUGUCAACC AAUGCGGUUAUAUUCUACGUGUUUGUAUCAUCCCAACGCACCCCAAUGCCUCUCU CAUAUGAAUUCCGGUUGUACAUUUACCUCGCCACAUUUAGCCCAGCGUGUUGCAA GCACAGUGUAUCAAAAUUGUGAACAUGCAGAUAACUACACCGCAUAUUGUCUGGG AAUAUCUCAUAUGGAGCCUAGCUUUGGUCUAAUCUUACACGACGGGGGCACCACG UUAAAGUUUGUAGAUACACCCGAGAGUUUGUCGGGAUUAUACGUUUUUGUGGUG UAUUUUAACGGGCAUGUUGAAGCCGUAGCAUACACUGUUGUAUCCACAGUAGAUC AUUUUGUAAACGCAAUUGAAGAGCGUGGAUUUCCGCCAACGGCCGGUCAGCCACC GGCGACUACUAAACCCAAGGAAAUUACCCCCGUAAACCCCGGAACGUCACCACUUC UACGAGGAGGGGGAGGGUCAGGAGGGGGAGGGUCAGGAGGGGGAGGGUCAGGAG GGGGAGGGUCAUUGAUCUUCAAGGGCGACCACGUGAGCUUGCAAGUUAACAGCAG |
| | UCUCACGUCUAUCCUUAUUCCCAUGCAAAAUGAUAAUUAUACAGAGAUAAAAGGA CAGCUUGUCUUUAUUGGAGAGCAACUACCUACCGGGACAAACUAUAGCGGAACAC UGGAACUGUUAUACGCGGAUACGGUGGCGUUUUGUUUCCGGUCAGUACAAGUAAU AAGAUACGACGGAUGUCCCCGGAUUAGAACGAGCGCUUUUAUUUCGUGUAGGUAC AAACAUUCGUGGCAUUAUGGUAACUCAACGGAUCGGAUAUCAACAGAGCCGGAUG CUGGUGUAAUGUUGAAAAUUACCAAACCGGGAAUAAAUGAUGCUGGUGUGUAUG UACUUCUUGUUCGGUUAGACCAUAGCAGAUCCACCGAUGGUUUCAUUCUUGGUGU AAAUGUAUAUACAGCGGGCUCGCAUCACAACAUUCACGGGGUUAUCUACACUUCU CCAUCUCUACAGAAUGGAUAUUCUACAAGAGCCCUUUUUCAACAAGCUCGUUUGU GUGAUUUACCCGCGACACCCAAAGGGUCCGGUACCUCCCUGUUUCAACAUAUGCU UGAUCUUCGUGCCGGUAAAUCGUUAGAGGAUAACCCUUGGUUACAUGAGGACGUU GUUACGACAGAAACUAAGUCCGUUGUUAAGGAGGGGAUAGAAAAUCACGUAUAUC CAACGGAUAUGUCCACGUUACCCGAAAAGUCCCUUAAUGAUCCUCCAGAA |
| 72 | Kozak sequence |
| | GCCACC |

### Specific Models for Carrying Out the present disclosure

The present disclosure will now be described with reference to the following examples which are intended to illustrate the present disclosure (but not to limit the present disclosure).

Unless otherwise specified, the molecular biology experimental methods and immunoassays used in the present disclosure are basically carried out in accordance with the methods described in J. Sambrook et al., Molecular Cloning: A Laboratory Manual, 2nd edition, Cold Spring Harbor Laboratory Press, 1989, and F. M. Ausubel et al., Compiled Molecular Biology Laboratory Manual, 3rd edition, John Wiley & Sons, Inc., 1995; the use of restriction endonucleases is in accordance with the conditions recommended by the product manufacturer. Those skilled in the art will appreciate that the examples describe the present disclosure by way of example and are not intended to limit the scope of protection claimed by the present disclosure.

### Example 1: Mass expression of fusion protein

The designed fusion protein structure was shown in Table 2:

**Table 2: Exemplary structure of fusion protein**

| | N-terminal to C-terminal | | |
|---|---|---|---|
| gE-gI fusion protein | gE (aa. 23-537) | linker | gI (aa. 21-271) |
| SEQ ID NO: 6 | SEQ ID NO: 13 | SEQ ID NO: 15 | SEQ ID NO: 14 |
| gI-gE fusion protein | gI (aa. 21-271) | linker | gE (aa. 23-537) |
| SEQ ID NO: 7 | SEQ ID NO: 14 | SEQ ID NO: 15 | SEQ ID NO: 13 |

Gene synthesis: The genes as set forth in SEQ ID NOs: 1, 2 and 3 were synthesized by Sangon Biotech (Shanghai) Co., Ltd.

The following exemplary experiment, using gE-gI fusion protein as an example, was carried out.

### Transfection of insect cells

(1) Confirmed that sf9 cells (purchased from Invitrogen, Cat. No.: 11496-015) or sf21 cells (purchased from Invitrogen, Cat. No.: 11497-013) were in the logarithmic growth phase (1.5-2.5×10⁶ cells/mL) and the survival rate was maintained above 90%, 200 µL of culture medium ESF 921 (purchased from Expression systems, Cat. No.: 96-001-01) containing 2% FBS, 0.1 µg of Baculovirus DNA (purchased from Expression systems, Cat. No.: 91-002) and 1 µg of pAc-gEgI plasmid (the plasmid was constructed to have the gE-gI fusion protein encoding nucleotide sequence, SEQ ID NO: 4) were added to a 24-well plate and mixed well. 1 µL of transfection reagent (purchased from Expression systems, Cat. No.: 95-055-075) was added to 50 µL of ESF921 medium (purchased from Expression systems, Cat. No.: 96-001-01) and mixed well. The above two were combined into one tube, mixed well, and allowed to stand at room temperature for 30 minutes. The cells were washed during the standing period (this was done before the standing process was about to finish): after the cells were completely attached to the wall, the medium was removed using a pipette, then 300 µL of ESF921 medium was added, the motion was carried out quickly to avoid dehydration of the cells. After gently shaking, the medium was discarded, and 300 µL of ESF921 medium was added. When the time was up, about 100 µL of the above mixture was added dropwise and evenly to the cells in each well. Then the cells were incubated at 27°C for 6 h, the supernatant was discarded, and 500 µL of complete culture medium (50% CCM3+50% TNM-FH (SIGMA-ALDRICH, T1032)+10% FBS) was added.
(2) The cell supernatant obtained in step (1) was collected, and centrifuged at 500 g for 5 min to remove cell debris and fragments, and the supernatant was stored at 4°C in the dark. This was the P1 virus seed solution.
(3) Amplification of baculovirus

Confirmed that sf9 cells or sf21 cells were in the logarithmic growth phase (1.5-2.5×10⁶ cells/mL) and the survival rate was maintained above 90%, 8 to 10 mL of the cells with a density of 6×10⁵ cells/mL was coated on a 10 cm plate. The cells were allowed to stand for 15 min to adhere to the wall. About 600 µL of the P1 virus solution was added dropwise and evenly, and incubated at 27°C for 3 to 4 days. The cytopathic effect was observed. The cell supernatant was collected, centrifuged at 1000rpm for 5 min to remove cell debris and fragments, and filtered with a 0.22 µm filter membrane. The supernatant was stored at 4°C in the dark. This was the P2 virus seed solution. The P2 virus titer was about 10⁶ to 10⁷ pfu/mL. The P3 virus could be obtained through amplification by volume in a 250 mL shake flask, according to this method.

### Protein expression in insect cell

250 mL of ESF921 medium cultivated H5 cells (purchased from Invitrogen, Cat. No.: B855-02) with a density of 2×10⁶ cells/mL and a survival rate of more than 90% were added to a 1L shake flask. The virus was added in an amount according to the corresponding MOI, the flask mouth was sealed with sealing film, and culturing was carried out in a 27°C shaker at 120 rpm. The cells in the shake flask were sampled every day, observed and counted, and relevant data were recorded. The appropriate MOI could ensure that more than 70% of the cells were diseased on the first day, all cells were diseased on the second day and had a survival rate of about 80%, and the cells ruptured on the third day and had a survival rate dropped to 30% to 50%. At this time, the cells could be harvested. The cells were collected by centrifugation at 10,000 rpm for 10 min, then the supernatant was separated and purified to obtain the gE-gI fusion protein with a sequence as set forth in SEQ ID NO: 10. The C-terminal of the fusion protein contained a 10×HIS.

### Protein expression in mammalian cell

Preparation: ExpiCHO^{™} Expression Medium (purchased from Thermo Scientific, Cat. No. A2910002) was preheated at 37°C, 10 mL of ExpiCHO^{™} Expression Medium was taken and placed in a sterile tube and pre-cooled in a refrigerator at 4°C, the plasmid was centrifuged at 13.3×10³ g for 10 min; the cells with a viability of more than 95% was diluted to 6×10⁶ cells/ml with the preheated ExpiCHO^{™} Expression Medium in a 250ml shake flask, and then coated onto a 6-well plate. 50 µg of plasmid was diluted with 2 mL of 4°C pre-cooled culture medium, mixed well, 160 µL of ExpiFectamine^{™} CHO Reagent (purchased from Thermo Scientific, Cat. No. A29133) was diluted with 1.84 mL of pre-cooled culture medium, mixed well, then the diluted transfection reagent was added to the diluted plasmid and mixed well, allowed to stand at room temperature for 1 to 5 minutes, the mixture of plasmid and transfection reagent was added to the diluted cells, and cultured at 37°C, 5% CO₂; after 24 h, 300 µL of ExpiCHO^{™} Enhancer and 12 mL of ExpiCHO^{™} Feed (provided with the ExpiFectamine^{™} CHO Reagent transfection kit) were added, and cultured at 37°C, 5% CO₂ for 7 days.

### Tag-free purification of gE-gI fusion protein

Chromatographic purification was performed using AKTA system;
Instrument system: AKTA Pure preparative type liquid chromatograph;
Chromatographic purification on Q-FF column (Cytiva)
   (1) Expression sample was collected, and centrifuged at 7000 rpm for 10 min, and the supernatant was dialyzed into 50 mM TB8.0, and allowed to stand at 4°C overnight;
   (2) The protein sample was allowed to flow through the column at 8 mL/min, and then the column was equilibrated with 50 mM TB8.0;
   (3) 50 mM TB8.0 and 1 M NaCl were used to gradiently elute the target protein, and the elution fraction at 0.3 M NaCl was collected.
Chromatographic purification on DEAE-FF column (Cytiva)
(1) The above eluted sample was allowed to pass through the column at 5 mL/min, and then the column was equilibrated with 50 mM TB8.0;
   (2) The target protein was gradiently eluted using 50 mM TB8.0 and 1 M NaCl, and the elution fraction at 0.3 M NaCl was collected.
Chromatographic purification on Butyl column (Cytiva)
(1) The above eluted sample was dialyzed into 50 mM TB8.0/2 M NaCl;
   (2) The protein sample was allowed to pass through the column at 5 mL/min, and then the column was equilibrated with 50 mM TB8.0/2 M NaCl;
   (3) The target protein was gradiently eluted using 50 mM TB8.0.

### Purification of gE-gI fusion protein by affinity chromatography

AKTA system was used for Ni-affinity chromatography purification;
Instrument system: AKTA Pure preparative type liquid chromatograph;
Purification medium: Ni Sepharose 6 Fast Flow affinity medium; Buffers: including A pump buffer and B pump buffer; generally, A pump buffer was 1×PBS buffer (160 g/L NaCl, 8.1 mmol/L Na₂HPO₄, 1.5 mmol/L KH₂PO₄, 2.7mmol/L KCl, pH7.4), B pump buffer was 1×PBS + 250 mmol/L imidazole buffer;
System flow rate: 5 mL/min; Detection wavelength: UV@280 nm
Elution conditions: 50 mM imidazole buffer (obtained by diluting 250 mmol/L imidazole buffer with 1×PBS buffer) was used to elute impurity proteins, followed by washing with 1×PBS, and then the target protein (gE-gI fusion protein) was eluted with 250 mM imidazole buffer;
The product eluted with 250 mM imidazole was collected to obtain 40 mL of purified sample. The supernatant was centrifuged for purification.

### Purification of gE-gI fusion protein by size exclusion chromatography

Instrument system: AKTA explorer 100 preparative type liquid chromatography system produced by GE Healthcare (formerly Amershan Pharmacia).

Chromatographic medium: Superdex 200 increase (cytiva).

Column volume: 20 cm×20 mm.

Buffer: 20 mM phosphate buffer, pH7.4.

Flow rate: 0.7 mL/min.

Detector wavelength: 280 nm.

The sample to be loaded was the one purified by Ni-affinity chromatography.

The elution procedure was: penetration peaks were collected in fractionations.

The penetration of chromatography on Supedex 200 increase was collected to obtain 5 mL of purified sample.

### SDS-PAGE

50 µL of each resulting elution was taken, added with 30 µL of 6× Loading Buffer and mixed well. After being incubated in a water bath at 80°C for 10 minutes, 10 µL of the mixture was taken and electrophoresed on 10% SDS-polyacrylamide gel at 120 V for 120 minutes. Then Coomassie Brilliant Blue staining was performed to show the electrophoresis bands.

### Western Blot

(1) Transfer to membrane: After electrophoresis, a vertical protein transfer instrument was used to transfer the protein to a nitrocellulose membrane.
(2) Blocking: The nitrocellulose membrane was immersed in 50 mL of 1× blocking solution and incubated at 37°C for 1 h.
(3) Incubation with primary antibody: The blocking solution was recovered, the nitrocellulose membrane was rinsed 3 times with deionized water, immersed in 50 mL of primary antibody (VZV gE protein-specific monoclonal antibody 1B11, stored in this laboratory), and incubated at 37°C for 1h.
(4) Incubation with secondary antibody: The primary antibody was recovered, the nitrocellulose membrane was rinsed 4 times with 1×PBST, 5 min each time, and the nitrocellulose membrane was immersed in 50 mL of secondary antibody (GAH-HRP) and incubated at 37°C for 1 h.
(5) Color development: The secondary antibody was recovered, the nitrocellulose membrane was rinsed 4 times with 1×PBST, 5 min each time. Color development solutions A/B in equal volumes were mixed well and used to soak the membrane surface for color development and imaging.

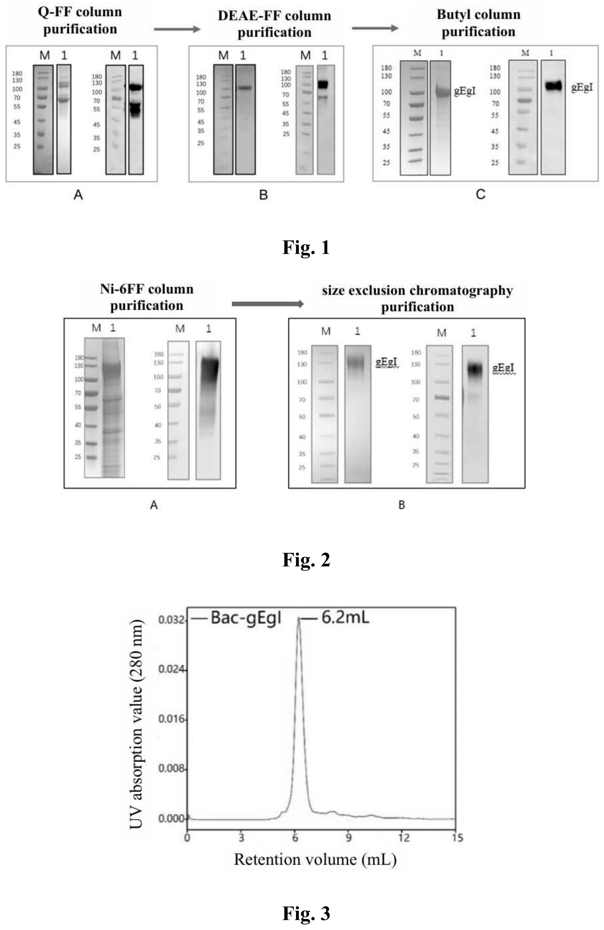

Protein expressed in insect cell: The results of SDS polyacrylamide gel electrophoresis (left panel) and Western Blot (right panel) of the gE-gI fusion protein samples at various purification stages (after purification with sequential chromatography on Q-FF column (Panel A in Fig. 1), on DEAE-FF column (Panel B in Fig.1) and on Butyl column (Panel C in Fig. 1)) were shown in Fig. 1, wherein lane M: molecular weight marker; lane 1: gE-gI fusion protein. The results showed that after three-column chromatography purification, the purity of the gE-gI fusion protein obtained was about 90%; and the Western Blot detection results based on VZV gE-specific monoclonal antibody showed positive.

Protein expressed in CHO cell: The results of SDS polyacrylamide gel electrophoresis (left panel) and Western Blot (right panel) of the gE-gI fusion protein samples at various purification stages (after sequential purification by chromatography on Ni-6FF column (Panel A in Fig. 2), and size exclusion chromatography on Superdex200 Increase (Panel B in Fig. 2)) were shown in Fig. 2, wherein lane M: molecular weight marker; lane 1: gE-gI fusion protein. The results showed that after two-column chromatography purification, the purity of the gE-gI fusion protein obtained was about 90%; the Western Blot detection results based on VZV gE-specific monoclonal antibody showed positive.

Example 2: High-performance size exclusion chromatography (HPSEC) analysis of gE-gl fusion protein

Instrument: Waters. System flow rate: G3000PW_{XL} was 0.5 mL/min. Wavelength was 190 to 600 nm, and column wavelengths were 280 nm and 254 nm.

### Buffer: PBS

Operational procedures: The chromatography column was pre-equilibrated for 30 to 60 min until there was no significant change in the absorption value at 280 nm, and the absorption value of the detector was set to zero. The chromatography operation method was edited, the sample was first centrifuged, the sample to be analyzed (gE-gI fusion protein obtained in Example 1) was injected into a 100 µL sample loop, and the instrument was set for automatic loading, and run for 30 min.

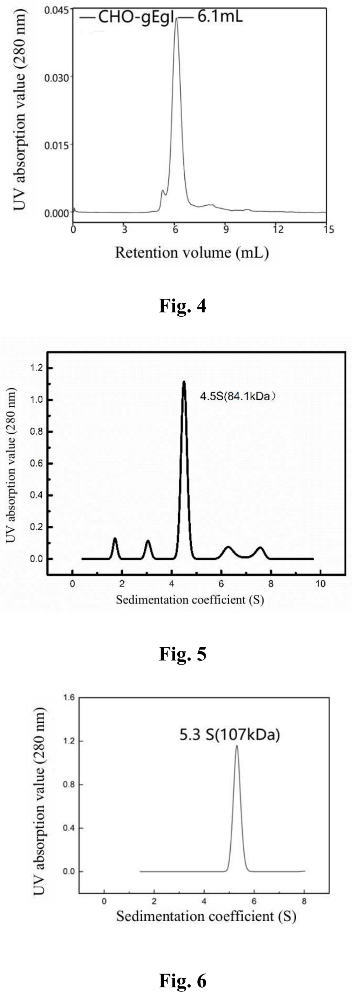

The results showed that the retention volume of the gE-gI fusion protein expressed by insect cells was about 6.2 mL, and the sample showed a single peak, and had uniformity and purity both higher than 90% (Fig. 3); the retention volume of the gE-gI fusion protein expressed by CHO cells was about 6.3 mL, and the sample showed a single peak, and had uniformity and purity both higher than 90% (Fig. 4).

Example 3: Calculation of sample sedimentation coefficient by analytical ultracentrifugation method

The instrument used was a Beckman XL-A analytical ultracentrifuge equipped with an optical detection system and an An-60Ti rotor.

The sample pool was installed according to the operating instructions, 400 µL of sample buffer (the same buffer used for sample) was added to the control pool, 380 µL of sample (the gE-gI fusion protein prepared in Example 1, OD280 was about 0.8) was added to the sample pool, the sample pool was balanced, and the weight difference was within 0.1g.

The sample pool was placed into the An-60Ti rotor, then the rotor was placed in the cavity of Beckman XL-A analytical ultracentrifuge, and the optical path detector was installed.

Parameter settings: temperature (20°C), Rmin (6.0 cm), Rmax (7.2 cm), wavelength (280 nm), step speed (0.003 cm), scan mode (continuous), data interval (30 sec) and number of data (150 scans). The centrifugal speed was set to 30000 rpm.

After the experiment was completed, SENDTERP software was used to calculate the density and viscosity of the buffer, as well as the partial specific volume of the known protein. The sedimentation coefficient was analyzed using the Origin version of Nonlin and SEDFIT software. The globulin friction ratio f/f0 was preset to 1.2. The analysis range was set according to the molecular weight and basic properties of sample protein. The calculation resolution was set to 100. Generally, the RMSD value was required to be no greater than 0.01 and the residue graph fluctuation was within 0.05.

The analytic ultracentrifugation results of the insect cell-derived gE-gI fusion protein were shown in Fig. 5. The results showed that the gE-gI fusion protein had a sedimentation coefficient of about 4.5 S and a molecular weight of about 84.1 kDa, which was in line with expectations, and existed in the form of monomer.

The analytic ultracentrifugation results of the CHO-derived gE-gI fusion protein were shown in Fig. 6. The results showed that the gE-gI fusion protein had a sedimentation coefficient of about 5.3 S and a molecular weight of about 107 kDa, which was in line with expectations, and existed in the form of monomer.

### Example 4: Evaluation of gE-gI fusion protein activity

ELISA operation process:
(1) The gE-gI fusion protein prepared in Example 1 was coated on a plate (200 ng/well), allowed to stand at 37°C for 2 h, the plate was washed once, and blocked with bovine serum albumin diluent (ED, 200 µL/well) at room temperature for 2 h;
(2) The plate was washed once, and gE protein-specific monoclonal antibodies (1B11, 4G4, 6H6, 6G7, 11B11, 11B12, 11E3, 14G1, 12E12, 13B6, 10H6, 17B7, 9H9; stored in this laboratory) and gI protein-specific monoclonal antibodies (18B2, 7E7, 9D11, 16F1, 13B2, 19F2, 14B11, 12D2; stored in this experiment) were separately diluted with SD-1 diluent, 5 µg/mL in the first well, 2-fold gradient dilution, and allowed to stand at 37°C for 1 h;
(3) The plate was washed 5 times, the secondary antibody GAH-HRP (1:5000) was added to the 96-well plate, 100 µL/well, and allowed to stand at room temperature for 1 h;
(4) The plate was washed 5 times, and subjected to color development at 37°C for 10 min; after stopping, detection was performed at 450 nm wavelength on an ELISA reader; and GraphPad Prism 5 (GraphPad, USA) software was used for data analysis.

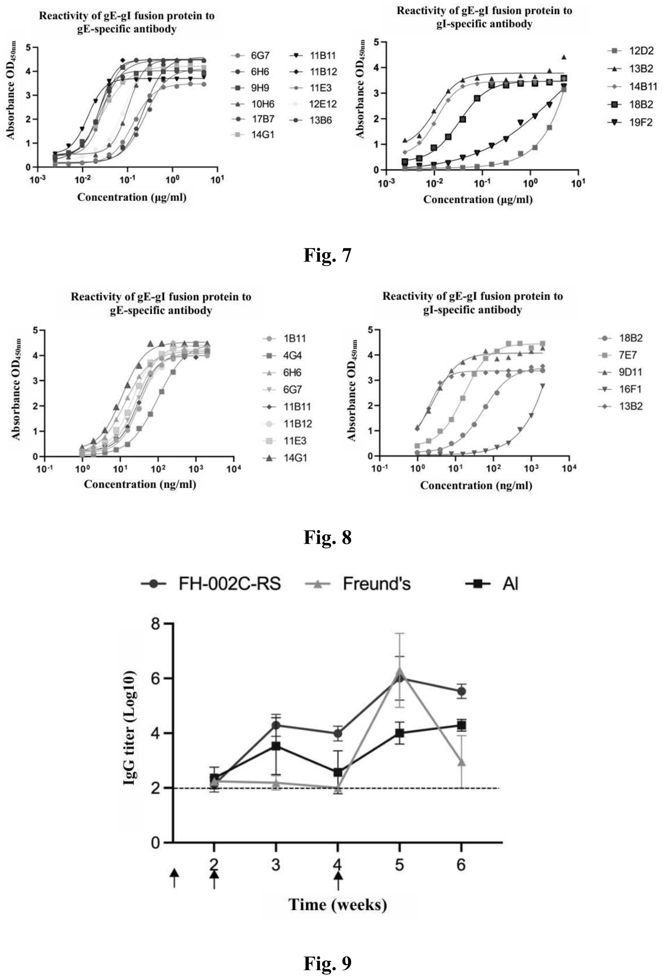

The ELISA results were shown in Fig. 7. Fig. 7 showed that the insect cell-derived gE-gI fusion protein prepared in Example 1 had good reactivity with both the gE protein-specific monoclonal antibodies and the gI protein-specific monoclonal antibodies.

The ELISA results were shown in Fig. 8. Fig. 8 showed that the CHO-derived gE-gI fusion protein prepared in Example 1 had good reactivity with both the gE protein-specific monoclonal antibodies and the gI protein-specific monoclonal antibodies.

### Example 5: Evaluation of gE-gI fusion protein immunogenicity

This experimental scheme was approved by the Experimental Animal Management Ethics Committee of Xiamen University. All operations were carried out strictly in accordance with the animal ethics guidelines and the approved procedures.

Six-week-old Balb/C mice were selected and divided into 4 groups, each with 5 mice. In each group, the gE-gI fusion protein prepared in Example 1 (immunization dose was 1 µg), and combination thereof with FH002C adjuvant (FH002C adjuvant, see PCT application No. PCT/CN2021/130588 (the full text of which was incorporated herein by reference) or journal literature Wu Y, et al. A recombinant spike protein subunit vaccine confers protective immunity against SARS-CoV-2 infection and transmission in hamsters. Sci Transl Med. 2021 Aug 11;13(606): eabg1143. DOI: 10.1126/scitranslmed.abg1143 (the full text of which was incorporated herein by reference)) or aluminum adjuvant (prepared in this laboratory) or 50% Freund's complete adjuvant (purchased from Sigma Aldrich, Cat. No.: F5506) were used for immunization, and intramuscular injection (150 µL) was performed into the left or right hind limb of mice at 0-, 1-, and 4-week. Ocular venous blood was collected at 0-, 1-, 2-, 3-, 4-, and 5-week, respectively, and blood was collected before injection at 0-, 1-, and 4-week. Blood samples were centrifuged at 13,000 g for 10 minutes, and serum samples were stored at -20°C. Antigen-specific IgG titers and neutralizing antibody titers were determined by endpoint enzyme-linked immunosorbent assay and vOka virus-based neutralization assay, respectively.

Neutralization operation process (ELISPOT assay):
(1) Guinea pig serum (purchased from Beijing Bersee Science and Technology Co., Ltd., Cat. No.: BM361Y) and vOka virus (purchased from Beijing Wantai Biological Pharmacy Enterprise Co., Ltd.) dry powders were redissolved with virus protection solution, and the complement was filtered with a 0.22 µm small filter for later use;
(2) The serum was diluted 50-fold with virus protection solution and added to the first well of a 24-well plate, subjected to 2-fold serial dilution in 4 gradients, and incubated with vOka virus at 37°C for 1 h;
(3) The serum-virus mixture was transferred to a 24-well plate pre-coated with ARPE-19 cells, incubated at 37°C for 1 h, and the liquid was discarded after 1 h and supplemented with F12 culture medium, and cultured at 37°C for 3 days;
(4) After 3 days, the culture medium was discarded, the plate was washed with PBS once; the fixation was performed using a fixative at room temperature for 5 min, the fixative was discarded, and the permeabilization was performed using a permeabilization solution at room temperature for 10 minutes;
(5) The primary antibody 1B11-HRP (1:2000) was added to a 24-well plate and allowed to stand at 37°C for 1 hour;
(6) The plate was washed 5 times, and subjected to color development at room temperature for 5 minutes, spots were read and counted using an enzyme-linked immunospot image analysis system; and GraphPad Prism 5 (GraphPad, USA) software was used for data analysis.

Neutralization operation process (plaque assay):
(1) Guinea pig serum and vOka virus dry powder were redissolved with virus protection solution, complement was filtered with a 0.22 µm small filter for later use;
(2) Serum was diluted 50-fold with virus protection solution and added to the first well of a 24-well plate, subjected to 2-fold serial dilution in 4 gradients, and incubated with vOka virus at 37°C for 1h;
(3) The serum-virus mixture was transferred to a 24-well plate pre-coated with ARPE-19 cells, and incubated at 37°C for 1 h, the liquid was discarded and supplemented with F12 culture medium after 1 h, and culturing was performed at 37°C for 7 to 10 days until plaques were formed;
(4) The culture medium was discarded, washing was performed once with PBS, and fixation was performed with a fixative solution at room temperature for 5 min;
(5) Staining was performed with crystal violet dye for 10 min, washing was performed with deionized water, spots were counted manually; and data analysis was performed using GraphPad Prism 5 (GraphPad, USA) software.

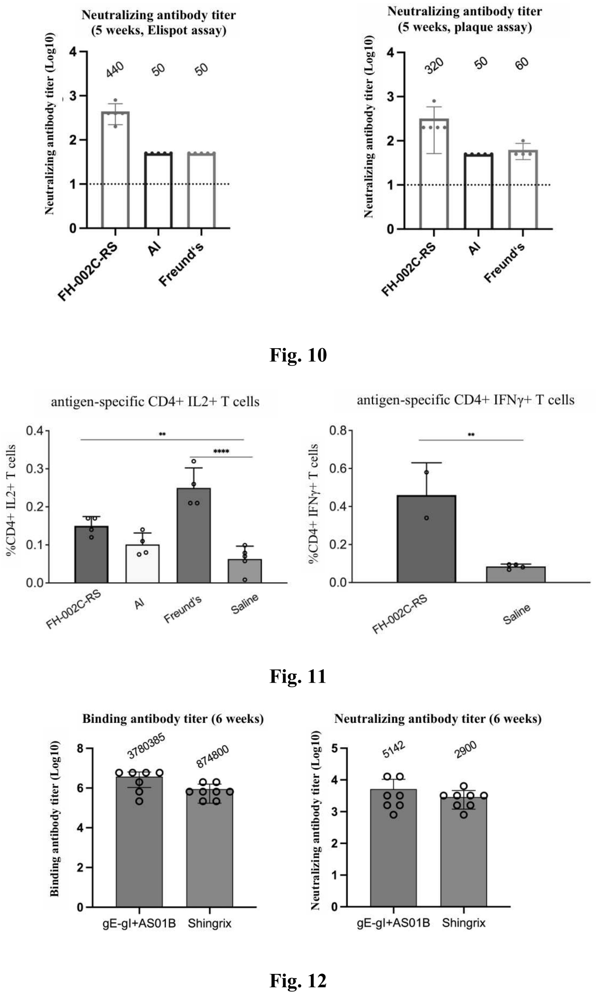

The antigen-specific IgG titers and neutralizing antibody titers in serum induced by insect cell-derived fusion proteins combined with different adjuvants were shown in Fig. 9 and Fig. 10, respectively. The results in Fig. 9 showed that after three injections of immunization, all mice showed IgG serum conversion in the second week, and the IgG titer increased over time. In the fifth week, all mice showed high IgG antibody titers, wherein the IgG titer in the aluminum adjuvant group was the lowest, and the IgG antibody titer in the FH002C adjuvant group was relatively high. Fig. 10 showed that all the mice in each adjuvant group showed neutralizing antibody titers, in which the neutralizing antibody titer in the FH002C adjuvant group was higher than that in the aluminum adjuvant group and the Freund's adjuvant group. Fig. 9 and Fig. 10 showed that the gE-gl fusion proteins possess high immunogenicity even at a low immunization dose.

### Example 6: Detection of cytokines by flow cytometry

According to the method described in Example 6, mice were immunized and the subsequent experimental operations were as follows:
a) Taking spleen: The mice were killed by cervical dislocation, and soaked in 75% ethanol for 3 to 5 minutes. The mice were placed on their right sides, and their spleens were taken out aseptically (fat was removed as much as possible);
b) Grinding: A 6-well cell plate was placed in a mesh, 1640 culture medium containing 10% FBS was added, the spleen was placed in the mesh (completely immersed in the culture medium) and ground with a 2 mL syringe head until no red tissue was visible, and the ground cells were transferred to a 50 mL tube and placed on ice;
c) The cells were centrifuged at 400 g and 4°C for 5 min, the supernatant was discarded, and the cell pellet was loosen by tapping the bottom of tube with a hemostatic forceps;
d) The cell pellet was added with 10 mL of pre-cooled RBC solution, resuspended, and placed on ice for 5 min for lysis (inverted and mixed during this period);
e) The cells were centrifuged at 400 g and 4°C for 5 min, the supernatant was discarded, and the cell pellet was loosen by tapping the bottom of tube with a hemostatic forceps;
f) The cell pellet was added with 10 mL of pre-cooled culture medium and resuspended, the red blood cell pellet or fat was washed away; after being pipetted evenly, 50 µl of the cells were taken for counting;
g) The cells were centrifuged at 400g and 4°C for 5 min, the supernatant was discarded, the cell pellet was loosen by tapping the bottom of tube with a hemostatic forceps, a certain amount of culture medium was added for dilution to reach a cell density of 2×10⁷ cells/mL;
h) Plating: A 96-well U-bottom plate was plated with 200 µL (2~4×10⁶ cells) per well, and centrifuged at 400 g and 4°C for 5 min, and the supernatant was discarded;
i) 100 µL of culture medium containing polypeptide ((gE/gI) overlapping polypeptide, diluted with FACS solution, the final concentration of polypeptide was 2 µg/mL) was added to resuspend the cells, and stimulation was performed for 18 h;
j) 20 µL of Golgi inhibitor (1:1000, diluted with culture medium) and incubated for 6 h;
k) Centrifugation was performed at 400 g and 4°C for 2 min, the supernatant was discarded, and 200 µL of FACS solution (1×PBS+10% FBS) was added to resuspend the cells; then the cells were centrifuged, and the supernatant was discarded;

(All the following operations were performed in the dark and kept at 4°C)
1) Cell surface staining: Staining was performed with FITC-conjugated anti-mouse CD4 antibody (purchased from Biolegend, Cat. No.: 100406), PE-Cy7-conjugated anti-mouse CD8α antibody (purchased from Biolegend, Cat. No.: 100722), and LIVE/DEAD^{™} fixable Aqua dead cell staining reagent (purchased from Biolegend, Cat. No.: 103248), 40 µL of surface staining reagent (AQUA/CD4/CD8 antibody, diluted with FACS solution) was added to each well to resuspend the cells, the treatment was carried out at 4°C in the dark for 30 to 60 min, then 200 µL of FACS solution was added and pipetted 7 to 8 times;
m) Fixation/Permeabilization: Centrifugation was performed at 2000 rpm and 4°C for 2 min, the supernatant was discarded, 75 µL of Fixation/Permeabilization solution was added to each well to resuspend the cells, and the treatment was carried out at 4°C in the dark for 60 min;
n) Centrifugation was performed at 2000 rpm and 4°C for 2 min, and the supernatant was discarded;
o) 200 µL of 1×BD Perm/Wash solution was added to each well to resuspend the cells, centrifugation was performed at 2000 rpm and 4°C for 2 min, and the supernatant was discarded;
p) Intracellular staining: Staining was performed with PE-conjugated anti-mouse IL-2 antibody (purchased from BD, Cat. No.: 554428) and APC-conjugated anti-mouse IFN-γ antibody (purchased from BD, Cat. No.: 554413), 50 µL of cytokine antibody (IL-2/IFN-γ antibody, diluted with 1×BD Perm/Wash solution) was added to each well to resuspend the cells, the treatment was carried out at 4°C in the dark for 60 min, and 200 µL of Perm Buffer was added to resuspend the cells;
q) Centrifugation was performed at 2000 rpm and 4°C for 2 min, the supernatant was discarded, and 200 µL of 1×BD Perm/Wash solution was added to each well to resuspend the cells;
r) The treated cells were subjected to filtration with a 200-mesh screen (the screen strips were placed on the surface of wells, and the cell suspension were pipetted with a pipette and added vertically and slowly), and transferred to flow cytometer tubes;
s) The samples were measured using a BD LSRFortessa X-20 flow cytometer, and the data were analyzed using FlowJo V10.

The experimental results were shown in Fig. 11, which showed that the gE-gI fusion protein combined with different adjuvants could all induce T cell immune responses.

Example 7: Parallel comparison experiment of insect cell-derived gE-gI fusion protein combined with AS01B adjuvant and Shingrix vaccine

This experimental scheme was approved by the Experimental Animal Management Ethics Committee of Xiamen University. All operations were carried out in strict accordance with the animal ethics guidelines and the approved procedures.

Six-week-old Balb/C mice were selected and divided into three groups, with 8 mice in each group. The experimental group was immunized with the insect cell-derived gE-gI fusion protein (immunization dose was 5 µg) prepared in Example 1 and the purchased AS01B adjuvant (purchased from GSK, batch number: 4N2AB), the control group was immunized with Shingrix vaccine (purchased from GSK, batch number: 4N2AB), and the blank group was immunized with saline. The mice were injected intramuscularly (50 µL) into the tibia muscle at 0- and 4-week, respectively. Ocular venous blood was collected at 0-, 2-, 4-, 6- and 8-week, respectively, wherein the blood collection at 0- and 4-week was performed before the injection. The blood samples were centrifuged at 13000 g for 10 minutes, and the serum samples were stored at -20°C. The titers of antigen-specific IgG and neutralizing antibodies were determined by end-point enzyme-linked immunosorbent assay and vOka virus-based neutralization assay, respectively.

The results were shown in Fig. 12. The results in Fig. 12 showed that both the binding antibody IgG titer and neutralizing antibody titer induced by the gE-gI fusion protein combined with the AS01B adjuvant were higher than those of the Shingrix vaccine; in which, the neutralizing antibody titer induced by the gE-gI fusion protein combined with the AS01B adjuvant was about 1.8 times that of the Shingrix vaccine.

At week 8, spleen was harvested for flow cytometry and Elispot assay of T cell response detection, wherein the flow cytometry was carried as the same in Example 6, and the ELISPOT cytokine detection process (the kit used was purchased from MABTECH, Cat. No.: 3441-4HPW-10 and 3321-4HPW-10) was follows:
(1) Splenocytes were isolated, and then 500,000 cells were plated per well. The supernatant was removed by centrifugation, and 100 µL of culture medium containing polypeptides ((gE/gI) overlapping polypeptide) was added to resuspend the cells, and stimulation was performed at 37°C for 20 h;
(2) The culture medium was discarded, and the cells were washed with sterile PBS for 5 times. The detection antibodies R4-6A2-biotin (for IFN-γ detection) and 5H4-biotin (for IL-2 detection) were diluted to 1 µg/mL, added at 100 µL per well, and incubated at room temperature for 2 h;
(3) The cells were washed with sterile PBS for 5 times, and 100 µL of Streptavidin-ALP (1: 1000) was added to each well, and incubated at room temperature for 1 h;
(4) The cells were washed with sterile PBS for 5 times, and 100 µL of substrate solution (BCIP/NBT-plus) was added to each well for color development, until spots appeared, and then the reaction was stopped by washing the plate;
(5) The plate was reversed and dried, then the spots were read and counted using an enzyme-linked immunospot image analysis system; and GraphPad Prism 5 (GraphPad, USA) software was used for data analysis.

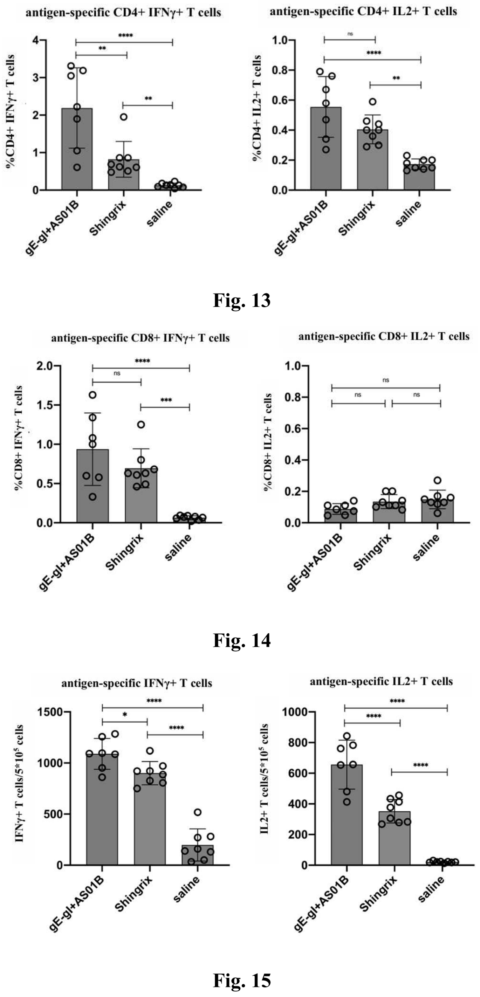

The experimental results were shown in Figs. 13 to 15, in which Fig. 13 showed the results of CD4⁺ T cell response stimulated by two different vaccines in this example. The results showed that the levels of antigen-specific CD4⁺IFN-γ⁺ T cells and CD4⁺IL-2⁺ T cells induced by the insect cell-derived gE-gI fusion protein combined with AS01B adjuvant were higher than those of the Shingrix vaccine, the proportion of CD4⁺IFN-γ⁺ T cells was twice that of the Shingrix vaccine, and there were significant differences.

Fig. 14 showed the results of CD8⁺ T cell response stimulated by two different vaccines in this example. The results showed that the gE-gI specific CD8⁺IFN-γ⁺ T cells induced by the gE-gI fusion protein combined with AS01B adjuvant were higher than those of the Shingrix vaccine.

Fig. 15 showed the levels of cytokines IFN-γ and IL-2 induced by two different vaccines in this example, as detected by Elispot assay. The results showed that the levels of IFN-γ and IL-2 induced by the gE-gI fusion protein combined with AS01B adjuvant were higher than those of the Shingrix vaccine, and there were significant differences.

Example 8: Parallel comparison experiment of CHO-derived gE-gI fusion protein combined with AS01B adjuvant and Shingrix vaccine

This experimental scheme was approved by the Experimental Animal Management Ethics Committee of Xiamen University. All operations were carried out in strict accordance with the animal ethics guidelines and the approved procedures.

Six-week-old Balb/C mice were selected and divided into three groups, with 8 mice in each group. The experimental group was immunized with the CHO-derived gE-gI fusion protein (immunization dose was 5 µg) prepared in Example 1 and the purchased AS01B adjuvant (purchased from GSK, batch number: 4N2AB), the control group was immunized with Shingrix vaccine (purchased from GSK, batch number: 4N2AB), and the blank group was immunized with saline. The mice were injected intramuscularly (50 µL) into tibialis muscle at 0- and 4-week, respectively. Ocular venous blood was collected at 0-, 2-, 4-, 6- and 8-week, respectively, wherein the blood collection at 0- and 4-week was performed before the injection. The blood samples were centrifuged at 13000 g for 10 minutes, and the serum samples were stored at -20°C. The titers of antigen-specific IgG and neutralizing antibodies were determined by end-point enzyme-linked immunosorbent assay and vOka virus-based neutralization assay, respectively.

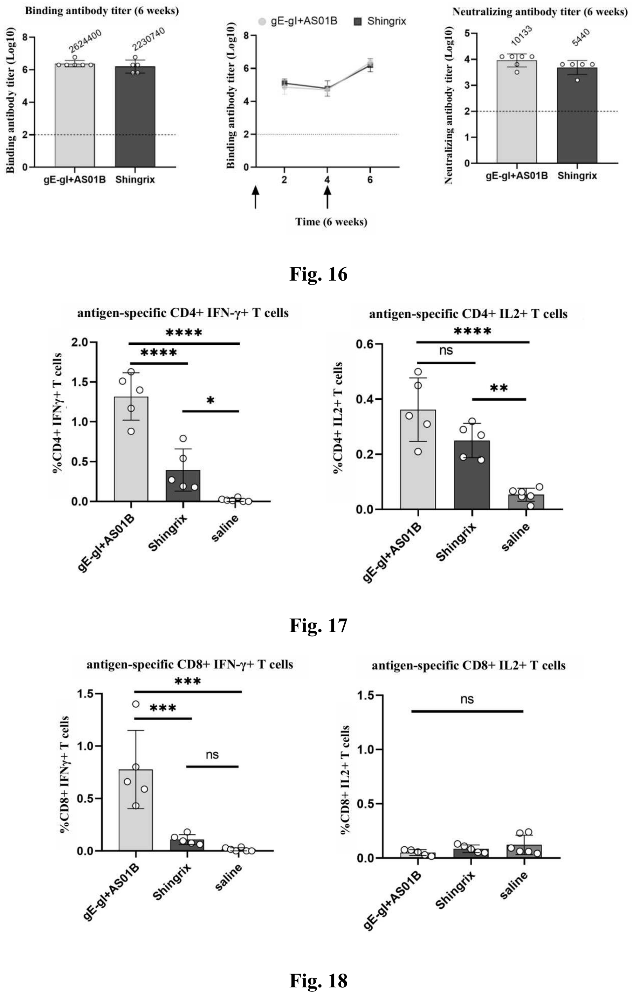

The results were shown in Fig. 16. The results in Fig. 16 showed that the binding antibody IgG induced by the CHO-derived gE-gI fusion protein combined with AS01B adjuvant were equivalent to those of the Shingrix vaccine; the titer of neutralizing antibodies induced by the gE-gI fusion protein combined with AS01B adjuvant was about twice that of the Shingrix vaccine.

The flow cytometry procedure referred to Examples 6 and 7.

The experimental results were shown in Fig. 17, wherein Fig. 17 showed the results of CD4⁺ T cell responses stimulated by two different vaccines in this example. The results showed that the antigen-specific CD4⁺IFN-γ⁺ T cells and CD4⁺IL-2⁺ T cells induced by the CHO-derived gE-gI fusion protein combined with AS01B adjuvant were higher than those of the Shingrix vaccine, and there were significant differences, in which the proportion of CD4⁺IFN-γ⁺ T cells was three times that of the Shingrix vaccine.

Fig. 18 showed the results of CD8⁺ T cell responses stimulated by two different vaccines in this example. The results showed that the gE-gI specific CD8⁺IFN-γ⁺ T cells induced by the gE-gI fusion protein combined with the AS01B adjuvant were higher than those of the Shingrix vaccine, and there were significant differences, in which the proportion of CD8⁺IFN-γ⁺ T cells were about seven times those of the Shingrix vaccine.

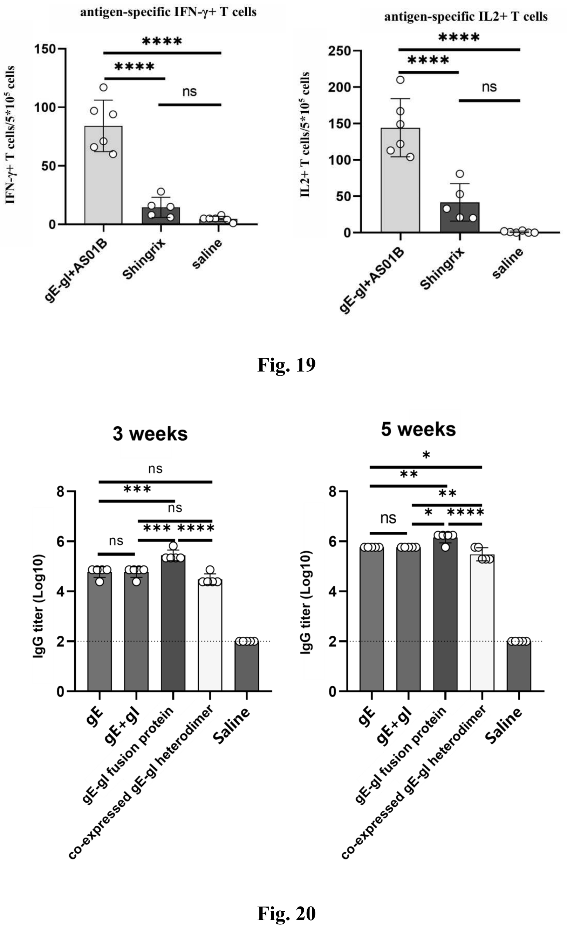

Fig. 19 showed the levels of cytokines IFN-γ and IL-2 induced by the two different vaccines in this example. The results showed that the proportions of gE-gI specific IFN-γ⁺ T cells and IL-2⁺ T cells induced by the gE-gI fusion protein combined with the AS01B adjuvant were higher than those of the Shingrix vaccine, and there were significant differences.

Example 9: Parallel comparison experiment of different forms of gE-gI protein combined with AS01B-like adjuvant

This experimental scheme was approved by the Experimental Animal Management Ethics Committee of Xiamen University. All operations were carried out in strict accordance with the animal ethics guidelines and the approved procedures.

Six-week-old C57BL/6 mice were selected and divided into five groups, with five mice in each group. The experimental group was immunized with the gE-gI fusion protein prepared in Example 1 (immunization dose was 8.3 µg) combined with AS01B-like adjuvant (prepared in this laboratory; for the preparation method, see Didierlaurent AM, et al. Enhancement of adaptive immunity by the human vaccine adjuvant AS01 depends on activated dendritic cells. J Immunol. 2014 Aug 15;193(4): 1920-30. doi: 10.4049/jimmunol.1400948. Epub 2014 Jul 14.; Dendouga N, et al. Cell-mediated immune responses to a varicella-zoster virus glycoprotein E vaccine using both a TLR agonist and QS21 in mice. Vaccine. 2012 Apr 26;30(20): 3126-35. doi: 10.1016/j.vaccine.2012.01.088. Epub 2012 Feb 10., which was incorporated herein by reference in its entirety), the control groups were immunized with gE protein alone (immunization dose was 5 µg), gE protein+gI protein (immunization dose was 5 µg+3.3 µg) and co-expressed gE-gI heterodimer (the preparation method referred to the literature pmid: 9697709, and the immunization dose was 8.3 µg), each in combination with the AS01B-like adjuvant (prepared in this laboratory), and the blank group was immunized with saline. The mice were injected intramuscularly (50 µL) into tibialis muscle at 0- and 3-week, respectively. Ocular venous blood was collected at 0-, 3- and 5-week, respectively, wherein the blood collection at 0- and 3-week was performed before the injection. The blood samples were centrifuged at 13000 g for 10 minutes, and the serum samples were stored at -20°C. The titers of antigen-specific IgG and neutralizing antibodies were determined by endpoint enzyme-linked immunosorbent assay and vOka virus-based neutralization assay, respectively. The results were shown in Fig. 20. The results in Fig. 20 showed that the titer of binding antibody IgG induced by the gE-gI fusion protein combined with AS01B-like adjuvant were significantly higher than those of the gE protein alone, gE protein+gI protein, and co-expressed gE-gI heterodimer.

In the eighth week, spleens were harvested for flow cytometry and Elispot assay of T cell responses, wherein the flow cytometry was carried as the same in Example 6, and the ELISPOT cytokine detection process was the same in Example 7.

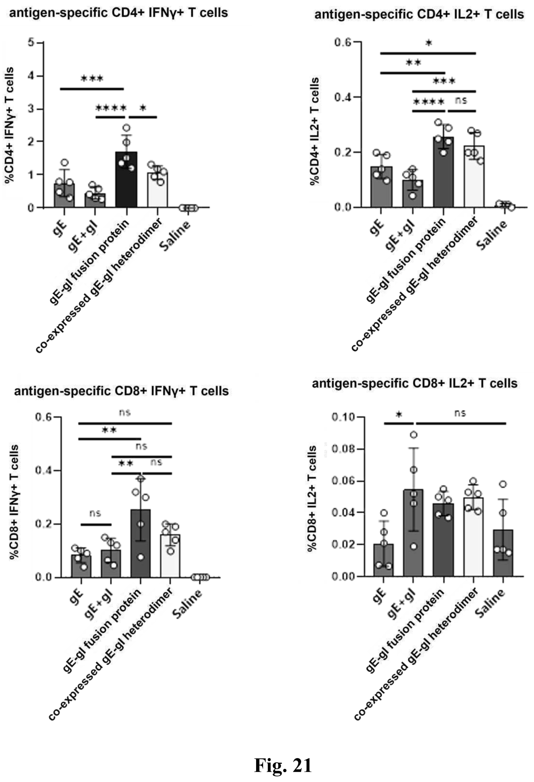

The experimental results were shown in Figs. 21 to 22, wherein Fig. 21 showed the results of CD4⁺ T and CD8⁺ T cell responses stimulated by the four different vaccines in this example. The results showed that the level of the gE-gI specific CD4⁺IFN-γ⁺ T cells induced by the gE-gI fusion protein combined with AS01B-like adjuvant was significantly higher than those of the gE protein alone, gE protein+gI protein and co-expressed gE-gI heterodimer, and the levels of the CD4⁺IL-2⁺ T cells and CD8⁺IFN-γ⁺ T cells were significantly higher than those of the gE protein alone and gE protein+gI protein, and slightly higher than those of the co-expressed gE-gI heterodimer.

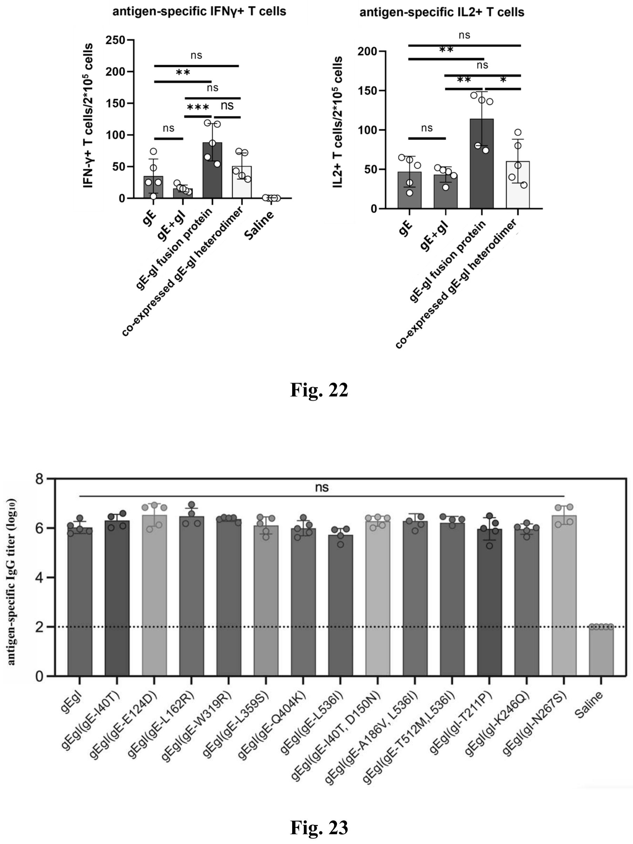

Fig. 22 showed the levels of cytokines IFN-γ and IL-2 induced by the stimulation with the four different vaccines in this example, as detected by Elispot assay. The results showed that the levels of IFN-γ and IL-2 induced by the gE-gI fusion protein combined with AS01B-like adjuvant were higher than those of the gE protein alone, gE protein+gI protein and co-expressed gE-gI heterodimer, and there were significant differences.

Example 10: Parallel comparison experiment of gE-gI fusion proteins based on different strains combined with AS01B-like adjuvant

The gE full-length amino acid sequence (SEQ ID NO.11) involved in Examples 1 to 9 of the present application was completely consistent with the gE protein sequence of pOka strain (GenBank: AB097933) and vOka strain (GenBank: AB097932). The gI sequence involved in Examples 1 to 9 of the present disclosure was completely consistent with the gI protein sequence of pOka strain (GenBank: AB097933), vOka strain (GenBank: AB097932), Dumas strain (GenBank: NC001348), and MSP strain (GenBank: AY548170).

In this example, the immunogenicity of the gE-gI fusion proteins constructed based on gE and gI from different strains was further evaluated, and the information of the fusion protein constructs involved was shown in the following table:

**Table 3: Structure information of fusion proteins**

| Fusion protein | N-terminal to C-terminal | | |
|---|---|---|---|
| gEgI | gE (aa. 23-537) | linker | gI (aa. 21-271) |
| SEQ ID NO: 6 | SEQ ID NO: 13 | SEQ ID NO: 15 | SEQ ID NO: 14 |
| gEgI(gE-I40T) | gE (aa. 23-537, 140T) | linker | gI (aa. 21-271) |
| SEQ ID NO: 20 | SEQ ID NO: 33 | SEQ ID NO: 15 | SEQ ID NO: 14 |
| gEgI(gE-E124D) | gE (aa. 23-537, E124D) | linker | gI (aa. 21-271) |
| SEQ ID NO: 21 | SEQ ID NO: 34 | SEQ ID NO: 15 | SEQ ID NO: 14 |
| gEgI(gE-L 162R) | gE (aa. 23-537, L162R) | linker | gI (aa. 21-271) |
| SEQ ID NO: 22 | SEQ ID NO: 35 | SEQ ID NO: 15 | SEQ ID NO: 14 |
| gEgI(gE-W319R) | gE (aa. 23-537, W319R) | linker | gI (aa. 21-271) |
| SEQ ID NO: 23 | SEQ ID NO: 36 | SEQ ID NO: 15 | SEQ ID NO: 14 |
| gEg1(gE-L359S) | gE (aa. 23-537, L359S) | linker | gI (aa. 21-271) |
| SEQ ID NO: 24 | SEQ ID NO: 37 | SEQ ID NO: 15 | SEQ ID NO: 14 |
| gEgI(gE-Q404K) | gE (aa. 23-537, Q404K) | linker | gI (aa. 21-271) |
| SEQ ID NO: 25 | SEQ ID NO: 38 | SEQ ID NO: 15 | SEQ ID NO: 14 |
| gEgI(gE-L536I) | gE (aa. 23-537, L536I) | linker | gI (aa. 21-271) |
| SEQ ID NO: 26 | SEQ ID NO: 39 | SEQ ID NO: 15 | SEQ ID NO: 14 |
| gEgI(gE-I40T, D150N) | gE (aa. 23-537, 140T&DI50N) | linker | gI (aa. 21-271) |
| SEQ ID NO: 27 | SEQ ID NO: 40 | SEQ ID NO: 15 | SEQ ID NO: 14 |
| gEgI(gE-A186V, L536I) | gE (aa. 23-537, A186V&L5361) | linker | gI (aa. 21-271) |
| SEQ ID NO: 28 | SEQ ID NO: 41 | SEQ ID NO: 15 | SEQ ID NO: 14 |
| gEgI(gE-T512M, L536I) | gE (aa. 23-537, T512M&L536I) | linker | gI (aa. 21-271) |
| SEQ ID NO: 29 | SEQ ID NO: 42 | SEQ ID NO: 15 | SEQ ID NO: 14 |
| gEgI(gI-T211P) | gE (aa. 23-537) | linker | gI (aa. 21-271, T211P) |
| SEQ ID NO: 30 | SEQ ID NO: 13 | SEQ ID NO: 15 | SEQ ID NO: 43 |
| gEgI(gI-K246Q) | gE (aa. 23-537) | linker | gI (aa. 21-271, K246Q) |
| SEQ ID NO: 31 | SEQ ID NO: 13 | SEQ ID NO: 15 | SEQ ID NO: 44 |
| gEgI(gI-N267S) | gE (aa. 23-537) | linker | gI (aa. 21-271, N267S) |
| SEQ ID NO: 32 | SEQ ID NO: 13 | SEQ ID NO: 15 | SEQ ID NO: 45 |

Among them, gE (aa. 23-537, I40T) is derived from the Dumas strain (GenBank: NC001348), which contains the I40T mutation as compared to SEQ ID NO: 13; gE (aa. 23-537, I40T&D150N) is derived from the MSP strain (GenBank: AY548170), which contains the I40T&D150N mutation as compared to SEQ ID NO: 13; gE (aa. 23-537, T512M&L536I) is derived from the gE protein shown in GenBank: AEW88980, which contains the T512M&L5361 mutation as compared to SEQ ID NO: 13; gE (aa. 23-537, Q404K) is derived from the gE protein shown in GenBank: AEW89124, which contains the Q404K mutation as compared to SEQ ID NO: 13; gE (aa. 23-537, W319R) is derived from the gE protein shown in GenBank: QCA43570, which contains the W319R mutation as compared to SEQ ID NO: 13; gE (aa. 23-537, E124D) is derived from the gE protein shown in GenBank: QCA45176, which contains the E124D mutation as compared to SEQ ID NO: 13; gE (aa. 23-537, L359S) is derived from the gE protein shown in GenBank: QWE79571, which contains the L359S mutation as compared to SEQ ID NO: 13; gE (aa. 23-537, L162R) is derived from the gE protein shown in GenBank: AEW89412, which contains the L162R mutation as compared to SEQ ID NO: 13; gE (aa. 23-537, A186V&L536I) is derived from the gE protein shown in GenBank: ANS12941, which contains the A186V&L5361 mutations as compared to SEQ ID NO: 13; gE (aa. 23-537, L536I) is derived from the gE protein shown in GenBank: AQT34120, which contains the L5361 mutation as compared to SEQ ID NO: 13. gI (aa. 21-271, K246Q) is derived from the gI protein shown in GenBank: AEW89051, which contains the K246Q mutation as compared to SEQ ID NO: 14; gI (aa. 21-271, T211P) is derived from the gI protein shown in GenBank: AEW89483, which contains the T211P mutation as compared to SEQ ID NO: 14; gI (aa. 21-271, N267S) is derived from the gI protein shown in GenBank: AGY34059, which contains the N267S mutation as compared to SEQ ID NO: 14.

In this example, the fusion proteins shown in Table 3 were purified and immunologically evaluated according to the methods described in Example 1 and Examples 5 and 6.

Fig. 23 showed the titers of antigen-specific IgG induced after two injections of the fusion proteins constructed based on different mutant strains in this example. The results showed that there was no significant difference in the levels of the IgG induced by the fusion proteins constructed based on different mutant strains combined with AS01B-like adjuvant, which were equivalent to that of the gE-gI fusion protein as set forth in SEQ ID NO: 6.

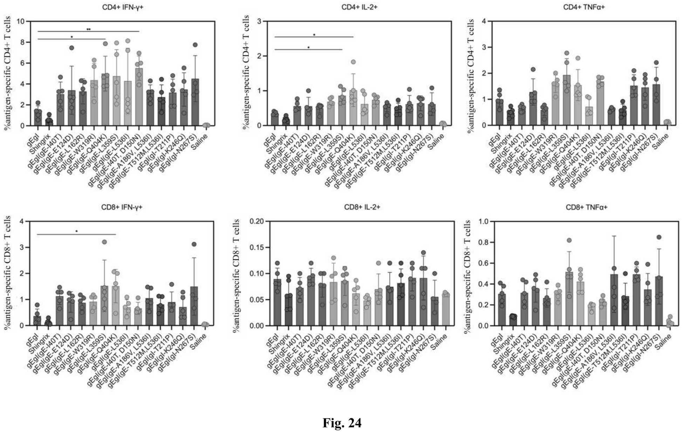

Fig. 24 showed the antigen-specific T cell response induced after two injections of the fusion proteins constructed based on different mutant strains in this example. The results showed that the gE-gI fusion proteins constructed based on different mutant strains could produce higher CD4⁺ T cell and CD8⁺ T cell responses as compared to Shingrix, among which the fusion protein based on certain strains could also induce higher proportion of CD4⁺IFN-γ⁺ T cells (e.g., gE-gI (gE-Q404K), gE-gI (gE-I40T, D150N)), CD4⁺IL2⁺ T cells (e.g., gE-gI (gE-L359S)), and CD8⁺IFN-γ⁺ T cells (e.g., gE-gI (gE-Q404K)), as compared to the gE-gI fusion protein as set forth in SEQ ID NO: 6. This indicated that the gE-gI fusion proteins constructed based on gE and gI of different strains possess significantly excellent immunogenicity and could induce higher immune responses as compared to Shingrix.

### Example 11: Design and preparation of truncated gE-gI fusion protein

In this experiment, the gE-gI fusion protein with truncated gE length was expressed, in which the length of gI was kept unchanged, the N-terminal and C-terminal of gE were truncated at the same time to construct the gE-gI fusion protein, and the immune activity of the gE-gI fusion protein was evaluated. The following 5 clones were designed: gE(43-517)-gI, gE(63-497)-gl, gE(83-477)-gl, gE(103-457)-gl, and gE(143-417)-gl. The information of the above five fusion proteins was as follows:

**Table 4: Molecular information of fusion protein**

| Fusion protein | N-terminal to C-terminal | | |
|---|---|---|---|
| gE(43-517)-gI | gE (aa. 43-517) | linker | gI (aa. 21-271) |
| SEQ ID NO: 59 | SEQ ID NO: 64 | SEQ ID NO: 15 | SEQ ID NO: 14 |
| gE(63-497)-gI | gE (aa. 63-497) | linker | gI (aa. 21-271) |
| SEQ ID NO: 60 | SEQ ID NO: 65 | SEQ ID NO: 15 | SEQ ID NO: 14 |
| gE(83-477)-gI | gE (aa. 83-477) | linker | gI (aa. 21-271) |
| SEQ ID NO: 61 | SEQ ID NO: 66 | SEQ ID NO: 15 | SEQ ID NO: 14 |
| gE(103-457)-gI | gE (aa. 103-457) | linker | gI (aa. 21-271) |
| SEQ ID NO: 62 | SEQ ID NO: 67 | SEQ ID NO: 15 | SEQ ID NO: 14 |
| gE(143-417)-gI | gE (aa. 143-417) | linker | gI (aa. 21-271) |
| SEQ ID NO: 63 | SEQ ID NO: 68 | SEQ ID NO: 15 | SEQ ID NO: 14 |

The fusion proteins were prepared according to Example 1. The obtained fusion proteins constructed by the truncated gE proteins were subjected to Western Blot.

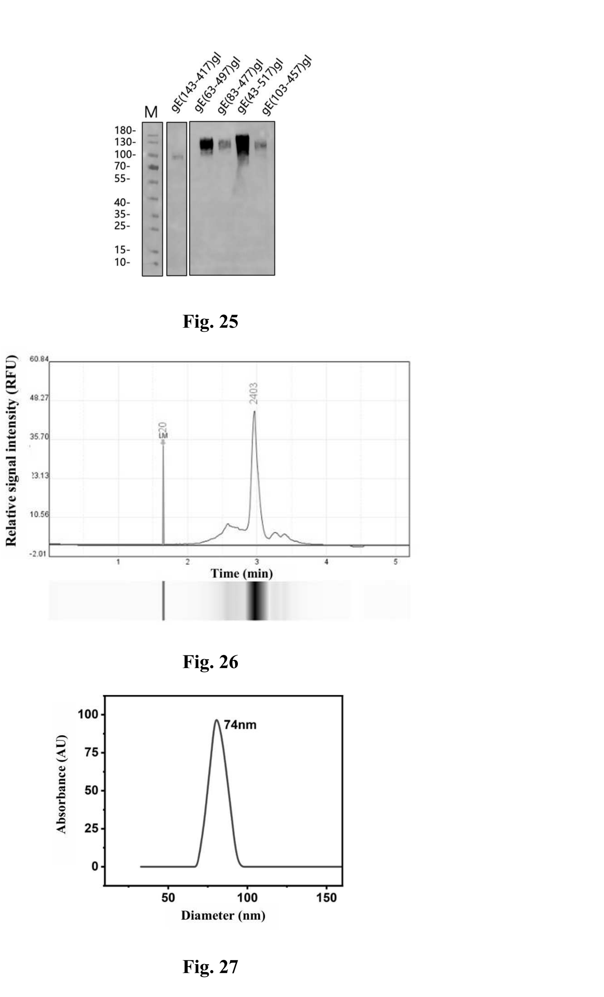

Fig. 25 showed the results of this example, which showed that although the above five fusion protein constructs had different expression levels, they all had good immune activity.

Example 12: Parallel comparison experiment of gE-gI mRNA vaccine combined with CpG adjuvant

### Linearization to obtain mRNA transcription template

To a 1.5 mL Ep tube, 25 µg of pUC-gEgI plasmid (the plasmid pUC57 was purchased from Sangon Biotechnology, Cat. No.: B522201, and an mRNA expression framework and gE-gI encoding nucleotide sequence were constructed on the plasmid, in which the framework was: a T7 promoter (SEQ ID NO: 16) + a DNA sequence corresponding to 5'UTR (SEQ ID NO: 17) + a coding sequence of kozak sequence (SEQ ID NO: 72) + a start codon + a nucleotide sequence (DNA sequence corresponding to SEQ ID NO: 71) encoding gE-gI fusion protein + a stop codon + a DNA sequence corresponding to 3'UTR (SEQ ID NO: 18) + a DNA sequence corresponding to PolyA (SEQ ID NO: 19)), 5 µL of BspQI restriction endonuclease (purchased from New England Biolabs, Cat. No.: R0712S), and 15 µL of NEBuffer r3.1 (purchased from New England Biolabs, Cat. No.: #B6003S) were added, supplemented with ddH₂O to a total volume of 150 µL, and mixed well. The enzyme digestion reaction was carried out in a 50°C water bath for 30 min. The resulting product of the enzyme digestion system was recovered in a cell-free clean bench according to the instructions of gel recovery kit (purchased from Tiangen Biochemical Technology (Beijing) Co., Ltd., Cat. No.: DP209-03). The recovered product was identified by agarose gel electrophoresis to obtain a linearized plasmid as a transcription template.

### mRNA in vitro transcription

(1) mRNA was prepared using T7 in vitro transcription kit (purchased from Nanjing Vazyme Biotech Co., Ltd., Cat. No.: DD4202). To a 1.5 mL RNase-free Ep tube, 2 µL of ATP, 2 µL of CTP, 2 µL fo GTP, 2 µL of UTP, 2 µL of Cap, 2 µL of RNase inhibitor, 2 µg of linearized gE-gI plasmid template were separately added, supplemented with DEPC water (purchased from Shanghai Sangon, Cat. No.: B501005-0500) to a total volume of 20 µL, and reacted in a metal water-bath at 37°C for 4 h;
(2) 2 µL of DNase I (purchased from New England Biolabs, Cat. No.: M0303L) was added, and incubated at 37°C for 20 min to digest the residual DNA template in the system;
(3) 30 µL of DEPC water was added to terminate the digestion reaction;
(4) 30 µL of LiCl (purchased from Invitrogen, Cat. No.: AM9480) was added, allowed to stand at -20°C for 30 min to precipitate mRNA;
(5) Centrifugation was performed at 4°C and 13000 rpm for 10 min, and the supernatant was carefully removed with the tip of a pipette;
(6) 1 mL of pre-cooled 75% ethanol was added, centrifuged at 4°C and 13000 rpm for 5 min, the supernatant was carefully removed with the tip of a pipette to remove residual protein in the system;
(7) The centrifuge tube was uncapped for air drying for 2 min, and 30 µL of DEPC water was added to resuspend the precipitate, thereby obtaining gE-gI mRNA.

### mRNA identification by Capillary electrophoresis

(1) 90 µL of DEPC water was taken and used to dilute 10 µL of 10× Dilution Buffer (Bioptic Inc., C104408-10X), thereby preparing 1× Dilution Buffer;
(2) 1 µL of mRNA sample was taken and mixed with 19 µL of 1× Dilution Buffer;
(3) Qsep100 was used to perform capillary electrophoresis to analyze the size of mRNA.

The results of the capillary electrophoresis experiment were shown in Fig. 26. The size of gE-gI mRNA measured by capillary electrophoresis was 2403 nt, and the theoretical size of the molecular as designed was 2676 nt. The size of mRNA was slightly smaller than the theoretical value, but within the error range. The capillary electrophoresis results showed that the gE-gI mRNA presented a single peak, indicating that the mRNA homogeneity was good.

### Mixture of mRNA and LNP

(1) A mRNA aqueous phase was prepared, in which 50 mM citric acid buffer pH=3.0 was used to dilute the mRNA to 80 µg/mL;
(2) A lipid mixture stock solution with a total lipid concentration of 100 mM was prepared according to the proportions in the table below, and stored at room temperature. It was placed in a 37 °C warm bath before use until no precipitate was visible. Dilution was performed with anhydrous ethanol to reach a final working concentration of 5 mM or 10 mM.

**Table 5: Preparation of lipid**

| Lipid name | Molar percentage, % | Source | Cat. No. |
|---|---|---|---|
| ALC-0315 | 46.3 | AVT (Shanghai) Pharmaceutical Tech Co., Ltd. | 2036272-55-4 |
| ALC-0519 | 1.6 | AVT (Shanghai) Pharmaceutical Tech Co., | 1849616-42-7 |
| | | Ltd. | |
| DSPC | 9.4 | AVT (Shanghai) Pharmaceutical Tech Co., Ltd. | S01005 |
| Cholesterol | 42.7 | AVT (Shanghai) Pharmaceutical Tech Co., Ltd. | 001001 |

(3) The mRNA and lipid phase were mixed. A microfluidic instrument was adopted, the pipeline was cleaned with ethanol, the dual channels were rinsed in advance with ethanol and citric acid buffer respectively, and after sufficient rinsing, the parameters to prepare sample loading were set as: mixed lipid phase : nucleic acid phase = 1:3 (v/v), flow rate ratio was 1:3, and total flow rate was 12 mL/min;
(4) The mixed mRNA/LNP was immediately diluted with 5 times volume of 1×PBS, and ultrafiltration was performed twice with a 10 kDa ultrafiltration tube. The replacement buffer was 1×PBS, and concentration was performed to reach the initial volume of the mixed mRNA/LNP.

### Determination of mRNA/LNP concentration and encapsulation efficiency

(1) The mRNA concentration was determined using the Quant-iT^{™} RiboGreen^{™} RNA Reagent and Kit (purchased from Thermo Fisher, Cat. No.: R11490). 4.75 mL of DEPC water was taken to dilute 250 µL of 20×TE to obtain 5 mL of 1×TE. 196 µL of 1×TE was taken to dilute 4 µL of RNA standard to obtain solution A. 1492.5 µL of 1×TE was taken to dilute 7.5 µL of Ribogreen to obtain solution B.
(2) On a 96-well U-bottom plate, 100 µL, 98 µL, 90 µL, 50 µL, 0 µL of 1×TE and 0 µL, 2 µL, 10 µL, 50 µL, 100 µL of solution A were added to corresponding wells, and 100 µL of solution B was added to each well and mixed well;
(3) Demulsification: 2% Triton buffer was prepared with 1×TE, and used to dilute mRNA/LNP 100-fold, and after being mixed well, the mixture was allowed to stand at room temperature for 5 min; the mRNA/LNP was diluted 100-fold with 1×TE; 100 µL of solution B was added to each well, mixed well, and allowed to stand at room temperature for 5 min;
(4) A multi-label detection/imaging analysis system instrument was used to read fluorescence, and a standard curve was drawn according to step (2) to calculate the mRNA sample concentration.

### Determination (DLS) of mRNA/LNP particle size

(1) A sample cup (UVette cuvette) was prepared, 45 µL of PBS was added with 5 µL of mRNA/LNP, mixed well, and placed into the cuvette;
(2) The particle size was measured according to the instrument operating instructions;
(3) The measurement results were recorded, the appropriate distribution method was chosen according to the actual results to process the measurement data.

The DLS measurement results were shown in Fig. 27. The results showed that the mRNA/LNP particle size was about 74 nm, which was consistent with the theoretical size, and the sample was a single peak, indicating that the mRNA/LNP had good homogeneity.

### Immunological evaluation experiment of gE-gI mRNA/LNP combined with CpG adjuvant

This experimental scheme was approved by the Experimental Animal Management Ethics Committee of Xiamen University. All operations were carried out in strict accordance with the animal ethics guidelines and the approved procedures.

Six-week-old C57BL/6 mice were selected and divided into four groups, with five mice in each group. The experimental groups were immunized with the gE-gI mRNA/LNP, and with the gE-gI mRNA/LNP plus 10 µg CpG, the immunization doses for each mouse were set as 10 µg, 100 µL. The control group was immunized with Shingrix vaccine (purchased from GSK, batch number: 4N2AB), and the blank group was immunized with saline. The mice were injected intramuscularly into tibia muscle at 0- and 3-week. Ocular venous blood was collected before injection at 0-, 2-, and 5-week, respectively. The blood samples were centrifuged at 13000 g for 10 minutes. The serum samples were stored at -20°C. The titers of antigen-specific binding antibody IgG were determined by ELISA, and cytokines were detected by flow cytometry.

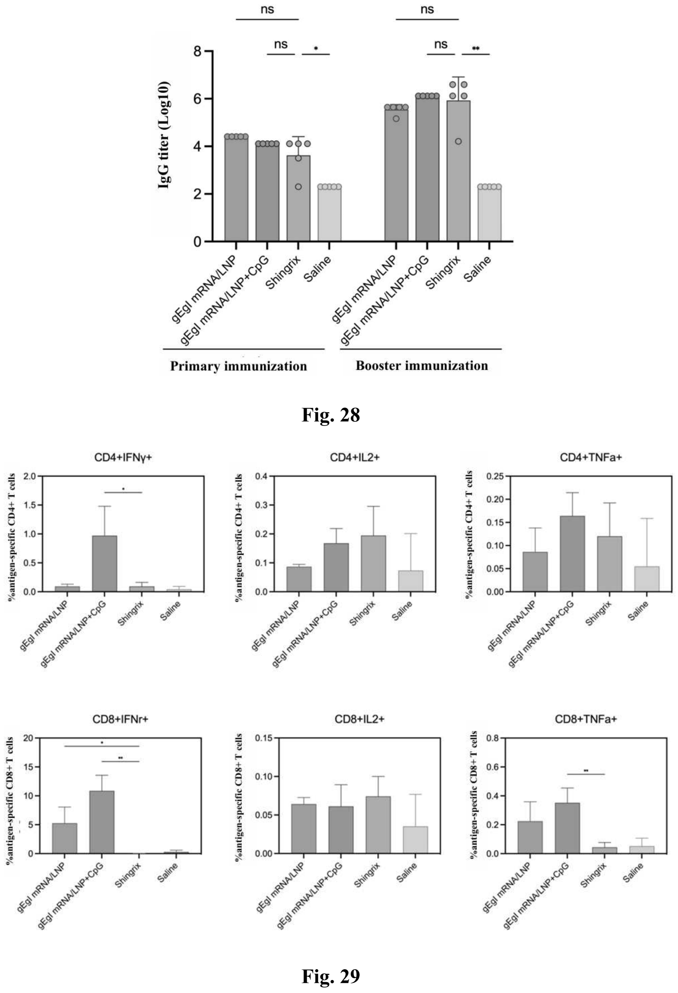

The ELISA results were shown in Fig. 28. The results in Fig. 28 showed that the titers of binding antibody IgG induced by immunization with the gE-gI mRNA/LNP and by immunization with the gE-gI mRNA/LNP combined with CpG, were comparable to that of the Shingrix vaccine, and there was no significant difference.

Fig. 29 showed the results of T cell responses stimulated by two different vaccines in this example. The results showed that the proportions of antigen-specific CD8⁺IFN-γ⁺ T cells induced by immunization with the gE-gI mRNA/LNP and by immunization with the gE-gI mRNA/LNP combined with CpG, were higher than those of the Shingrix vaccine, and there were significant differences. This indicated that the mRNA vaccines corresponding to the gE-gI fusion protein of the present disclosure had the ability to induce robust cellular immunity, which was better than that of commercial vaccines, thereby exhibiting promising application prospects.

### Example 13: Preparation and evaluation of gE-gI fusion protein derived from stable cell line

In order to facilitate the subsequent production and transformation applications, a gE-gI fusion protein derived from a stable CHO cell line was prepared, an immune evaluation was conducted in the present disclosure.

Preparation of gE-gI fusion protein from stable CHO cell line:
To facilitate the subsequent generation and transformation, the stable cell line used the original signal peptide of gE, so the sequence of gE was 1-537 aa of the full-length gE protein (SEQ ID NO: 11), and the original signal peptide was about 1-30 aa. During the expression of the fusion protein, the signal peptide 1-30aa would be removed, so that the gE extracellular region contained in the fusion protein after enzyme digestion was 31-537 aa (SEQ ID NO: 69), and the gI extracellular region contained was 21-271 aa (SEQ ID NO: 14). The amino acid sequence of the fusion protein after enzyme digestion and removal of the signal peptide was set forth in SEQ ID NO: 70.

The specific preparation method was as follows:
The gE (1-537) and gI (21-271) were also connected using linker (SEQ ID NO: 15), and the encoding sequence of gE-gI fusion protein was constructed into the plasmid for stable transformation, Canton PMGT vector (Guangzhou Canton Biologics Co., Ltd.), using a tag-free design, and the constructed recombinant plasmid was transfected into CHOZN CHOK1 GSKO (purchased from Merck, USA, Cat#CHOGS) host cells, and the cell pool was obtained by screening under stress of MSX (Sigma, Cat#GSS-1015-F). The expression levels of the target protein in different cell pools were compared by Fed-batch, and finally the cell pools with high expression level were selected for monoclonal plating. 238 monoclonal clones with good viability were obtained by SINGLE CELL PRINTER (Cytena, model F.sight) plating combined with cell imaging, and the culture was expanded step by step. The Top 20 clones were obtained by screening according to the expression levels of the clones in the 24-well plate and 6-well plate stages. The expression levels of the target proteins of the Top 20 clones were compared by Fed-batch, and the Top 5 clones were screened by combining the results of cell growth data, ELISA, SDS-page and Western blot to establish a research cell bank (RCB), and 35 cells were frozen for each clone. The Top 1 clone was selected for culture to express protein, using EX-CELL CD CHO Fusion Medium, culture conditions: 37°C, 140rpm, 5% CO₂, 85% humidity, 25mm rotation radius (Kuhner, model ISF1-XC). After 6 days of culture, the cell supernatant was harvested by centrifugation at 200 g for 8 min, purified and prepared by the tag-free purification method of gE-gI fusion protein in Example 1.

Table 6 showed the comprehensive data of the cell passage stability, yield change, etc. in the stability study for the Top 5 clones. The best Top 1 clone was CB4019-clone-16.

**Table 6: Summary of Top 5 clones**

| Clone | CB4019-clone -16 | CB4019-cl one-89 | CB4019-cl one-104 | CB4019-c lone-146 | CB4019-c lone-212 |
|---|---|---|---|---|---|
| Yield (g/L) | 6.6 | 5.96 | 7.57 | 4.21 | 4.59 |
| Cell-specific productivity (pg/cell/day) | 9.51 | 7.58 | 11.75 | 7.03 | 12.61 |
| Maximum viable cell density (×10⁶ cells/mL)/(day) | 58.6 | 55.3 | 45.8 | 44.6 | 26.1 |
| Culture days (days) | 13* | 14 | 14 | 14 | 14 |
| Harvest viability (%) | 87.2 | 86.1 | 91.8 | 97.6 | 96.8 |

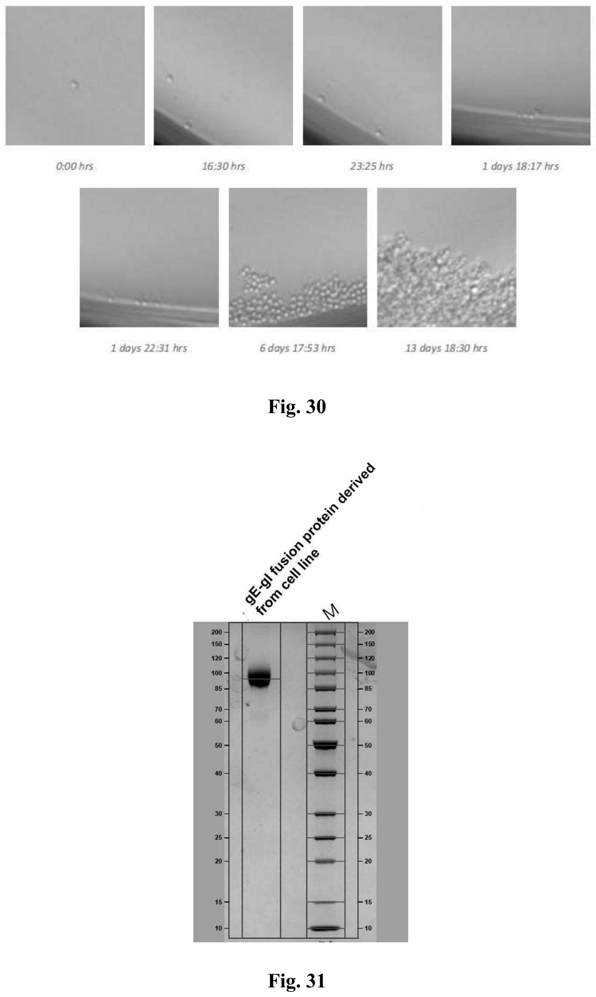

Fig. 30 showed the monoclonality confirmation results of Top 1 clone (CB4019-clone-16), and the results showed that CB4019-clone-16 was derived from the division of a single cell.

Fig. 31 showed the gE-gI fusion protein obtained by the tag-free purification method of Example 1, with a purity greater than 95%.

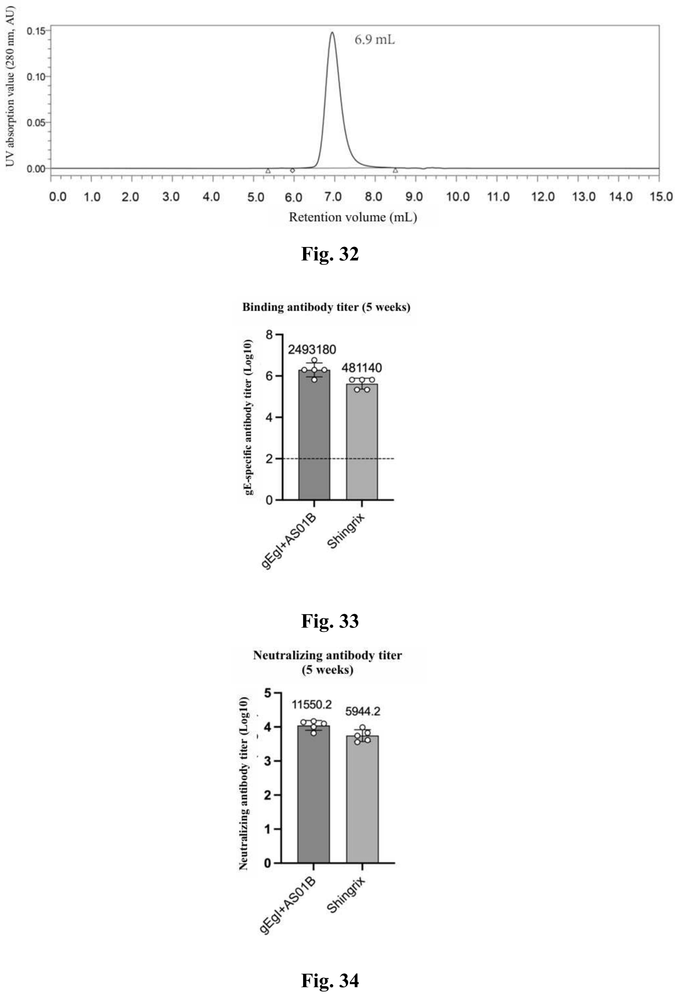

The results of Fig. 32 showed that the retention volume of the gE-gI fusion protein derived from the CHO stable cell line was about 6.9 mL, the sample presented a single peak, and both the homogeneity and purity were greater than 90%.

Immunological evaluation of gE-gI fusion protein derived from stable CHO cell line:
The obtained gE-gI fusion protein was immunologically evaluated according to the method of Example 8, and the results of the immunological evaluation were shown in Figs. 33 to 36.

As shown in Fig. 33, the binding antibody IgG induced by the gE-gI fusion protein derived from the CHO stable cell line combined with the AS01B adjuvant were 5 times that of the Shingrix vaccine.

Fig. 34 showed that the titer of neutralizing antibodies induced by the gE-gI fusion protein combined with AS01B adjuvant was about 2 times that of Shingrix vaccine.

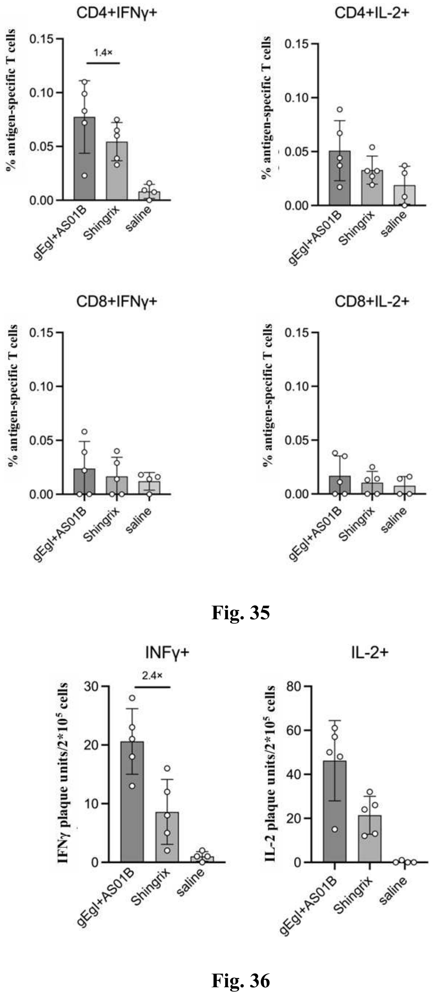

Fig. 35 showed the results of CD4⁺ T and CD8⁺ T cell responses stimulated by two different vaccines in this example. The results showed that the levels of antigen-specific CD4⁺IFN-γ⁺ T cells induced by the gE-gI fusion protein combined with AS01B-like adjuvant was significantly higher than that of Shingrix, i.e., about 1.4 times that of Shingrix.

Fig. 36 showed the levels of cytokines IFN-γ and IL-2 stimulated by two different vaccines in this example, as detected by Elispot assay. The results showed that the levels of IFN-γ and IL-2 induced by the gE-gI fusion protein with AS01B-like adjuvant were higher than those of Shingrix, in which IFN-γ was 2.4 times that of Shingrix.

Although the specific embodiments of the present disclosure have been described in detail, those skilled in the art will understand that various modifications and changes can be made to the details according to all the teachings that have been disclosed, and these changes are within the scope of protection of the present disclosure. All the divisions of the present disclosure are given by the appended claims and any equivalents thereof.

## Claims

1. A fusion protein, comprising a first peptide segment and a second peptide segment, wherein the first peptide segment comprises or is composed of an extracellular region of varicella-zoster virus (VZV) gE protein or a fragment thereof; and the second peptide segment comprises or is composed of an extracellular domain of VZV gI protein or a fragment thereof.

2. The fusion protein according to claim 1, wherein the first peptide segment comprises at least 100, at least 150, at least 200 or at least 250 consecutive amino acid residues within the amino acid residues of the gE protein at positions corresponding to the positions 150-410 of SEQ ID NO: 11; and/or the second peptide segment comprises at least 50, at least 100, at least 150 or at least 180 consecutive amino acid residues within the amino acid residues of the gI protein at positions corresponding to the positions 45-240 of SEQ ID NO: 12.

3. The fusion protein according to claim 1 or 2, wherein the first peptide segment comprises the amino acid residues of the gE protein at positions corresponding to the positions 150-410 of SEQ ID NO: 11;
preferably, the first peptide segment comprises the amino acid residues of the gE protein at positions corresponding to the positions 70-485 of SEQ ID NO: 11;
preferably, the first peptide segment comprises the amino acid residues of the gE protein at positions corresponding to the positions 63-497 of SEQ ID NO: 11;
preferably, the first peptide segment comprises the amino acid residues of the gE protein at positions corresponding to the positions 31-537 of SEQ ID NO: 11;
preferably, the gE protein has: (a) an amino acid sequence as set forth in any one of SEQ ID NOs: 11, 46-55; (b) an amino acid sequence having an identity of at least 90% (e.g., at least 95%, at least 96%, at least 97%, at least 98%, at least 99%) as compared to the amino acid sequence as set forth in any one of SEQ ID NOs: 11, 46-55; or, (c) an amino acid sequence having a substitution (preferably conservative substitution), addition or deletion of one or several (e.g., 1, 2, 3, 4, 5, 6, 7, 8 or 9) amino acids as compared to the amino acid sequence as set forth in any one of SEQ ID NOs: 11, 46-55;
preferably, the first peptide segment comprises or is composed of an amino acid sequence as set forth in any one of SEQ ID NOs: 13, 33-42, 64-69, or, a sequence having an identity of at least 90%, for example, at least 95%, at least 96%, at least 97%, at least 98%, at least 99%, or, having a substitution (preferably conservative substitution), addition or deletion of one or several (e.g., 1, 2, 3, 4, 5, 6, 7, 8 or 9) amino acids as compared to the amino acid sequence as set forth in any one of SEQ ID NOs: 13, 33-42, 64-69.

4. The fusion protein according to any one of claims 1 to 3, wherein the second peptide segment comprises the amino acid residues of the gI protein at positions corresponding to the positions 45-240 of SEQ ID NO: 12;
preferably, the second peptide segment comprises the amino acid residues of the gI protein at positions corresponding to the positions 30-260 of SEQ ID NO: 12;
preferably, the second peptide segment comprises the amino acid residues of the gI protein at positions corresponding to the positions 21-271 of SEQ ID NO: 12;
preferably, the gI protein has: (a) an amino acid sequence as set forth in any one of SEQ ID NOs: 12, 56-58; (b) an amino acid sequence having identity of at least 90% (e.g., at least 95%, at least 96%, at least 97%, at least 98%, at least 99%) as compared to the amino acid sequence as set forth in any one of SEQ ID NOs: 12, 56-58; or, (c) an amino acid sequence having a substitution (preferably conservative substitution), addition or deletion of one or several (e.g., 1, 2, 3, 4, 5, 6, 7, 8 or 9) amino acids as compared to the amino acid sequence as set forth in any one of SEQ ID NOs: 12, 56-58;
preferably, the second peptide segment comprises or is composed of an amino acid sequence as set forth in any one of SEQ ID NOs: 14, 43-45, or, a sequence having an identity of at least 90%, for example, at least 95%, at least 96%, at least 97%, at least 98%, at least 99%, or, having a substitution (preferably conservative substitution), addition or deletion of one or several (e.g., 1, 2, 3, 4, 5, 6, 7, 8 or 9) amino acids as compared to the amino acid sequence as set forth in any one of SEQ ID NOs: 14, 43-45.

5. The fusion protein according to any one of claims 1 to 4, wherein the second peptide segment is connected to the N-terminal or C-terminal of the first peptide segment, optionally via a linker (e.g., a peptide linker, for example, a peptide linker as set forth in SEQ ID NO: 15);
preferably, the linker is a peptide linker comprising one or more (e.g., 5-20) flexible amino acids;
preferably, the linker is a peptide linker comprising one or more glycines and/or one or more serines;
preferably, the fusion protein comprises or is composed of an amino acid sequence as set forth in any one of SEQ ID NOs: 6-7, 20-32, 59-63, 70.

6. The fusion protein according any one of claims 1 to 5, wherein the fusion protein comprises a signal peptide and/or a detectable label (e.g., a tag protein);
preferably, the fusion protein comprises at its N-terminal a signal peptide (e.g., a natural signal peptide of VZV gE protein, a natural signal peptide of VZV gI protein, a gp67 signal peptide, a melittin signal peptide (MSP), or a tissue-type plasminogen activator signal peptide (tPA signal peptide));
preferably, the fusion protein comprises at its C-terminal a detectable label.

7. An RNA molecule, which comprises a nucleotide sequence encoding the fusion protein according to any one of claims 1 to 6.

8. The RNA molecule according to claim 7, further comprising one or more selected from 5'UTR, Kozak sequence, start codon, stop codon, 3'UTR, poly-A tail;
preferably, the RNA molecule comprises from 5' to 3': 5'UTR, Kozak sequence, start codon, nucleotide sequence encoding the fusion protein, stop codon, 3'UTR, poly-A tail.

9. The RNA molecule according to claim 7 or 8, which has one or more of the following characteristics:
(1) the nucleotide sequence encoding the fusion protein has a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the nucleotide sequence as set forth in SEQ ID NO: 71; preferably, the nucleotide sequence encoding the fusion protein is set forth in SEQ ID NO: 71;
(2) there are one or more stop codons at the 3' end of the nucleotide sequence encoding the fusion protein;
(3) the 5'UTR has a nucleotide sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the nucleotide sequence as set forth in SEQ ID NO: 17; preferably, the 5'UTR has the nucleotide sequence as set forth in SEQ ID NO: 17;
(4) the 3'UTR has a nucleotide sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the nucleotide sequence as set forth in SEQ ID NO: 18; preferably, the 3'UTR has the nucleotide sequence as set forth in SEQ ID NO: 18;
(5) the Kozak sequence is set forth in SEQ ID NO: 72;
(6) the poly-A tail comprises one or more polyadenylic acid sequences, each of the one or more polyadenylic acid sequences is independently composed of 20 to 120 consecutive adenylic acids; preferably, the poly-A tail comprises a plurality of polyadenylic acid sequences, and the adjacent polyadenylic acid sequences are connected by a non-A spacer sequence; preferably, the poly-A tail has a nucleotide sequence having a sequence identity of at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared to the nucleotide sequence as set forth in SEQ ID NO: 19; preferably, the poly-A tail has the nucleotide sequence as set forth in SEQ ID NO: 19;
(7) the 5' end of the RNA molecule is modified with a 5' cap (e.g., CAP-0, CAP-1, CAP-2).

10. An isolated nucleic acid molecule, comprising a nucleotide sequence encoding the fusion protein according to any one of claims 1 to 6 or the RNA molecule according to any one of claims 7 to 9.

11. A vector, comprising the isolated nucleic acid molecule according to claim 10.

12. A delivery composition, comprising a delivery carrier, and the RNA molecule according to any one of claims 7 to 9, or the isolated nucleic acid molecule according to claim 10, or the vector according to claim 11;
preferably, the delivery carrier is selected from the group consisting of lipid particle, sugar particle, metal particle, protein particle, liposome, exosome, microvesicle and viral vector (e.g., replication-defective retrovirus, lentivirus, adenovirus or adeno-associated virus);
preferably, the delivery composition comprises the RNA molecule according to any one of claims 7 to 9.

13. The delivery composition according to claim 12, wherein the delivery carrier is a lipid nanoparticle (LNP).

14. A host cell, comprising the RNA molecule according to any one of claims 7 to 9, or the isolated nucleic acid molecule according to claim 10, or the vector according to claim 11;
preferably, the host cell is selected from the group consisting of prokaryotic cell (e.g., *E. coli* cells), and eukaryotic cell (e.g., yeast cell, insect cell, plant cell, mammalian cell);
preferably, the host cell is an insect cell;
preferably, the host cell is a mammalian cell.

15. A method for preparing the fusion protein according to any one of claims 1 to 6, comprising culturing the host cell according to claim 14 under a condition that allows protein expression, and recovering the fusion protein from a cell culture.

16. An immunogenic composition, comprising the fusion protein according to any one of claims 1 to 6, or the RNA molecule according to any one of claims 7 to 9, or the isolated nucleic acid molecule according to claim 10, or the vector according to claim 11, or the delivery composition according to claim 12 or 13, and optionally a pharmaceutically acceptable carrier and/or excipient (e.g., adjuvant);
preferably, the immunogenic composition comprises the fusion protein according to any one of claims 1 to 6, or the RNA molecule according to any one of claims 7 to 9, or the isolated nucleic acid molecule according to claim 10, or the vector according to claim 11, or the delivery composition according to claim 12 or 13, and an adjuvant, wherein the adjuvant is selected from the group consisting of: aluminum salt adjuvant, zinc-aluminum hybrid adjuvant (e.g., FH002C), Freund's adjuvant, oil emulsion adjuvant (e.g., MF59 adjuvant), cytokine, TLR agonist, CpG adjuvant, nucleic acid adjuvant, liposome, saponin adjuvant, AS01B adjuvant and any combination thereof;
preferably, the immunogenic composition comprises the fusion protein according to any one of claims 1 to 6 or the delivery composition according to claim 12 or 13, and the adjuvant;
preferably, the immunogenic composition is a vaccine.

17. Use of the fusion protein according to any one of claims 1 to 6, or the RNA molecule according to any one of claims 7 to 9, or the isolated nucleic acid molecule according to claim 10, or the vector according to claim 11, or the delivery composition according to claim 12 or 13, or the host cell according to claim 14, or the immunogenic composition according to claim 16, in the manufacture of an immunogenic composition, wherein the immunogenic composition is used for inducing an immune response against VZV in a subject and/or for preventing and/or treating a VZV infection or a disease associated with VZV infection in a subject;
preferably, the immunogenic composition is a vaccine;
preferably, the VZV infection is a primary infection or recurrent infection of VZV;
preferably, the disease associated with VZV infection is selected from the group consisting of: herpes zoster, varicella, and complication thereof (e.g., postherpetic neuralgia, pneumonia, encephalomyelitis, conjunctivitis);
preferably, the subject is a mammal, such as a human.

18. A method for inducing an immune response against VZV in a subject and/or for preventing and/or treating a VZV infection or a disease associated with VZV infection in a subject, comprising: administering to the subject in need thereof an effective amount of the fusion protein according to any one of claims 1 to 6, or the RNA molecule according to any one of claims 7 to 9, or the isolated nucleic acid molecule according to claim 10, or the vector according to claim 11, or the delivery composition according to claim 12 or 13, or the host cell according to claim 14, or the immunogenic composition according to claim 16;
preferably, the VZV infection is a primary infection or recurrent infection of VZV;
preferably, the disease associated with VZV infection is selected from the group consisting of: herpes zoster, varicella, and complication thereof (e.g., postherpetic neuralgia, pneumonia, encephalomyelitis, conjunctivitis);
preferably, the subject is a mammal, such as a human.

19. A method for detecting the presence of an antibody specific to VZV gI protein and/or an antibody specific to VZV gE protein in a sample, comprising using the fusion protein according to any one of claims 1 to 6;
preferably, the method is an immunological detection, such as immunoblotting assay, enzyme immunoassay (e.g., ELISA), chemiluminescent immunoassay, fluorescent immunoassay or radioimmunoassay;
preferably, the method comprises: (1) contacting the sample with the fusion protein according to any one of claims 1 to 6; (2) detecting the formation of a fusion protein-antibody immune complex or detecting an amount of the immune complex; wherein the formation of the immune complex indicates the presence of the antibody specific for VZV gI protein and/or the antibody specific for VZV gE protein in the sample;
preferably, the method further comprises using a second antibody with a detectable label (e.g., an enzyme (e.g., horseradish peroxidase or alkaline phosphatase), a chemiluminescent agent (e.g., acridinium ester compound, luminol and derivative thereof, or ruthenium derivative), a fluorescent dye (e.g., fluorescein or fluorescent protein), a radionuclide or biotin) to detect the presence of the antibody specific for VZV gI protein and/or the antibody specific for VZV gE protein in the sample;
preferably, the second antibody is specific for a constant region contained in an antibody of a species (e.g., human) from which the sample to be tested comes;
preferably, the second antibody is an anti-immunoglobulin (e.g., human immunoglobulin) antibody, such as an anti-IgG antibody;
preferably, the sample is a body fluid sample (e.g., whole blood, plasma, serum, salivary excreta or urine) from a subject (e.g., a mammal, preferably a human).

20. Use of the fusion protein according to any one of claims 1 to 6, or the RNA molecule according to any one of claims 7 to 9, or the isolated nucleic acid molecule according to claim 10, or the vector according to claim 11, or the delivery composition according to claim 12 or 13, or the host cell according to claim 14, in the manufacture of a detection reagent, wherein the detection reagent is used for detecting the presence of an antibody specific for VZV gI protein and/or an antibody specific for VZV gE protein in a sample;
preferably, the detection reagent detects the presence of an antibody specific for VZV gI protein and/or an antibody specific for VZV gE protein in a sample by the method according to claim 19;
preferably, the sample is a body fluid sample (e.g., whole blood, plasma, serum, salivary excreta or urine) from a subject (e.g., a mammal, preferably a human).

21. A kit, comprising the fusion protein according to any one of claims 1 to 6, or the RNA molecule according to any one of claims 7 to 9, or the isolated nucleic acid molecule according to claim 10, or the vector according to claim 11, or the delivery composition according to claim 12 or 13, or the host cell according to claim 14;
preferably, the kit comprises the fusion protein according to any one of claims 1 to 6, and a second antibody, wherein the second antibody is as defined in claim 19.
